# EUROPEAN PATENT APPLICATION

(11) **EP 4 360 616 A1**
(43) Date of publication of application: **01.05.2024**
(21) Application number: 23206713.2
(22) Date of filing: 30.10.2023
(51) Int. Cl.: A61K 8/36, A61K 8/37, A61K 8/92, A61K 31/20, A61K 31/23, A61K 35/644, A61K 36/889, A61K 9/00, A61K 8/65, A61K 8/9706, A61K 8/98, A61K 35/60, A61K 47/42, A61K 47/44, A61Q 19/00, A61Q 17/00, A61P 17/02

(54) **COMPOSITION OF AND METHOD FOR TREATING INJURY AND/OR SKIN CONDITIONS**

(30) Priority: 28.10.2022 US 202263420202 P; 28.10.2022 US 202263420412 P; 28.10.2022 US 202263420509 P
(71) Applicant: Omeza Holdings, Inc., Sarasota, Florida 34234 (US)
(72) Inventor: BETTLE, Griscom, Sarasota, 34234 (US); HAYES, Lalania, Sarasota, 34234 (US); BAKEWELL, Suzanne, Sarasota, 34234 (US)
(74) Representative: Stepney, Gregory John

(57) **Abstract**

Disclosed herein relates to compositions and methods for the treatment of skin damage and/or wounds, while simultaneously relieving pain and/or cosmetic damage. As such, the present disclosure relates to an anhydrous topical composition for retarding and/or inhibiting scarring of a wound, burn and/or skin condition located on the skin of a subject while retaining a natural characteristic and/or elasticity of the skin. The anhydrous topical composition may comprise MCT, monolaurin, cetyl esters, marine oil, vegetable oil having an omega 3 fatty acid content greater than 9 wt%, and/or hempseed oil. Additionally, the anhydrous topical composition may comprise antimicrobial properties and/or characteristics, such that the anhydrous topical composition may be configured to inhibit any pathogen (e.g., a bacteria, a virus, a fungus, etc.) within the wound, burn and/or skin condition, when applied to the skin of the subject.

## Description

### CROSS-REFERENCE TO RELATED APPLICATIONS

This nonprovisional application claims the benefit of U.S. Provisional Application No. 63/420,202 entitled "AN ANHYDROUS TOPICAL GEL COLLAGEN-FREE COMPOSITION COMPRISED OF MARINE OIL" filed October 28, 2022 by the same inventors, U.S. Provisional Application No. 63/420,509 entitled "COMBINATION THERAPY FOR THE TREATMENT OF WOUNDS, BURNS AND SKIN CONDITIONS" filed October 28, 2022 by the same inventors, and U.S. Provisional Application No. 63/420,412 entitled "COMPOSITIONS COMPRISING MCT AND MARINE OIL" filed October 28, 2022 by the same inventors, all of which are incorporated herein by reference, in their entireties, for all purposes.

### BACKGROUND OF THE INVENTION

### 1. Field of the Invention

This invention relates, generally, to treating skin conditions. More specifically, it relates to a composition and method for the treatment of skin damage and/or wounds, while simultaneously relieving pain and/or cosmetic damage.

### 2. Brief Description of the Prior Art

Marine oil, including cod liver oil, salmon oil and oil from algae, such as seaweed, contains, among other components, omega-3 fatty acids, which have a variety of anti-inflammatory and immune-modulating effects that may be of relevance for treating diseases and conditions where inflammation is an underlying cause. Inflammation is the body's attempt at self-protection where the aim is to remove harmful stimuli and start the healing process. Inflammation may be divided into two types of inflammation: acute and chronic inflammation. Acute inflammation is short-term inflammation which starts rapidly and quickly in response to tissue damage. Examples of acute inflammation include acute bronchitis and acute appendicitis. Chronic inflammation is a slow and prolonged inflammation response which involves a progressive shift in the type of cells present at the site of inflammation characterized by the simultaneous destruction and repair of tissue during the inflammatory process. Examples of chronic inflammation include, but are not limited to, failure to eliminate the causing agent, an autoimmune response to a self-antigen and a chronic irritant of low intensity that persists. Chronic inflammation may, however, mature into severe diseases such as chronic obstructive pulmonary disease (COPD), cancer, atherosclerosis, Alzheimer's disease (AD), amyotrophic lateral sclerosis (ALS), and the like.

The omega-3 fatty acids are essential to life at any stage, even before birth. They are essential building blocks of the membrane of every cell in the body, and their presence is a necessity for maintaining an adequate cell membrane. They also contribute to the regulation of most biological functions.

The richest dietary source of long-chain omega-3 polyunsaturated fatty acids (PUFA) comes from marine oil, such as fish oil and algae oil. Fatty acids are the building blocks of dietary fats and are stored substantially in the form of triglycerides. The body cannot, produce omega 3 and omega 6 polyunsaturated fatty acids and therefore must obtain them from food sources or from supplements. Four fatty acids in the omega-3 family include alpha-linolenic acid (ALA), stearidonic acid (SDA), eicosapentaenoic acid (EPA) and docosahexaenoic acid (DHA). ALA and SDA are found in nuts (*e.g.,* walnuts), some types of beans, hempseed oil and/or olive oils. EPA and DHA are found in fish, including fish oil and algae and supplements. These omega-3 fatty acids may be useful in treating wounds, burns and skin conditions.

A recent study at Brigham and Women's Hospital in Boston revealed that omega-3 fatty acids actually convert into compounds that are 10,000 times more potent than the original fatty acids themselves. These compounds include resolvins, which help bring an inflammatory response in the body to an end. Resolvins and protectins are oxygenated metabolites derived from EPA and DHA, and a part of the molecular mechanisms contributing to removal of inflammatory cells and restoration of tissue once the need for inflammatory response is over. It has been shown that aspirin treatment enhances the conversion of EPA and DHA to resolvins which carry potent anti-inflammatory signals. The mechanisms by which their effects are exerted are still a matter of controversy, but it seems likely that these oxygenated metabolites play a significant role as they have potent anti-inflammatory and immunoregulatory actions even in concentrations in the nanomolar and picomolar range. As tissues return to normal, resolvins and protectins together with further oxygenated metabolites, such as lipoids and maresins, promote resolution of the inflammation through removal of leukocytes and cellular debris.

It is important to recognize that the healing of wounds, burns and skin conditions does not occur just at the situs of the burn and/or skin condition, but also requires assistance from the broadly-defined periwound and from increased blood flow, either from sharp debridement or blood-driven oxidative bursts (also known as "respiratory bursts").

During the healing process, neutrophils and monocytes are recruited to the injury site to destroy pathogenic organisms and to facilitate the inflammatory-proliferative transition. In the following phase, keratinocytes proliferate to reform the epidermis. Fibroblasts migrate to the injury site and are stimulated to proliferate by platelet-derived growth factor (PDGF) and other growth factors. During this phase, fibroblasts and their derived myofibroblasts break down the fibrin clots by secreting plasminogen activators that convert plasminogen to plasmin, which cleaves fibrin to dissolve the clot. Fibroblasts and myofibroblasts also secrete collagen and other extracellular matrix proteins that form granulation tissue. The formation of granulation tissue is accompanied by the development of new blood vessels, re-epithelialization at the site of injury, and contraction of the granulation tissue to bring the edges of the skin where the injury occurred closer together. In a final phase, fibroblasts, macrophages, and endothelial cells secrete extracellular matrix proteins and matrix metalloproteinases that remodel the granulation tissue, replacing the collagen type III matrix with a stronger, more organized collagen type I matrix.

Reactive oxygen species (ROS) play a pivotal role in the orchestration of the normal healing response. They act as secondary messengers to many immunocytes and non-lymphoid cells, which are involved in the repair process, and appear to be important in coordinating the recruitment of lymphoid cells to the injury site and effective tissue repair. ROS also possess the ability to regulate the formation of blood vessels (angiogenesis) at the injury site and the optimal perfusion of blood into the injury-healing area. ROS act in the host's defense through phagocytes that induce a ROS burst onto the pathogens present at the injury site, leading to their destruction. Moreover, during this period, excess ROS leakage into the surrounding environment has further bacteriostatic effects. In light of these important roles of ROS in healing and the continued quest for therapeutic strategies to treat burns and skin conditions, the manipulation of ROS represents a promising avenue for improving these responses when they are stalled.

A number of recent studies have demonstrated that mitochondrial energy metabolism has a strong correlation with inflammatory responses, including macrophage polarization. Specifically, classically activated M1 macrophages depend on glycolysis and further lactate fermentation, even in the presence of excess oxygen. In contrast, alternatively activated M2 macrophages seem to utilize oxidative phosphorylation (OXPHOS), for which fatty acids from dietary lipids might aid in the supply of substrate acetyl-CoA through β-oxidation.

MCT oil drives macrophages to exhibit anti-inflammatory functions by enhancing mitochondrial respiration. MCT oil exhibited no effect on oxygen consumption in a non-stimulated M0-like status. Interestingly, however, under M1-like inflammatory conditions following LPS (lipopolysaccharide, an endotoxin) stimulation, MCT oil significantly increased OCR (oxygen consumption rate) for basal respiration, maximal respiration, and ATP production, indicating that MCT not only suppresses inflammatory responses in M1 status, but also actively upregulates the switch of macrophage function towards M2-like repolarization. MCT enhances anti-inflammatory responses of macrophages via up-regulating mitochondrial respirations, in accordance with previous reports, in which increased energy expenditure by medium-chain fatty acids (MCFAs) drives macrophages to exhibit anti-obesity and anti-inflammatory phenotypes.

Overall, up-regulated β-oxidation by MCT contributes to the anti-inflammatory M2-like status of macrophages, which may aid in the prevention and/or amelioration of inflammation.

MCT may stimulate anti-inflammatory macrophages to decrease inflammation and to help create ROS, but excess ROS has to be quenched. Free radical quenching is done by PUFA from vegetable and marine sources.

In particular, the lipophilic structure of PUFA and molecular space dimensions allow EPA fatty acids to insert efficiently into lipoprotein particles and cell lipid membranes where it scavenges free radicals. In contrast to EPA, DHA serves essential functions in nervous tissues where it is abundant and has pronounced effects on neuronal and retinal membrane organization.

Many formulations for treating wounds, burns and skin disorders applied topically to the site of injury are oils, gels, salves, or paste and contain omega-3 fatty acids. In order to effect treatment, the omega-3 fats in the formulations must pass through the skin. However, these formulations are difficult to use topically because they partition into a retentate and a permeate fraction. The permeate passes through the stratum corneum into the epidermis and then into the bloodstream (transdermal).

Transdermal compositions, where physical chemistry can modify the barrier of the stratum corneum to allow low molecular weight, lipid-soluble active pharmaceutical ingredients (API), to pass into the bloodstream in the dermis. In order for the API to enter the bloodstream, the API must pass through the stratum corneum, either by the transcellular pathway or the intercellular pathway, and across the epidermis. The dermis sits atop the hypodermis, a fatty layer also called the subcutaneous fascia. In addition, the hair follicle is rooted in the hypodermis and extends upwards through the dermis and epidermis.

A second pathway for topical compositions to pass through skin is by the hair follicle (i.e., interfollicular route). The hair follicle route deposits unconsumed permeate at the cutaneous fat layer below the dermis. The effect and ramifications of the API passing through the epidermis via the intercellular route, or the hair follicle route are different.

There is very little research on the effect of topical omega-3 fatty acids. Problems with a topical application of omega-3 fatty acids isolated from fish oil is smell (e.g., fishy and/or rancid fat), greasy skin and the barrier associated with the stratum corneum, the outermost layer of the epidermis. Long chain fats may not migrate through this barrier easily and even if they do get across the outer barrier, they have to migrate through the rest of the epidermis and the "basement membrane" that separates the epidermis from the dermis. Only in the dermis may the omega-3 fats enter the blood stream.

Additionally, pressure sores and/or wounds are injuries to the skin and/or the tissues under the skin. These types of wounds commonly form on persons confined to a chair or bed. Pressure wounds may range from red areas on the surface of the skin to severe tissue damage that goes deep into muscle and bone. Constant pressure on an area of skin reduces blood supply to that area and, over time, may break down the skin and form an open sore. Pressure wounds usually form on the skin over bony areas where there is little tissue between the bone and the skin. A common place for pressure wounds to form is in the lower back above the sacrum and coccyx. Treatment of these "sacral" wounds includes keeping the wound clean and covered with wound dressings.

One of the most common forms of pressure sores and/or wounds are pressure ulcers. Pressure ulcers are wounds that form as a direct result of pressure over a bony prominence. Seventy-five percent of these injuries occur around the pelvic girdle, Factors that prevent normal healing from occurring include poor nutrition, infection, edema, persistent moisture, fecal and urinary soiling, and shearing forces. Specific risk factors for sacral pressure ulcers include lying in bed and fecal incontinence.

Unfortunately, there is inadequate treatment available for someone who is suffering from pressure wounds. The present disclosure may provide compositions and methods for treating a plurality of wounds.

Accordingly, what is needed is a composition and method for the treatment of skin damage and/or wounds, while simultaneously relieving pain and/or cosmetic damage. However, in view of the art considered as a whole at the time the present invention was made, it was not obvious to those of ordinary skill in the field of this invention how the shortcomings of the prior art could be overcome.

### SUMMARY OF THE INVENTION

The long-standing but heretofore unfulfilled need, stated above, is now met by a novel and non-obvious invention disclosed and claimed herein. In an aspect, the present disclosure pertains to an anhydrous topical composition comprising an omega fatty acid product. In an embodiment, the omega fatty-acid product may comprise the following: (a) at least 5% marine oil by weight; (b) at least 5% vegetable oil by weight; and (c) a fish collagen, such that the marine oil and/or the vegetable oil comprise an omega-3 fatty acid content greater than 9% by weight. In this embodiment, the omega fatty acid product may be antimicrobial, such that the omega fatty acid product may comprise an inhibition zone of a dermal area of at least 1.2 cm².

In some embodiments, the anhydrous topical composition may further comprise the following, including but not limited to hempseed oil, triethyl citrate, ethyl linoleate, a caprylic acid, a monolaurin, a cetyl ester, an ascorbyl palmitate, a beeswax, a sea salt, and/or a combination of thereof. In some embodiments, the composition may comprise a thickening agent. In these other embodiments, the composition may also comprise at least 0.5% ascorbyl palmitate by weight.

In some embodiments, the vegetable oil may comprise the following, including but not limited to coconut oil, hempseed oil, red palm olein, and/or a combination of thereof. In some embodiments, the omega fatty acid product may also comprise a penetration rate into a dermal area of at least 1.0 to at most 3.0. In these other embodiments, the omega fatty acid product is configured to inhibit MRSA, PA27312, or both.

Another aspect of the present disclosure pertains to an anhydrous topical composition comprising an omega fatty acid product. In an embodiment, the omega fatty-acid product may comprise the following: (a) at least 5% marine oil by weight; (b) at least 10% hempseed oil by weight; and (c) at least 1% cetyl esters by weight, such that the marine oil, the and/or hempseed oil may comprise an omega-3 fatty acid content greater than or equal to 9% by weight. In this embodiment, the omega fatty acid product may be antimicrobial, such that the omega fatty acid product may comprise an inhibition zone of a dermal area of at least 2.1 cm².

In some embodiments, the composition may comprise at least 0.8% lidocaine by weight. The composition may also comprise at least 0.5% ascorbyl palmitate by weight. In these other embodiments, the composition may further comprise a thickening agent.

In some embodiments, the anhydrous topical composition may further comprise the following, including but not limited to a hempseed oil, a monolaurin, medium chain triglycerides (hereinafter "MCT"), a ascorbyl palmitate, free fatty acids (hereinafter "FFA"), and/or a combination of thereof. In some embodiments, the omega fatty acid product may comprise a semi-solid state, such that when the omega fatty acid product is sheared, the omega fatty acid product may not form local capillaries. In these other embodiments, the omega fatty acid product may also be configured to inhibit MRSA, PA27312, or both.

Furthermore, an additional aspect of the present disclosure pertains to a method for synthesizing an anhydrous topical composition comprising an omega fatty acid product. In an embodiment, the method may comprise the following steps: (a) providing a mixture comprising a marine oil; (b) subjecting said mixture to a heating vessel and a cooling vessel to yield a colloidal product; ad (c) using the colloidal product, a derivative thereof, or both to form the composition comprising the omega fatty acid product, such that the omega fatty acid product may comprise (i) at least 5% marine oil by weight; (ii) at least 5% vegetable oil by weight; and (iii) a fish collagen. In this embodiment, the marine oil and/or the vegetable oil may comprise an omega-3 fatty acid content greater than 9% by weight. In addition, the omega fatty acid product may be antimicrobial, such that the omega fatty acid product may comprise an inhibition zone of a dermal area of at least 1.2 cm².

In some embodiments, the method may further comprise the step of using a thickening agent to aggregate the mixture and/or the colloidal product, or both. In these other embodiments, the omega fatty acid product may comprise a penetration rate into a dermal area of at least 1.0 to at most 3.0.

In some embodiments, the anhydrous topical composition may further comprise the following, including but not limited to hempseed oil, triethyl citrate, ethyl linoleate, a caprylic acid, a monolaurin, a cetyl ester, a ascorbyl palmitate, a beeswax, a sea salt, and/or a combination of thereof. In these other embodiments, the anhydrous topical composition may comprise at least 0.5% ascorbyl palmitate by weight. In some embodiments, the vegetable oil may comprise the following, including but not limited to coconut oil, hempseed oil, red palm olein, and/or a combination of thereof.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not restrictive.

The invention accordingly comprises the features of construction, combination of elements, and arrangement of parts that will be exemplified in the disclosure set forth hereinafter and the scope of the invention will be indicated in the claims.

### BRIEF DESCRIPTION OF THE DRAWINGS

For a fuller understanding of the invention, reference should be made to the following detailed description, taken in connection with the accompanying drawings, in which:
**FIG. 1** is a flow chart depicting the steps of a method of forming a collagen-integrated tropical composition, according to an embodiment of the present disclosure.
**FIG. 2** is a flow chart depicting the steps of a method of forming a collagen-free tropical composition comprising an analgesic, according to an embodiment of the present disclosure.
**FIG. 3** is a flow chart depicting the steps of a method of forming a collagen-free tropical composition, according to an embodiment of the present disclosure.
**FIG. 4** is a graphical representation of the change in the tottle surface temperature as a function of time when the hot fluid CS1i is cooling in a plastic tottle bottle, according to an embodiment of the present disclosure.
**FIG. 5** is a graphical representation of the cooling curve of K7LH5, according to an embodiment of the present disclosure.
**FIG. 6** is a graphical representation of the cooling curve of K7LH5 indicating the temperatures of phase transitions, according to an embodiment of the present disclosure.
**FIG. 7** is an image depicting treatment of a wound on a 79 year-old man before and after daily treatment for 45 days with K7LH5, according to an embodiment of the present disclosure, according to an embodiment of the present disclosure.
**FIG. 8** is an image depicting a suspension consisting of approximately 10¹⁰ colony forming units/ml (CFU/ml) for each bacteria, according to an embodiment of the present disclosure. Serial dilutions were made until a concentration of 10⁴ CFU/ml is achieved for all bacteria.
**FIG. 9** is a series of images depicting serial dilutions of the suspensions were plated onto specific media for these microorganisms using the Spiral Plater System that deposits a small amount (50 µl) of suspension over the surface of a rotating culture media agar plate to quantitate the exact concentration of viable organisms prior to the experiment, according to an embodiment of the present disclosure.
**FIG. 10** is a graphical exemplification of a methodology of EXAMPLE 15, disclosed herein below, according to an embodiment of the present disclosure.
**FIG. 11** is a graph depicting an area of inhibition zone of Methicillin Resistant Staphylococcus aureus MRSA USA300, bacteria concentration 10⁴, according to an embodiment of the present disclosure.
**FIG. 12** is a graph depicting an area of inhibition zone of Pseudomonas aeruginosa ATCC 27312, bacteria concentration 10⁴, according to an embodiment of the present disclosure.
**FIG. 13** is a graph depicting S. aureus MRSA USA300 bacterial counts after treatment application of topical compositions as compared to a baseline before debridement, according to an embodiment of the present disclosure. Statistical results comparison of treatments per assessment day.
**FIG. 14** is a graph depicting S. aureus MRSA USA300 bacterial counts after treatment application of topical compositions as compared to an untreated control and AQUACEL^{®} AG advantage, according to an embodiment of the present disclosure.
**FIG. 15** is a graph depicting the results of animals infected with Pseudomonas aeruginosa ATCC 27312 bacterial counts after treatment application of topical compositions as compared to a baseline before debridement, according to an embodiment of the present disclosure. Statistical results comparison of treatments per assessment day.
**FIG. 16** is a graph depicting the results of animals infected with Pseudomonas aeruginosa ATCC 27312 bacterial counts, measured at Day 4, Day 8, and Day 12 after treatment application of topical compositions as compared to an untreated control and AQUACEL^{®} AG advantage, according to an embodiment of the present disclosure.

### DETAILED DESCRIPTION OF THE INVENTION

In the following detailed description of the preferred embodiments, reference is made to the accompanying drawings, which form a part thereof, and within which are shown by way of illustration specific embodiments by which the invention may be practiced. It is to be understood that one skilled in the art will recognize that other embodiments may be utilized, and it will be apparent to one skilled in the art that structural changes may be made without departing from the scope of the invention. Elements/components shown in diagrams are illustrative of exemplary embodiments of the disclosure and are meant to avoid obscuring the disclosure. Any headings, used herein, are for organizational purposes only and shall not be used to limit the scope of the description or the claims. Furthermore, the use of certain terms in various places in the specification, described herein, are for illustration and should not be construed as limiting.

Reference in the specification to "one embodiment," "preferred embodiment," "an embodiment," or "embodiments" means that a particular feature, structure, characteristic, or function described in connection with the embodiment is included in at least one embodiment of the disclosure and may be in more than one embodiment. The appearances of the phrases "in one embodiment," "in an embodiment," "in embodiments," "in alternative embodiments," "in an alternative embodiment," or "in some embodiments" in various places in the specification are not necessarily all referring to the same embodiment or embodiments. The terms "include," "including," "comprise," and "comprising" shall be understood to be open terms and any lists that follow are examples and not meant to be limited to the listed items.

### Definitions

As used in this specification and the appended claims, the singular forms "a," "an," and "the" include plural referents unless the content clearly dictates otherwise. As used in this specification and the appended claims, the term "or" is generally employed in its sense including "and/or" unless the context clearly dictates otherwise.

As used herein, the terms "omega-3 fatty acids" and "omega-3 fats", "omega-3s", and "omega-3's", whether in the plural or in the singular, are synonymous and are used interchangeably and refer to the omega-3 fatty acids.

As used herein, the term "fish oil" refers to the oil extracted from fish carcasses or crustaceans or any other marine animal life. Examples of fish containing omega-3 fatty acids in its oil include salmon, tuna, swordfish, halibut, tilefish, cod fish, anchovies, green mussels, and sardines. Cold-pressed fish oil and/or heat-treated fish oils are subsumed under the term "fish oil." Non-limiting examples of crustaceans from which the oil may include krill, a crustacean in the Antarctic (the source of krill oil) and the New Zealand green lipped mussel (also known as *Perna canaliculus*)

As used herein, the terms "collagen-free" and "absence of collagen," "non-collagen" or synonyms thereto are used interchangeably, signifying that the composition contains no measurable amount of collagen and/or including collagen hydrolysate, from any animal

As used herein, the term "marine oil," comprises algal oil as well as fish oils. Some experts may consider marine oil as a drug and/or pharmaceutical; however, as used in the context of this application, and for purposes of this application, including cod liver oil, marine oil is not considered as an active pharmaceutical ingredient.

As used herein, the term "collagen" and "fish collagen" refers to any collagen known in the art comprising at least nine (9) amino acids. Non-limiting examples of collagen and/or fish collagen may be deep sea fish (e.g., white fish) collagen; skin collagen; bone collagen; skin and bone collagen, and/or collagen hydrolysate. For ease of reference, the exemplary embodiment described herein refers to collagen hydrolysate derived from marine oil and/or deep sea fish, but this description should not be interpreted as exclusionary of other collagens and/or fish collagens.

As used herein, the terms "algal oil" and "algae oil" are synonymous; these terms refer to oil made from certain marine or fresh water or farm-raised algae, such as algae grown in greenhouses or algae grown from any other source. Industrial sources of algae typically come from open recirculating ponds or from glass-enclosed growing containers (similar to greenhouses but are often vertical to better capture light). Open ponds are cheaper but are subject to contamination from wild algae. Glass-enclosed growing areas are used with specialty, high-efficiency algal strains. Useful oil is derived from marine vegetation, such as Marine algae, farm-raised algae, glass-raised algae and phytoplankton.

All of these fish oils and algae oil are sources of omega-3 fatty acids. These oils are extracted from the livers of the fish or from algae using techniques known in the art. For example, cod liver oil comes from cod fish, e.g., Atlantic cod (Gadus morhua) or from Pacific cod (Gadus microcephalus). Both Atlantic and Pacific cod liver oils are acceptable, but Pacific cod liver oil is preferred because the Bering Sea water is pristine with very little heavy metals. Pacific cod liver oil is obtained from wild, line-caught, adult cod. The immediately eviscerated liver, either from Pacific cod liver oil or Atlantic cod liver oil, is frozen on board to retain its nutrients. Frozen livers are transferred to a shore-side processing plant, rendered, and pressed into commercial cod liver oil with almost no odor. Nutritionally important omega-3 fatty acids present in fish and algae oil include stearidonic acid (SDA, C18:4) (18 carbon, 4 polyunsaturated fatty acids), Eicosapentaenoic acid (EPA, C20:5) (20 carbon, 5 polyunsaturated fatty acids) and docosahexaenoic acid (DHA, C22:6) (22 carbon, 6 polyunsaturated fatty acids).

As used herein, the terms "vegetable oil having an omega-3 fatty acid content greater than 9 wt%" and/or "vegetable oil having an omega-3 fatty acid content of at least 9 wt%" refer to vegetable oils having more than 9 wt% omega-3 fatty acids. The composition ranges of many vegetable oils are known. For example, canola oil may have an alpha-linolenic acid content comprising a range of at least 5 wt% to at most 15 wt%, encompassing every value in between. Flaxseed oil may have an alpha-linolenic acid content comprising a range of at least 50 wt% to at most 60 wt%, encompassing every value in between, and/or hempseed oil may have an alpha-linolenic acid content comprising a range of at least 15 wt% to at most 25 wt%, encompassing every value in between.

As used herein, the terms "hempseed oil" and "hemp oil" are synonymous. Hempseed oil may be prepared by pressing hemp seeds, such as hemp seeds that are cold-pressed. Unrefined hempseed oil is dark to clear light green in color with a nutty flavor

As used herein, the term "monoglyceride", unless indicated to the contrary, is a monoglyceride of the formula R-C(O)-O CH₂-CH(OH)-CH₂OH such that R may be an alkyl group containing 7-11 carbon atoms. The alkyl group may be linear or branched. The alkyl group does not contain any carbon-carbon multiple bonds, such as carbon-carbon double bonds or carbon-carbon triple bonds.

As used herein, the term "monolaurin" is also known as glycerol monolaurate, glyceryl laurate or 1-Lauroyl-glycerol. It is a monoglyceride. It is the mono-ester formed from glycerol and lauric acid. Its chemical formula is C₁₅H₃₀O₄. Glycerol monolaurate may also be identified as 2-Lauroyl glycerol. 1-Lauryoyl glycerol is preferred.

As used herein, the term "linear fatty acid ester," has the formula R1COOR2, such that R1 and R2 may be independently straight-chained alkyl groups, such that the sum of the number of carbon atoms of R1 and R2 may range from 9 to 49. For example, R1 and R2 may independently contain 1-49 carbon atoms, as long as the sum of R1 and R2 may range from 11-49. Examples are cetyl esters and waxes.

As used herein, the terms "cetyl esters" and "cetyl ester" are used interchangeably. These terms, as defined herein, refer to unbranched esters formed from cetyl alcohol and a C14, C16 or C18 fatty acid or mixture thereof. The fatty acids may be saturated or unsaturated. For example, non-limiting examples may comprise esters of C14, C16, or C18 fatty acid or mixtures thereof and cetyl alcohol. The fatty acids may be saturated or unsaturated. Cetyl Esters is a synthetic wax that has similar composition and chemical properties to a natural wax which is found in the spermacetti of sperm whales. The esters that are found in Cetyl Esters include cetyl palmitate, cetyl stearate, myristyl myristate, myristyl stearate, cetyl myristate, and stearyl stearate.

As used herein, the term "free fatty acid" refers to any added FFA, as well as the linear carboxylic acid that is formed after the triglyceride is hydrolyzed into free fatty acid and glycerin or into free fatty acid and a monoglyceride or diglyceride. Industrial hydrolysis is complete (e.g., free fatty acid and glycerin).

As used herein, the term "sea salt" refers to the salt produced from the evaporation of seawater. The sea salt used herein may be refined or unrefined. The colors and variety of flavors in sea salt are due to local clays and algae found in the waters from which the salt is harvested. For example, some boutique salts from Korea and France are pinkish gray, some from India are black. The chemical composition of sea salt is typically the same as the ions dissolved in seawater.

As used herein, the term "collagen" refers to a main protein component constituting connective tissue in animals that is characterized by having a collagen triple helical structure. A total of not less than 30 types of collagens have been reported which are respectively termed Type I, Type II, and so on. Type I collagen is the primary component of the derma, ligaments, tendons, bones and the like; and Type II collagen is the primary component of articular cartilage. Type III collagen is found in the skin, lungs, intestinal walls, and the walls of blood vessels. Further, Type IV collagen is mainly contained in a basal membrane, which is the undercoat of all epithelial tissues. Type I collagen is the most abundant collagen in the body. The term collagen includes the various types of collagens.

As used herein, the term "collagen-free" or synonym thereto also means that the compositions herein are also free of collagen hydrolysate (hereinafter referred to as collagen peptide), which is a low molecular weight (6-8kDa) collagen obtained by hydrolyzing, for example, collagen isolated from fish skins with an acid, alkali, or enzyme.

As used herein, the term "oils", when used alone, refers to sum total of all of the oils present in each of the compositions described herein.

As used herein, the term "vegetable oil," which is distinct from "vegetable oil having an omega-3 fatty acid content greater than 9 wt%", refers to vegetable oils containing no or less than or equal to 9.0 weight % of omega-3 fatty acids. Examples include coconut oil, corn oil, cottonseed oil, grapeseed oil, olive oil, palm oil, peanut oil, soybean oil, sunflower oil, cottonseed oil, and the like.

As used herein, the term "coconut oil" is a generic term that includes crude coconut oil and coconut oil that has been refined. Coconut oil is the raw minimally processed oil from coconuts, and as used herein, refers to "crude coconut oil" or sometimes "virgin coconut oil." Refined coconut oil is coconut oil that has been refined, bleached, and deodorized, and is referred to herein as refined or "RBD coconut oil." Refined coconut oil has a higher melting point than crude coconut oil. Unless indicated to the contrary, the term "coconut oil" includes both crude coconut oil and refined coconut oil.

As used herein, the term "palm olein" is synonymous with the term "palm oil" and both are used interchangeably. The term "palm oil" is the liquid portion which is separated from the semi-solid palm oil by fractionation. As used herein, the term includes red palm olein, and super red palm olein. The liquid portion is sold as cooking oils and the solid portion is known as "palm stearin." When palm olein is fractionated again to get a more liquid fraction, such as by chilling and removing the solid fraction of C18:0 (saturated C18 fatty acids) and some C16:0 (saturated C16 fatty acids), it is known as "super palm olein" or "CP6" (Cloud Point 6°C, meaning the fractionation took place at 6° C). Palm super olein is capable of withstanding colder temperature in comparison with palm olein after which they turn into solid. Palm olein is commonly used as cooking oil in the tropical countries. But the problem in temperate climate countries is that due to cold weather, it tends to get cloudy and crystallize. To overcome this problem, palm olein may be blended with more unsaturated vegetable oils. This blended form may be used in a wide range of climates and has a better cold stability. These blends are also cheaper than non-blended forms. The vegetable oils from rice bran (e.g., a wax), groundnuts and/or rapeseed may be blended with palm olein to get a superior form in terms of quality and stability,

As used herein, the term "anhydrous," refers to the water content of the composition of the present disclosure. As defined herein, the water content of the present composition refers to free water, that is, water not chemically bound to a substrate. As defined, the composition contains less than 5% by weight of free water or any range therein. A composition of the present invention may have a water content of less than 5wt% 5,%, by weight, for example, or 4.5, wt%, or, 4, wt%, or, 3.5, wt%, or, 3, wt%, or, 2.5, wt%,%, or, 2 wt%, or 1.5%, or 1.5, wt%, or 1, wt%, or %, 0.5 wt%, and/or any range in-between, by weight of the composition. Water content may be measured by methods known to those of skill in the art, such as, but not limited to, Karl Fischer titration.

As used herein, the term "homogenous," as it relates to a composition described herein, refers to the components in the composition that are substantially uniformly distributed throughout the composition.

As used herein, the term "penetration factor," refers to the sum by weight of the triglycerides of C8 and C10 saturated fatty acids (as triglycerides)/sum by weight of the other triglycerides, including saturated and unsaturated fatty acids larger than C10 present in a formulation of the present disclosure. Fatty acids are mainly composed of long chains of hydrocarbons ending in a carboxyl group. By definition, a fatty acid is a molecule of the formula R4COOH, such that R4 may be an organic group, such as a hydrocarbyl group, attached to the carboxyl group, COOH, containing an even number of carbon atoms and at least 6 carbon atoms and up to 30 carbon atoms. The carbon atoms may be a straight chain or branched or aromatic or a combination of aromatic and straight or branched chains. A fatty acid is saturated when the bonds between carbon atoms are all single bonds and is unsaturated when one or more of the bonds between the carbon atoms are unsaturated, e.g., carbon-carbon double or triple bonds or when the fatty acid contain an aromatic functionality. A triglyceride is by definition 3 fatty acids esterified to the three hydroxyl groups of a glycerol molecule (triacylglycerol). The fatty acids esterified to the glycerol molecule may be the same or different. Since the components used herein are natural products and/or are commercially available, the wt% of triglycerides of saturated fatty acids and triglycerides of unsaturated fatty acids in each composition may be easily determined by adding up the percentage by weight of the triglycerides of the saturated fatty acids and the unsaturated fatty acids in the various components present in each of the formulations. As a shorthand notation, it is referred to as (C8 +10)/(sum other triglycerides).

As used herein, the terms "Penetration ratio" and "Penetration factor, "permeation" " Penetration," "Permeation factor" or "permeation," "Penetration" and "Permeation ratio" are synonymous and are used interchangeably. This value may be calculated by an artisan of ordinary skill in the art without much difficulty

As used herein, the term "transdermal," when used alone or in combination, refers to complete passage, such as absorption, through the three layers of the skin for systemic distribution.

As used herein, unless indicated to the contrary, the terms "compositions" and "formulations" alone or in combination with other terms, such as for example, topical, are synonymous and may be used interchangeably. Further, as used herein, unless indicated to the contrary, the terms "composition" and "formulation" and "composition of the present disclosure" and "formulation of the present disclosure" and "compositions described herein" and "formulations described herein" and "topical compositions" and "topical formulations" and "topical formulations of the present disclosure" and "topical compositions of the present disclosure" and the like are being used interchangeably and synonymously.

As used herein, unless indicated to the contrary, the terms "drugs" and "medicament" and "active ingredient" are synonymous and are used interchangeably.

As used herein, the terms "Treatment" or "treating," refer to complete elimination as well as to any clinically or quantitatively measurable healing or alleviation of the symptoms of the wound or skin condition or burn.

As used herein, a "therapeutically effective amount" refers to the amount of a composition that, when administered to a subject for treating a wound, burn and/or skin condition is sufficient to effect a desirable treatment for the wound, burn and/or skin condition, respectively. The "therapeutically effective amount" will vary depending on the particular composition, the condition and its type and severity, and the age, weight, etc., of the subject to be treated. The actual amount, which comprises the "effective amount", will vary depending on a number of conditions including, but not limited to, the particular disorder being treated, the severity of the disorder, the size and health of the patient, and the route of administration. A skilled medical practitioner may readily determine the appropriate amount using methods known in the medical arts.

As used herein, the terms "Patient" or "subject" refers to animals, and may include any mammal, such as humans, rats, mice, cats, dogs, goats, sheep, horses, monkeys, apes, rabbits, cattle, bears, etc. The mammalian subject may be in any stage of development including adults, children, infants, and neonates.

As used herein, unless indicated to the contrary, percentages are by weight and ratios are weight ratios.

As used herein, the terms "components" and "ingredients" are synonymous and are used interchangeably.

As used herein, the terms "thickener" and "thickening agent" are synonymous and are used interchangeably.

As used herein, the terms "drug" and/or "pharmaceutical" are used interchangeably and refer to a medicine or other substance which has a physiological effect when applied topically to a subject used for treating a wound, burn and/or skin condition.

As used herein, the terms "about," "approximately," or "roughly" refer to being within an acceptable error range for the particular value as determined by one of ordinary skill in the art, which will depend in part on how the value is measured or determined (e.g., the limitations of a measurement system), (e.g., the degree of precision required for a particular purpose, such as producing a anhydrous topical composition). As used herein, "about," "approximately," or "roughly" refer to within ±20% of the numerical.

All numerical designations, including may range, are approximations which are varied up or down by increments of 1.0, 0.1, 0.01 or 0.001 as appropriate. It is to be understood, even if it is not always explicitly stated, that all numerical designations are preceded by the term "about". It is also to be understood, even if it is not always explicitly stated, that the compounds and structures described herein are merely exemplary and that equivalents of such are known in the art and may be substituted for the compounds and structures explicitly stated herein.

Wherever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Wherever the term "no more than," "less than," or "less than or equal to" precedes the first numerical value in a series of two or more numerical values, the term "no more than," "less than" or "less than or equal to" applies to each of the numerical values in that series of numerical values. For example, less than or equal to 1, 2, or 3 is equivalent to less than or equal to 1, less than or equal to 2, or less than or equal to 3.

### Anhydrous Topical Composition(s)

The present disclosure pertains to a combination therapy of at least two compositions, the first composition may comprise a collagen-free anhydrous topical pharmaceutical composition having at least two active pharmaceutical ingredients and having a penetration ratio of at least 1 to at most 3. In an embodiment, the composition may comprise marine oil, (e.g., fish oil or algal oil), which comprises ALA, SDA, EPA and/or DHA, vegetable oil having an omega-3 fatty acid content greater than 9 wt%, (e.g., hempseed oil, canola oil, or flax seed oil, linear fatty acid esters (e.g., cetyl esters and/or wax having 10-50 carbon atoms), monoglyceride of the formula R-C(O)-O CH2-CH(OH)-CH2OH (e.g., monolaurin where R may be an alkyl group of 7-11 carbon atoms), and/or medium chain triglycerides ("MCT"). In this embodiment, the topical composition may comprise monoglyceride in an amount of at least 6 wt%, and/or MCT in an of at least 40 wt%.

Moreover, in an embodiment, the topical composition may comprise a Penetration Ratio less than 1 and/or the topical composition may comprise at least one active pharmaceutical ingredient (e.g., ascorbyl palmitate). As such, the weight ratio of the sum of the weights of (ALA + SDA)/sum of the weights of (EPA and DHA) may be present in an amount comprising a range of at least 0.5 wt% to at most 21.5 wt%, encompassing every value in between. In this embodiment, the topical composition may also comprise collagen, the marine oil (e.g., fish oil and/or algal oil), which may also comprise ALA, SDA, EPA and/or DHA, vegetable oil having an omega-3 fatty acid content of at least 9 wt% (e.g., hempseed oil, canola oil, and/or flax seed oil), linear fatty acid esters (e.g., cetyl esters and/or wax having 10 - 50 carbon atoms), the monoglyceride of the formula R-C(O)-O CH2-CH(OH)-CH2OH (e.g., monolaurin, where R may be an alkyl group of 7-11 carbon atoms). In this embodiment, the topical composition may comprise monoglyceride in an amount of at most 6 wt%, and/or the topical composition may additionally comprise MCT. As such, the sum of the wt% of MCT, marine oil (e.g., cod liver oil), monoglyceride (e.g., monolaurin), linear fatty acid ester (e.g.,cetyl esters), vegetable oil having an omega-3 fatty acid content of at least 9 wt%, and/or ascorbyl palmitate may be at least equal to or greater than 70 wt%.

In an embodiment, both the topical composition comprising collagen and the collagen-free composition may be anhydrous and/or homogenous. As such, in this embodiment, the topical compositions may not exhibit an odor (e.g., a fish odor found in fish oil). Further when applied to the skin of a person, the both topical compositions may moisturize the skin (e.g., epidermis layer and/or dermis layer) of the person.

In an embodiment, the hempseed oil used herein may be refined, such that the hempseed oil may be substantially free of tetrahydrocannabinol. For example, in some embodiments, the hempseed oil may be manufactured from varieties of Cannabis *sativa* that are substantially free of tetrahydrocannabinol (THC). In the manufacturing process, THC may be removed prior to pressing the seed oil. In an embodiment, the hempseed oil of the topical composition may comprise at most 1% by weight of THC, and/or in an additional embodiment, at most 0.1% by weight THC, and/or, in still further embodiment, at most 0.01% by weight, if any, of THC.

In an embodiment, the THC of the hempseed oil of the topical composition may not be detectable, (e.g., at most 10 ppm). In addition, the hempseed oil may comprise gamma-linolenic acid (i.e., GLA), which may be an omega-6 fatty acid, as well as alpha-linolenic acid (i.e., ALA), which may be an omega-3 fatty acid. In this embodiment, the hempseed oil may also comprise at 14% oleic acid (e.g., C18:1), an omega-9 oil.

In an embodiment, the MCT of the topical compositions of the present disclosure may comprise a mixture of triglycerides of saturated C8 and/or saturated C10 fatty acids, such that the amount by weight of triglycerides of C8 saturated fatty acids present may be greater than or equal to the amount by weight of triglycerides of saturated C10 fatty acids present. It is to be understood that MCT may comprise additional triglycerides besides saturated C8 and/or saturated C10 triglycerides. As such, MCT may comprise a triglyceride backbone having attached thereto at least three saturated fatty acid chains that are generally in the range of at least C6 to at most C12 in length, although shorter and/or longer chains may be included within the term in differing contexts, as understood by those having ordinary skill in the art. In this manner, the longer and/or shorter chains may also be present in negligible amounts, for example, in some embodiments, usually less than 3% by weight of MCT. The three medium chain fatty acids that are attached to the triglyceride backbone of the MCT may be, but need not be, identical. Non-limiting examples of medium chain fatty acids that comprise the medium chain triglycerides described herein may comprise C6 (e.g., caproic fatty acid), C8 (e.g., caprylic fatty acid), C10 (e.g., capric fatty acid), and/or C12 (e.g., lauric fatty acid), as well as mixtures thereof.

In an embodiment, MCT of the topical composition may comprise a mixture ranging from at least 60 wt% saturated C8 triglycerides and/or at most 40 wt% C10 saturated triglyceride to a mixture of at most 70 wt% saturated C8 triglyceride and/or at least 30 wt% saturated C10 triglyceride. In another embodiment, the MCT of the topical composition may comprise a mixture of at least 51 wt% saturated C8 triglycerides and/or at most 49 wt% saturated C10 triglycerides to at most 70 wt% saturated C8 triglycerides and/or at least 30 wt% saturated C10 triglycerides by weight. In some embodiments, the MCT may comprise at least 55 wt% saturated C8 triglycerides and/or at most 45 wt% saturated C10 triglycerides to at most 65 wt% saturated C8 triglycerides and/or at least 35 wt% C10 triglycerides by weight. Further, as indicated hereinabove, the MCTs of the present disclosure may include minor amounts of triglycerides of short and/or long chain fatty acids, such as C6 and/or C4 fatty acids and/or C12 and/or C14 and/or C16 fatty acids. In this manner, the short fatty acids and/or long fatty acids may be present in minor amounts (e.g., at most 3 wt% by weight of MCT). In some embodiments, the MCT of the topical composition may not comprise triglycerides of a C12 fatty acid.

In an embodiment, in a mixture of C8 fatty acid and C10 fatty acid of the MCT of the topical composition, the amount of C8 fatty acid present by weight may be greater than or equal to the amount of C10 fatty acid present by weight. For example, in some embodiments, the weight ratio of C8 fatty acid to C10 fatty acid may range from at least 1.8 to at least 1.1, while in another embodiment it may range from at least 1.6 to at least 1.3. Capric/caprylic fatty acid is typically a natural product derived from coconut oil. Commercial product may range from at least 53% to at least 63% C8 and/or at least 47% to at least 37% by weight C10. Thus, in an embodiment, the weight ratio of C8 fatty acid to C10 fatty acid may range from at least 53:47 to at least 63:37, which may be a ratio ranging from at least 1.13 to at least 1.71.

In an embodiment, the topical composition may also comprise the following ions, present by dry weight percent, including but not limited to: (1) Sodium, at least 30.8 wt%; (2) Potassium, at least 1.1 wt%; (3) Magnesium, at least 3.7 wt%; (4) Calcium, at least 1.2 wt%; (5) Chloride, at least 55.5 wt%; and/or (6) Sulfate, at least 7.7 wt%. In this manner, sea salt, as the term is used herein, may comprise, as a minimum, those aforesaid ions. However, trace elements, such as titanium, silver, cobalt, and lead in synthetic sea salt may be much higher than those in sea water. The magnitude of the difference may be as large as 104 times. Unrefined sea salt may also comprise small amounts of magnesium and/or calcium halides and/or sulphates, traces of algal products, salt-resistant bacteria, and/or sediment particles. The calcium and/or magnesium salts may confer a faintly bitter overtone, and/or the calcium and/or magnesium salts may make unrefined sea salt hygroscopic (e.g., it gradually absorbs moisture from air if stored uncovered). Algal products may also contribute a mild "sea-air" smell, the latter from organobromine compounds. Sediments, the proportion of which varies with the source, give the salt a dull grey appearance. All of these different varieties of sea salts are contemplated by the term sea salt.

In an embodiment, the collagen of the topical composition comprising collagen (hereinafter "collagen-integrated topical composition") may be derived from marine life (e.g., cold-water fish). As such, the marine life may come from unpolluted water and/or may not have the diseases associated with land animals.

In an embodiment, the collagen used in the present disclosure may also comprise a Tg (e.g., glass transition temperature) of at most 37°C (e.g., normal body temperature). As such, any marine collagen from a cold-water source may be acceptable within the collagen of the topical composition. A non-limiting example may include fish collagen (e.g., deep sea white fish and/or cod). In this manner, in some embodiments, animal-derived collagen bovine, hog, and/or horse collagens may not be included in the collagen.

In addition, in an embodiment, the topical composition may also comprise a rice bran wax. In this embodiment, the rice bran wax of the topical composition may act as a thickener, binder, plasticizer, and/or gelling agent within the topical composition.

As indicated hereinabove, the topical composition of the present disclosure may be collagen-free (hereinafter "collagen-free topical composition"). By collagen-free, it is understood to mean that the composition may comprise no measurable amount of collagen.

In an embodiment, the collagen-free topical composition may comprise monoglyceride (e.g., monolaurin) having a the wt% of at least 6 wt%. In some embodiments, the amount of monoglyceride (e.g., monolaurin) may be present in the collagen-free topical composition comprising a range of at least 6 wt% to at most 14 wt%, encompassing every value in between. In some embodiments, the amount of monoglyceride (e.g., monolaurin) may be present in the collagen-free topical composition comprising a range of at least 7 wt% to at most 11 wt%, encompassing every value in between. In some embodiments, the amount of monoglyceride (e.g., monolaurin) may be present in the collagen-free topical composition comprising a range of at least 8 wt% to at most 10 wt%, encompassing every value in between. In some embodiments, the amount of monoglyceride (e.g., monolaurin) may be present in the collagen-free topical composition of at least 9.5 wt%.

As indicated hereinabove, in an embodiment, the collagen-integrated topical composition and/or the collagen-free topical composition (hereinafter "the topical compositions") of the present disclosure may be free of colloidal oatmeal (e.g., contains no measurable amount of colloidal oatmeal). As such, in this embodiment, the compositions may comprise MCT having a wt% of at least 50 wt% MCT and/or at least 60 wt% MCT. In an embodiment, the compositions comprising at least 60 wt% MCT may contain no colloidal oatmeal and/or a small amount of colloidal oatmeal (e.g., about 0% to at most 0.25 wt% colloidal oatmeal).

Values of the penetration ratio being less than 1.0 indicate that the composition is slow to penetrate the skin and remains on the surface of the skin for a long time. Penetration ratio greater than or equal to 3.0 indicates a quick penetration of the composition through the skin and values greater than or equal to 1 and less than 3 indicate an indeterminate penetration of the skin, such as minutes or hours to penetrate. The higher value indicates a faster penetration time, and a lower value indicates a slower penetration time.

Without wishing to be bound, it is believed that a penetration ratio greater than or equal to 1 up to and including 3 may be an indication that the composition is absorbed to the dermal space (e.g., is epidermal absorption and/or dermal absorption), while value greater than or equal to 3 may be an indication that the composition is absorbed to the transdermal space (e.g., transdermal absorption).

In an embodiment, the topical compositions of the present disclosure may exhibit "cosmetically elegant" non-sticky surface skin feel, while at the same time, the topical compositions of the present disclosure may also be capable of transferring important ingredients into the targeted layers of the skin. By "cosmetically elegant," it is meant that the composition may comprise the following: (1) may not have that greasy feeling, but may feel like a smooth lotion may be applied to the skin; (2) may be moisturizing in the absence of added moisture (via reducing TEWL); (3) may have little or no off odors (e.g., like rancid fat (e.g., oxidized), like "fishy" smell (e.g., trimethylamine)); (4) may be configured to penetrate the epidermis during rub-in; (5) may comprise a "silky-smooth" post-rub-in skin feel; (6) may comprise a long-lasting skin feel; and/or (7) may have little or no stickiness.

In an embodiment, the penetration factor of the present composition may have an effect of the properties of the composition. In an non-limiting example, it was found that incorporating an analgesic, such as, *cannabinoid, e.g.,* cannabidiol ("CBD"), into a composition comprising MCT (hereinafter "medium chain triglycerides") and/or waxes having a penetration factor greater than or equal to 1, but less than 3, may be very effective in alleviating pain. Such a composition may provide immediate and/or long-lasting relief from severe pain. In addition, in an embodiment, the topical compositions of the present disclosure may be used in the combination therapy. As such, in this embodiment, the topical composition may comprise a collagen-free anhydrous marine oil composition having MCT. Additionally, the topical composition may be comprised of at least two drugs.

In an embodiment, the topical composition may not contain rice bran wax and/or colloidal oatmeal. In this manner, the topical composition may be directed to an anhydrous topical composition comprised of marine oil, such as fish oil or algae, algal oil, vegetable oil having an omega-3 fatty acid content greater than 9 wt%, MCT (medium chain triglycerides), and/or waxes having a penetration factor greater than or equal to 1, but less than 3. In addition, in this embodiment, the topical composition may comprise hempseed oil, canola oil, or flax seed oil, monoglyceride (e.g., monolaurin), and/or linear fatty acid esters (e.g., cetyl esters). Accordingly, in this embodiment, the topical composition may be very effective in alleviating pain, based on the penetration factor comprising a range of at least 1 to at most 3, encompassing every value in between. As such, the topical composition may provide immediate and/or long-lasting relief from severe pain. Moreover, in some embodiments, the topical composition may also comprise ascorbyl palmitate

Various analgesics may be utilized in the topical compositions. For example, a series of drugs, such as Bupivacaine (e.g., Marcaine), Bupivacaine (e.g., Sensorcaine), Chloroprocaine (e.g., Nesacaine), Lidocaine (e.g., Xylocaine), Mepivacaine (e.g., Carbocaine), and/or the like; NSAIDS (e.g., ibuprofen, naproxen, diclofenac, celecoxib, mefenamic acid, etoricoxib, indomethacin., aspirin, and/or the like), morphine, camphor, and/or the like.

Cannabinoids are a group of substances found in the cannabis plant. Tetrahydrocannabinol (THC) and CBD are two examples of cannabinoid. As used herein, the term cannabinoid includes the natural product and the synthetic product.

In an embodiment, the topical compositions may comprise CBD. In some embodiments, analgesic drugs may also be used, as long as the drug is sufficiently oil soluble or sufficiently lipophilic. The term lipophilic may encompass compounds whose solubilities range from exclusive solubility in non-polar, water-immiscible organic solvents, to complexes having solubility both in these solvents and/or non-aqueous water immiscible solvents. The gradation of lipophilicity of the topical compositions of the present invention may be established by reference to partition coefficients using n-octanol/water, and/or n-octanol/buffer, and/or n-octanol/saline. In general, those analgesic drugs having n-octanol/saline partition coefficients of at least 10 and/or especially of at least 100 may be useful drugs for which the present compositions may be carriers. For example, in some embodiments, these drugs may be present in an amount ranging from at least 0.2% to at most 5 wt%, encompassing every value in between. In some embodiments, these drugs may be present in the amount of 0.5%.

Organic salts may not dissociate in anhydrous lipophilic compositions. For example, unexpectedly, it was found that benzethonium chloride may be soluble in lipophilic compositions when camphor is added at greater than 0.3%.

In an embodiment, the collagen-free topical composition and/or the collagen-integrated topical composition may comprise a penetration factor ranging from at least 1 to at most 3, encompassing every value in between. For example, in some embodiments, the penetration rate may comprise 1.1, or 1.2, or 1.3, or 1.4, or 1.5, or 1.6, or 1.7, or 1.8, or 1.9, or 2.0, or 2.1, or 2.2, or 2.3, or 2.4, or 2.5, or 2.6, or 2.7, or 2.8, or 2.9, or 3.0, or any value in between.

In an embodiment, the topical compositions of the present disclosure may comprise MCT, marine oil (e.g., fish oil and/or algae oil), vegetable oil having an omega-3 fatty acid content greater than 9 wt%, MCT (e.g., medium chain triglycerides), monoglyceride, (e.g., monolaurin), linear fatty acid esters (e.g., cetyl esters), and/or analgesic drug (e.g., cannabinoid (e.g., CBD)).

In an embodiment, MCT of the topical compositions may be present in an amount of at least 35 wt%. In some embodiments, MCT may be present in an amount ranging from at least 35 wt% to at most 65%. In some embodiments, MCT may be present in an amount ranging from at least 35 wt% to at most 75 wt%. In some embodiments, MCT may be present in an amount ranging from at least 40 wt% to at most 75 wt%. In some embodiments, MCT may be present in an amount ranging from at least 45 wt% to at most 75 wt%. In some embodiments, MCT may be present in an amount ranging from at least 50 wt% to at most 75 wt%. In some embodiments, MCT may be present in an amount ranging from at least 55 wt% to at most 75 wt%. In some embodiments, MCT may be present in an amount ranging from at least 60 wt% to at most 75 wt%. In some embodiments, MCT may be present in an amount ranging from at least 65 wt% to at most 75 wt%. In some embodiments, MCT may be present in an amount ranging from at least 70 wt% to at most 75 wt%. For example, in some embodiments, MCT may be present in 35 wt%, or 36 wt%, or 37 wt%, or 38 wt%, or 39 wt%, or 40 wt%, or 41 wt%, or 42 wt%, or 43 wt%, or 44 wt%, or 45 wt%, or 46 wt%, or 47 wt%, or 48 wt%, or 49 wt%, or 50 wt%, or 51 wt%, or 52 wt%, or 53 wt%, or 54 wt%, or 55 wt%, or 56 wt%, or 57 wt%, or 58 wt%, or 59 wt%, or 60 wt%, or 61 wt%, or 62 wt%, or 63 wt%, or 64 wt%, or 65 wt%, or 66 wt%, or 67 wt%, or 68 wt%, or 69 wt%, or 70 wt%, or 71 wt%, or 72 wt%, or 73 wt%, or 74 wt%, or 75 wt%, or any value in between.

In an embodiment, the MCT of the topical composition may vary and/or may be dependent on the function of the anhydrous collagen-free composition. If the function of the anhydrous collagen-free composition comprised of drugs is as a skin protectant (hereinafter "skin protectant topical composition"), which is used prior to the topical administration of the collagen composition and subsequent to the topical administration of the collagen composition after the closing of the skin, then, in this embodiment, the amount of MCT used may be at most 55 wt%. In some embodiments, the amount of MCT used may range from at least 40 wt% at most 52 wt%. In some embodiments, the amount of MCT used may range from at least 45 wt% to at most 48 wt%.

If, on the other hand, the collagen-free topical composition is also used as a therapeutic to reduce the size of the wound, burn and/or the size of the skin condition, then, in an embodiment, the amount of MCT present may be at least 55 wt%. More specifically, if this is the objective for adding MCT, then the amount of MCT present, in an embodiment, may range from at least 56 wt% to at most 75 wt%, and/or in another embodiment, from at least 60 wt% to at most 73 wt% and/or, in another embodiment, from at least 65 wt% to at most 68 wt%. In an embodiment, the MCT of the topical compositions may be present in about 1.5 to about 2 times the weight amount of the sum of the marine oil and vegetable oil having an omega-3 fatty acid content greater than 9 wt%.

The marine oil, e.g., cod liver oil, may be present in the composition of the present disclosure in an amount ranging from at least 1 wt% to at most 13.56 wt%, encompassing every value between, and/or in another embodiment, from at least 2 wt% to at most 13.56 wt%, encompassing every value in between, and/or in another embodiment from at least 2.2 wt% to at most 13.56 wt%, encompassing every value in between, and/or in another embodiment, from at least 12 wt% to at most 13.56 wt%, encompassing every value in between, and/or in another embodiment the marine oil may be present in the amount of at most 15 wt%. For example, in some embodiments, the marine oil, such as cod liver oil, may be present in 1.0 wt%, or 1.1 wt%, or 1.2 wt%, or 1.3 wt%, or 1.4%, or 1.5 wt%, or 1.6 wt%, or 1.7 wt%, or 1.8 wt%, or 1.9 wt%, or 2.0 wt%, or 2.1 wt%, or 2.2 %, or 2.3 wt%, or 2.4 wt. %, or 5.5 wt%, or 5.6 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or 4.1 wt%, or 4.2 wt%, or 4.3 wt%, or 4.4 wt%, or 4.5 wt%, or 4.6 wt%, or 4.7 wt%, or 4,8 wt%, or 4.9 wt%, or 5.0 wt%, or 5.1 wt, 5.2 wt%, 5.3 wt%, 5.4 wt%, or 5.5 wt%, or 5.6 wt%, or 5.7 wt%, or 5.8 wt%, 5.9 wt%, or 6.0 wt%, 6.1 wt%, or 6.2 wt%, or 6.3 wt%, or 6.4 wt%, or 6.5 wt%, or 6.6 wt%, or 6.7wt%, or 6.8%, or 6.9%, or 7.0 wt%, or 7.1 wt%, or 7.2 wt%, or 7.3 wt%, or 7.4 wt%, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 5.6 wt%, or 8.4 wt%, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt%, or 9.5 wt%, or 9.6 wt%, or 9.7 wt%, or 9.8 wt%, or 9.9 wt%, or 10.0 wt%, 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.5 wt%, or 13.6 wt%, 13.7 wt%, or 13.8 wt%, or 13.9 wt%, or 14.0 wt%, or 14.1 wt%, or 14.2 wt%, or 14.3 wt%, or 14.4 wt%, or 14.5 wt%, or 14.6 wt%, or 14.7 wt%, or 14.8 wt%, or 14.9 wt%, or 15.0 wt%, or any value in between.

The amount of marine oil present varies and is dependent on the function of the anhydrous collagen-free composition. If the function of the anhydrous collagen-free composition comprised of drugs is as a skin protectant (i.e., the skin protectant topical composition), which is topically administered prior to the usage of the collagen composition and subsequent to the use of the collagen composition after the closing of the skin, then the amount of marine oil present may range from 9 wt% to about 13.56 wt%, and/or in another embodiment, from about 11 wt% to about 13.56 wt% , and in a further embodiment, from about 12 wt% to about 13.56 wt%. If, on the other hand, the collagen-free composition is also used as a therapeutic to reduce the size of the wound, burn and/or skin condition, then the amount of marine oil present may range, in an embodiment, from 1 wt% to about 5 wt% and/or in another embodiment, from about 1.5 wt% to about 4 wt% and/or in another embodiment, from about 2 wt% to about 3 wt%.

In an embodiment, the weight ratio of the sum of the weights of (ALA + SDA)/sum of the weights of (EPA and DHA) (hereinafter the "PUFA ratio") present in the composition may range from about 0.5 to about 1.5 and/or in another embodiment, 0.75 to about 1.25, and/or in another ratio, from about 0.9 to about 1.1 and/or in another embodiment, the weight may be about 1.0.

In these formulations of the present disclosure, the function of the monoglyceride, such as monolaurin, when present, may be to help reduce the undesirable smell of the marine oil, such as cod liver oil. Without wishing to be bound, it is believed that the monoglyceride, such as monolaurin, may also help the marine oil be absorbed by the skin.

In a collagen-free, gelled embodiment, Monoglycerides, such as monolaurin may be present within the topical compositions. In an embodiment, to form a stable gel in, the monoglyceride, such as monolaurin, may be present in amounts of about 6 wt%, and/or greater than 6 wt%. In an embodiment, it may be present from about 6 to about 15 wt%, encompassing every value in between, and/or in another embodiment, from about 8 to about 12 wt%, encompassing every value in between, and/or in another embodiment from about 9 to about 11 wt%, encompassing ever value in between. In a collagen environment, Monoglycerides of the topical composition may be present in about 2 to about 5 wt% of the composition, encompassing every value in between. In a collagen-free oil embodiment, monoglycerides of the topical composition may be restricted by its inherent solubility to less than or equal to about 0.6%. In another embodiment, it may be present in about 0.6 wt%. For example, in some embodiments, monoglycerides, such as monolaurin, may be present in 0.01 wt%, or 0.1 wt%, or 0.2 wt% 0.3 wt%, or 0.4 wt%, or 0.5 wt%, or 0.6 wt%, 0.7 wt%, 0.8 wt%, or 0.9 wt%, or 1.0 wt%, or 1.1 wt%, or 1.2 1.3 wt%, or 1.4 wt%,%, or 1.5 wt%, or 1.6 wt%, or 1.7 wt%, or 1.8 wt%, or 1.9 wt%, or 2.0 wt%, or 2.1 wt%, or 2.2 t%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt. %, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt%, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt%, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt%, or 9.5 wt%, or 9.6 wt%, or 9.7 wt%, or 9.8 wt%, or 9.9 wt%, or 10.0 wt%, or 10.1, or 10.2 wt%, or 10.3 wt%, or 10.4 wt%, or 10.5 wt%, or 10.6 wt%, or 10.7 wt%, or 10.8 wt%, or 10.9 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.6 wt%, or 13.7 wt%, or 13.8 wt%, or 13.9 wt%, or 14.0 wt%, 14.1 wt%, or 14.2 wt%, or 14.3 wt%, or 14.4 wt%, or 14.5 wt%, or 14.6 wt%, or 14.7 wt%, or 14.8 wt%, or 14.9 wt%, or 15 wt%, or any value in between.

The amount of monoglycerides (e.g., monolaurin), present varies and is dependent on the function of the anhydrous collagen-free composition and if the composition may be a gel or oil. If the anhydrous collagen-free composition is to be an oil, then the monoglycerides, such as monolaurin, may be present in at least 3 wt%. In addition, if the function of the anhydrous collagen-free composition comprised of drugs is used as a skin protectant (i.e., the skin protectant topical composition), which is used prior to the administration of the collagen composition and/or subsequent to the use of the collagen composition after the closing of the skin, then the amount of monoglycerides, such as monolaurin, also may be present in at least 0.3 wt%. If, on the other hand, the composition may be a gel, then the monoglycerides, such as monolaurin may be present in at least 6 wt% and in an embodiment, from at least about 9 wt% to at most about 10.3 wt%, encompassing ever value in between, such as 9.5 wt% and/or 10.0 wt%. In addition, if the collagen-free composition is also used as a therapeutic to reduce the size of the wound, burn and/or skin condition, then the amount of monoglyceride, such as monolaurin present may range, in an embodiment, in amounts of 6 wt% and/or greater.

Without wishing to be bound, it is believed that monoglyceride, such as monolaurin, at concentrations of 6 wt% and/or greater than 6 wt%, slow the rate of absorption significantly, for example, by as much as 75% (versus collagen-free cod liver oil compositions as known in the art (e.g., WO 2019/036578)). In addition, in an embodiment, the collagen-free topical composition and/or the collagen-integrated topical composition may actually be penetration enhancers once through the stratum corneum and/or the remainder of the epidermis. In other words, these gelled formulations may be absorbed slowly through the stratum corneum and in the remainder of the epidermis. That is, the gels may be slow-to-release, but once in the epidermis, monoglycerides, such as monolaurin, may help drag the marine oil, such as cod liver oil and the vegetable oil having an omega-3 fatty acid content greater than 9 wt%, for example, hempseed oil into the dermis. Only when the concentration of monoglycerides, such as monolaurin, above or equal to 6 wt%, the gel may be metastable, that is, it is stable in a thick form, but with minor mixing may become a thin gel. With intense mixing, the gel may be broken. If broken gel is reheated to clarity, the process may begin again. In other words, the thick gel may be reproducible in all of the gel that is melted.

In addition, another ingredient that helps remove the odor in compositions described herein may be the linear fatty acid esters, especially cetyl esters. In an embodiment, the linear fatty acid esters, such as cetyl esters may be present in an amount ranging from about 0.5 wt% to about 7 wt%, encompassing every value in between, and/or in another embodiment, from about 1.0 wt% to about 6.5 wt%. In another embodiment, the linear fatty acid esters, such as cetyl esters, may be present in an amount ranging from about 1.2 wt% to about 6 wt% of the composition. For example, in some embodiments, linear fatty acid esters, such as cetyl esters, may be present in 0.5 wt%, or 0.6 wt%, or 0.7 wt%, or 0.8 wt%, or 0.9 wt%, or 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt. %, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or any value in between. Further, the addition of cetyl esters to the formulation creates a silky smooth skin surface (finish) that is very pleasant to the touch.

The amount of linear fatty acid esters, such as cetyl esters, which may be present varies and is dependent on the function of the anhydrous collagen-free composition. If the function of the anhydrous collagen-free topical composition comprised of drugs may be used as a skin protectant (i.e., the skin protectant topical composition), which is used prior to the administration of the collagen composition and subsequent to the use of the collagen composition after the closing of the skin, then the amount of linear fatty acid esters, such monoglycerides, such as monolaurin, present may comprise a range from about 0.5 wt% to about 2.5 wt%, encompassing every value in between and/or in another embodiment, from about 1% to about 2.0 wt%, encompassing every value in between. On the other hand, the if the collagen-free composition is also used as a therapeutic to reduce the size of the wound, burn and/or skin condition, then the amount of linear fatty acid esters, such as cetyl esters such present may range, in an embodiment, from about 4 wt% to about 8.7 wt%, encompassing every value in between, and/or in another embodiment, and/or in another embodiment, from about 4.5 wt% to about 7.0 wt%, encompassing every value in between, and/or in another embodiment, from about 5 wt% to about 6 wt%, encompassing every value in between.

With respect to the formulations described herein, without wishing to be bound, it is believed that the monoglycerides(e.g., monolaurin), may help drive the omega-3 fatty acids through the stratum corneum, leaving only a small residual of potentially oxidizable fat on the skin surface. The linear fatty acid esters, such as Cetyl Esters, without wishing to be bound, may provide a late-drying odor-occlusive layer over any potentially oxidizable fat. The net result may comprise no odor after at most 30 seconds.

A further means of reducing the fish oil in compositions described herein may be to use less of the marine oil, such as cod liver oil. Vegetable oil having an omega-3 fatty acid content greater than 9 wt%, such as hempseed oil, is another source of omega-3 fatty acids. Marine oil, such as cod liver oil, may provide very long chain omega-3 fatty acids (C> 18); vegetable oil having an omega-3 fatty acid content greater than 9 wt%, such as hempseed oil, may provide long chain omega-3 fatty acids (C=18). The body enzymatically takes omega-3 fatty acids and either shortens or lengthens the chain length to make, for example, prostaglandins. If the omega-3 fatty acid is C18 or less, the elongation process produces both inflammatory and anti-inflammatory compounds. If the omega-3 fatty acid is C> 18 (e.g., greater than or equal to C20), then the produced products are all anti-inflammatory.

In the topical compositions of the present disclosure, it is advantageous to use the vegetable oil having an omega-3 fatty acid content greater than 9 wt% (e.g., hempseed oil). In addition, for example, in an embodiment, the vegetable oil having an omega-3 fatty acid content greater than 9 wt% may be present in an amount ranging from about 1 wt% to about 40 wt%, encompassing every value in between, and/or in another embodiment, from about 1.5 wt% to about 40 wt%, encompassing every value in between. Additionally, the combination of marine oil and vegetable oil may provide the most effective ratios of C=18 + C> 18 of about 35 wt%, and/or in another embodiment, from about 1.0 wt% to about 30 wt%, encompassing every value in between. For example, in some embodiments, vegetable oil having an omega-3 fatty acid content greater than 9 wt%, (e.g., hempseed oil) may be present in 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt. %, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt. %, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt. %, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt. %, or 9.5 wt%, or 9.6 wt%, or 9.7 wt%, or 9.8 wt%, or 9.9 wt%, or 10.0 wt%, or 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.6 wt%, or 13.7 wt%, 13.8 wt%, or 13.9 wt%, or 14 wt%, or 15 wt%, or 16 wt%, or 17 wt%, or 18 wt% omega-3-fatty acids, or any value in between. However, there is also the trade-off between inflammatory byproducts and/or anti-inflammatory products with odor control. Nevertheless, in compositions described herein, the combination of C18 + C>18 omega-3-fatty acids may be configured to lower the odor of the collagen-free topical composition and/or the collagen-integrated topical composition. Thus, in some embodiments, the combination of C18 + C>18 omega-3-fatty acids may comprise greater than or equal to 19 wt%, or 20 wt%, or 21 wt%, or 22 wt%, or 23 wt%, or 24 wt%, or 25 wt%, 2 or 6 wt%, or 27 wt%, or 28 wt%, or 29 wt%, or 30 wt%, or 31 wt%, or 32 wt%, or 33 wt%, or 34 wt%, or 35 wt%, or 36 wt%, or 37 wt%, or 38 wt%, or 39 wt%, or 40 wt%, or any value in between.

The amount of vegetable oil having an omega-3 fatty acid content greater than or equal to about 9 wt%, (e.g., hempseed oil), such that less marine oil (e.g., cod fish liver oil) may be necessary. Accordingly, as described hereinabove, by utilizing more vegetable oil having an omega-3 fatty acid content of at least about 9 wt%, (e.g., hempseed oil), present varies and/or may be dependent on the function of the anhydrous collagen-free composition. If the function of the anhydrous collagen-free topical composition comprised of drugs is as a skin protectant (i.e., the skin protectant topical composition), which may be administered prior to the usage of the collagen composition and subsequent to the use of the collagen composition after the closing of the skin, then, in an embodiment, the amount of vegetable oil having an omega-3 fatty acid content greater than about 9 wt%, (e.g., hempseed oil) may range from about 9 wt% to about 40 wt%, encompassing every value in between, and/or in another embodiment, from about 16 wt% to about 35 wt%, encompassing every value in between, and in a further embodiment, from about 21 wt% to about 30 wt%, encompassing ever value in between. If, on the other hand, if the collagen-free topical is also used as a therapeutic to reduce the size of the wound, burn or the amount of skin condition, then the amount of marine present may range, in an embodiment, from about 1 wt% to about 5 wt%, encompassing every value in between, and/or in another embodiment, from about 1.5 wt% to about 4 wt%, encompassing every value in between, and/or in another embodiment, from about 2 wt% to about 3 wt%, encompassing every value in between.

In an embodiment, the weight ratio of vegetable oil having an omega-3 fatty acid content greater than 9 wt%, (e.g., hempseed oil) to marine oil (e.g., cod liver oil) is at least about 1:1. In this manner, in this embodiment, the weight ratio within present formulations may be configured to reduce and/or eliminate the smell of the marine oil. In an embodiment, the weight ratio may range from about 1:1 to about 2.5:1, encompassing every value in between and/or in another embodiment, from about 1.01 to about 2:1, encompassing every value in between, and/or in another embodiment, from about 1.01:1 to about 1.99:1, encompassing every value in between.

In an embodiment, vegetable oil having an omega-3 fatty acid content greater than 9 wt%, such as hempseed oil, may be present in an amount ranging from about 5 wt% to about 20 wt%, encompassing every value in between, and/or in another embodiment, from about 8 wt% to about 18 wt%, encompassing every value in between, and in a further embodiment, from about 12 wt% to about 16 wt%, encompassing every value in between, and/or in another embodiment, from about 13 wt% to about 15 wt%, encompassing every value in between, and/or in another embodiment, at about 14 wt%. For example, in some embodiments, vegetable oil having an omega-3 fatty acid content greater than 9 wt% may be present in 5 wt%, or 6 wt%, or 7 wt%, or 8 wt%, or 9 wt%, or 10 wt%, or 11 wt%, or 12 wt%, or 13 wt%, or 14 wt%, or 15 wt%, or 16 wt%, or 17 wt%, or 18 wt%, or 19 wt%, or 20 wt%, or any value in between.

In another embodiment, the weight of vegetable oil having an omega-3 fatty acid content greater than 9 wt% is%, such as hempseed oil, and the marine oil, such as cod liver oil may be present in greater than or a weight ratio of about 1:2 to about 2: 1, and/or in another embodiment, from about 1:1.5 to about 1.5:1, and/or in another embodiment, at about a 1:1 ratio (e.g., they are both present in about equal weight amounts to that of marine oil. For example, in some embodiments, the weight ratio of vegetable oil having an omega-3 fatty acid content greater than or equal to 9 wt%, e.g., hempseed oil to marine oil may range from 1.0 to about 1.1, and/or in another embodiment, from about 1.0 to about 1.07, and/or in another embodiment, from about 1.0 to about 1.05, and/or in another embodiment, from about 1.03 to about 1.05.

In the compositions described herein the relative amount of vegetable oil having an omega-3 fatty acid content greater than 9 wt%, such as hempseed oil, in amounts greater than (versus less than) marine oil depends on the need for a composition that promotes more inflammation or less inflammation. For already inflamed skin, less inflammation is preferable. For normal skin, a balance of inflammation and anti- inflammation is preferred because the body picks and chooses (enzymatically) the fatty acid chain length that needs conversion into more biologically active compounds. This may be a level of complexity impossible to predict, so a "buffet table" of fatty acid chain lengths may be offered. The body chooses what it needs for the particular individual. More hempseed oil (omega-6 rich) may increase inflammation; more fish oil (very long omega-3-rich) may reduce inflammation.

In an embodiment, an additional ingredient present in the formulation of the collagen-free topical composition may be ascorbyl palmitate ("AP"). It may be a stabilizing, high melting point wax that acts like an adhesive, ascorbyl palmitate may be present in about 0.2 to about 1.0 wt% of the composition, encompassing every value in between, and/or in another embodiment, it may be present in about 0 3 wt% to about 0.8 wt%, encompassing every value in between, and/or in another embodiment, in about 0.4 wt% to about 0.6 wt%, encompassing ever value in between, and/or in another embodiment, about 0.45 wt% to about 0.55 wt%, encompassing every value in between, and in a further embodiment, at about 0.5 wt%. For example, in some embodiments, AP may be present in 0.20 wt%, or 0.21 wt%, or 0.22 wt%, or 0.23 wt%, or 0.24 wt%, or 0.25 wt%, or 0.26 wt%, or 0.27 wt%, or 0.28 wt%, or 0.29 wt%, or 0.30 wt%, or 0.31 wt%, or 0.32 wt%, or 0.33 wt%, or 0.34 wt%, or 0.35 wt%, or 0.36 wt%, or 0.37 wt%, or 0.38 wt%, or 0.39 wt%, or 0.40 wt%, or 0.41 wt%, or 0.42 wt%, or 0.43 wt%, or 0.44 wt%, or 0.45 wt%, or 0.46 wt%, or 0.47 wt%, or 0.48 wt%, or 0.49 wt%, or 0.50 wt%, or 0.51 wt%, or 0.52 wt%, or 0.53 wt%, or 0.54 wt%, or 0.55 wt%, or 0.56 wt%, or 0.57 wt%, or 0.58 wt%, or 0.59 wt%, or 0.60 wt%, or 0.61 wt%, or 0.62 wt%, or 0.63 wt%, or 0.64 wt%, or 0.65 wt%, or 0.66 wt%, or 0.67 wt%, or 0.68 wt%, or 0.69 wt%, or 0.70 wt%, or 0.71 wt%, or 0.72 wt%, or 0.73 wt%, or 0.74 wt%, or 0.75 wt%, or 0.76 wt%, or 0.77 wt%, or 0.78 wt%, or 0.79 wt%, or 0.80 wt%, or 0.81 wt%, or 0.82 wt%, or 0.83 wt%, or 0.84 wt%, or 0.85 wt%, or 0.86 wt%, or 0.87 wt%, or 0.88 wt%, or 0.89 wt%, or 0.90 wt%, or 0.91 wt%, or 0.92 wt%, or 0.93 wt%, or 0.94 wt%, or 0.95 wt%, or 0.96 wt%, or 0.97 wt%, or 0.98 wt%, or 0.99 wt%, or 1.00 wt%, or any value in between.

In an embodiment, the weight of vegetable oil having an omega-3 fatty acid content greater than 9 wt% may be present in greater than or equal weight amounts to that of marine oil within the collagen-free topical composition. In an embodiment, the weight ratio of vegetable oil having an omega-3 fatty acid content greater than 9 wt% to marine oil may range from 1:0.1 to about 1:1, encompassing every value in between and/or in another embodiment, from about 1:0.1 to about 1:07, encompassing every value in between, and/or in another embodiment, from about 1:0.1 to about 1:0.5, encompassing every value in between, and/or in another embodiment, from about 1:0.3 to about 1:0.5, encompassing every value in between.

Another component of the collagen-free topical composition of the present disclosure may be used as a carrier for drugs useful for treating skin condition, wounds, or burns, as long as the drug is oil soluble and/or lipophilic. The term "lipophilic" as used in the invention implies that the drug is entirely purely lipophilic or has both a hydrophilic and lipophilic character, but more lipophilic character than hydrophilic character. The term lipophilic therefore encompasses solubilities. In general, those drugs having n-octanol/saline partition coefficients greater than about 10 and/or especially 100 may be useful drugs for which the present compositions, whether containing fish collagen or not, may be carriers, benzethonium chloride, methylbenzethonium chloride and benzalkonium chloride cocaine, ketamine, pramoxine, phenol, and pharmaceutically acceptable salts thereof. When present, the drug(s) in total may be present in a safe to be effective for and effective amount of a topical anesthetic. In an embodiment, the anesthetic drug is lidocaine. However, the API (Active Pharmaceutical Agent) is lipophilic. Other non-inclusive examples include lipophilic salts such as Amphotericin B and itraconazole. Further, the collagen-free compositions may be carriers for active ingredients other than anesthetics, such as benzethonium chloride, methylbenzethonium chloride and benzalkonium chloride, camphor, GABA,statins, such as valsartan; salicylic acid, for treating psoriasis, ethyl linoleate, as an anti-inflammatory or anti-bacterial agent or they may be carriers for dietary supplements, such as melatonin or glutathione, and the like.

One or more drugs may be present; however, one of the compositions, be it a collagen-free topical composition and/or collagen-integrated topical composition present, must have at least two drugs. In an embodiment, the weight ratio of the sum is the collagen-free composition that may comprise two and/or more drugs. The amount of each drug present may be a therapeutically effective amount of the weights of (ALA + SDA)/sum of the weights of (EPA and DHA) (hereinafter the "PUFA ratio") the present drug for whatever treatment required. For example, in some embodiments, in the composition(s), the total amount of drug present may range from about 0.5 wt% to about 1.5 wt%, encompassing every value in between, and/or in another embodiment from about 0.75 wt% to about 1.25 wt%, encompassing every value in between, and/or in another ratio, from about 0.9 wt% to about 1.1 wt%, encompassing every value in between, and/or in another embodiment, about 1.0 wt%.

The cannabinoid, such as CBD, may be present in analgesic effective amounts. In an embodiment, it may be present in an amount ranging from about 1 wt% to about 5 wt%, encompassing every value in between, and/or in another embodiment from about 1.75 wt% to about 4.0 wt%, encompassing every value in between, and/or in another embodiment, from about 2.0 wt% to about 4.0 wt%, encompassing every value in between, and/or in another embodiment, at about 3.7 wt%.

In addition, the composition may comprise additional optional ingredients, which will be discussed before the exemplification, but which is incorporated by reference.

All of the possible combinations and permutations of the components listed herein are contemplated to be within the scope of the present collagen-free compositions. However, the sum of all of the components present in the compositions herein adds to 100 wt%. In an embodiment, the sum of the weight % of MCT, marine oil, monoglycerides, linear fatty esters, vegetable oil having an omega-3 fatty acid content greater than 9 wt%, and the total amount of drug may range from about 80 wt% to about 100 wt%, encompassing every value in between, and/or in another embodiment, from about 85 wt% to about 100 wt%, encompassing every value in between, and/or in another embodiment, from about 90 wt% to about 100 wt%, encompassing every value in between, and/or in another embodiment, from about 92 wt% to about 100 wt%, encompassing every value in between. For example, in some embodiments, the sum of all the components may be present in total in 80 wt%, or 81 wt%, or 82 wt%, or 83 wt%, or 84 wt%, or 85 wt%, or 86 wt%, or 87 wt%, or 88 wt%, or 89 wt%, or 90 wt%, or 91 wt%, or 92 wt%, or 93 wt%, or 94 wt%, or 95 wt%, or 96 wt%, or 97 wt%, or 98 wt%, or 99 wt%, or 100 wt%, or any value in between.

Another embodiment of the present formulation may comprise an anhydrous topical pharmaceutical composition where collagen may be present. The collagen-integrated topical compositions may be in the form of an oil, gel, salve, paste, ointment, and/or whip. In an embodiment where collagen may be present, the anhydrous topical composition is comprised of marine oil, such as fish oil or algal oil; vegetable oil having an omega-3 fatty acid content greater than 9 wt%, such as hempseed oil, canola oil, or flax seed oil; monoglyceride, such as monoglycerides; linear fatty acid esters, such as cetyl esters and/or wax; medium chain triglycerides ("MCT"), and/or fish collagen and/or at least one drug and/or pharmaceutical, such that the weight ratio of the sum of the weights of (ALA + SDA)/sum of the weights of (EPA and DHA) may be present in the composition ranging from about 0.5 to about 1.5.

In an embodiment, the compositions of the present disclosure comprised of collagen (i.e., the collagen-integrated topical composition) may have a penetration values of less than or equal to 1.0, and/or in another embodiment, less than or equal to 0.5, but greater than 0. In an embodiment, the penetration value may range from about 0.2 to about 0.45, encompassing every value in between, in another embodiment, from about 0.25 to about 0.40, encompassing every value in between, and/or in another embodiment, from about 0.30 to about 0.40, encompassing every value in between. In an embodiment, the penetration value is 0.2, or 0.21, or 0.22, or 0.23, or 0.24, or 0.25, or 0.26, or 0.27, or 0.28, or 0.29, or 0.30, or 0.31, or 0.32, or 0.33, or 0.34, or 0.35, or 0.36, or 0.37, or 0.38, or 0.39, or 0.40, or 0. 41, or 0.42, or 0.43, or 0.44, or 0.45, or any value in between.

Collagen, as defined herein, may be present in an amount greater than or equal to 30 wt% of the composition. It may be present in the largest amount of any ingredient in the collagen-integrated topical composition. In an embodiment, collagen may be present in about 30 wt% to about 50 wt%, encompassing every value in between, and/or in another embodiment, from about 35 wt% to about 45 wt%, encompassing every value in between, and/or in another embodiment, from about 38 wt% to about 42 wt%, encompassing every value in between. For example, in some embodiments, it may be present in 30 wt%, or 31 wt%, or 32 wt%, or 33 wt%, or 34 wt%, or 35 wt%, or 36 wt%, or 37 wt%, or 38 wt%, or 39 wt%, or 40 wt%, or 41 wt%, or 42 wt%, or 43 wt%, or 44 wt%, or 45 wt%, or 46 wt%, or 47 wt%, or 48 wt%, or 49 wt%, or 50 wt%, or any value in between.

Where fish collagen (e.g., salt water fish and/or cold-water fish) may be present, in an embodiment, sea salt may or may not be present. If sea salt may be present, it may be present in amounts ranging from about 0.5 to about 2.5 wt%, encompassing every value in between, and/or in another embodiment, it may be present in amounts ranging from about 1 wt% to about 2.0 wt%, encompassing every value in between, and/or, in another embodiment, from about 1.3 wt% to about 1.7 wt%, encompassing every value in between. In an embodiment, it may be present in 0.5 wt%, or 0.6 wt%, or 0.7 wt%, or 0.8 wt%, or 0.9 wt%, or 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or any value in between.

The sea salt may be a process aid in an anhydrous matrix. In the present formulation, when present, it is added as ground sea salt. The average size of the sea salt crystal may comprise a range of at least about 0.4 mm to about at most 2.0 mm, encompassing every value in between. As described hereinbelow, the ground sea salt may be added after the collagen is mixed with the oil/wax mixture and cooled to room temperature. Sea salt may be added during a nitrogen-blanketed whipping. Sea salt acts to make the dry collagen/oil/nitrogen gas mix into freeze/thaw-stable extruded amorphous solid (e.g., putty).

The sea salt is a surprising process aid in an anhydrous matrix and/or solid. The sea salt must be ground and then post mixed after the collagen addition and cooling to <29°C (e.g.below that temperature where the monoglycerides first forms a gel). Sea salt acts to make the dry collagen/oil mix into extruded amorphous solid (e.g., putty). This physical characteristic may be implemented in treating non-healing wounds, such that the treatment may be optimized. Non-healing wounds may comprise unpredictable crevices and/or fissures. An anhydrous mixture and/or solid that may flow into all the wound surfaces may also be particularly advantageous for wound healing.

In the collagen-integrated topical composition, in an embodiment, the MCT may be present in an amount ranging from about 6 wt% to about 12 wt%, encompassing every value in between, and/or in another embodiment, from about 7 wt% to about 11 wt%, encompassing every value in between, and/or in another embodiment, from about 7.5 wt% to about 11.5 wt%, encompassing every value in between. For example, in some embodiments, MCT may be present in 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt. %, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt. %, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt. %, or 9.5 wt%, or 9.6 wt%, or 9.7 wt%, or 9.8 wt%, or 9.9 wt%, or 10.0 wt%, or 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or any value in between. Unlike the collagen-free containing composition, in the collagen-integrated topical compositions, the amount of MCT present may not be dependent on the function of the composition.

In the collagen-integrated topical composition, in an embodiment, the marine oil may be present in an mount ranging from about 6 wt% to about 13.6 wt%, encompassing every value in between, and/or in another embodiment, from about 7 wt% to about 13 wt%, encompassing every value in between, and/or, in another embodiment from about 8 wt% to about 12 wt%, encompassing every value in between, and/or in another embodiment, from about 9 wt% to about 11 wt%, encompassing every value in between. For example, in some embodiments, the marine oil, such as cod liver oil, may be present in 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt%, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt. %, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt. %, or 9.5 wt%, or 9.6 wt%, or 9.7 wt%, or 9.8 wt%, or 9.9 wt%, or 10.0 wt%, or 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.6 wt%, or any value in between. Unlike the collagen-free compositions, the amount of marine oil present is not dependent upon the function of the composition.

In an embodiment, MCT of the collagen-integrated topical composition may be present in an amount ranging from 50 wt% to about 70 wt%, encompassing every value in between, marine oil may be present in an amount ranging from about 5 wt% to 13.5 wt%, encompassing every value in between, monoglycerides may be present in 6.0 wt% to about 14.6 wt%, encompassing every value in between, cetyl esters may be present in an amount ranging from about 0.5 wt% to about 4 wt%, encompassing every value in between, vegetable oil having an omega-3 fatty acid content greater than 9 wt% may range from about 5 wt% to about 20 wt%, encompassing every value in between, and/pr ascorbyl palmitate may be present in an amount ranging from about 0.2 wt% to about 1.0 wt%, encompassing every value in between.

In another embodiment, in the collagen-integrated topical composition, MCT may be present in an amount ranging from 50 wt% to about 60 wt%, encompassing every value in between, marine oil may be present in an amount ranging from about 8 wt% to 13.6 wt%, encompassing every value in between, monoglycerides may be present in about 7 wt% to about 14 wt% but not less than 6 wt%, encompassing every value in between; cetyl esters may be present in an amount ranging from about 0.5 wt% to about 3 wt%, encompassing every value in between, vegetable oil having an omega-3 fatty acid content greater than 9 wt% may range from about 8 wt% to about 18 wt%, encompassing every value in between, and ascorbyl palmitate may be present in an amount ranging from about 0.3 wt% to about 0.8 wt%, encompassing every value in between. In a further embodiment, MCT may be present in an amount from about 51 wt% to about 55 wt%, but not less than 50 wt%, encompassing every value in between; marine oil may be present in an amount ranging from about 10 wt% to 13.7wt%, encompassing every value in between; monoglycerides may be present in about 8.5 wt% to about 10.5 wt%, encompassing every value in between; cetyl esters may be present in an amount ranging from about 0.8 wt% to about 2.2 wt%, encompassing every value in between; vegetable oil having an omega-3 fatty acid content greater than 9 wt% may range from about 12 wt% to about 16 wt%, encompassing every value in between; and/or ascorbyl palmitate may be present in an amount ranging from about 0.4 wt% to about 0.6 wt%, encompassing every value in between. In a still further embodiment, MCT may be present in an amount from about 52 wt% to about 54 wt%, but not less than 50 wt%, encompassing every value in between; marine oil may be present in an amount ranging from about 12 wt% to 13.8 wt%, encompassing every value in between; monoglycerides may be present in about 9 wt% to about 10 wt%, encompassing every value in between; cetyl esters may be present in an amount ranging from about 1 wt% to about 2 wt%, encompassing every value in between; vegetable oil having an omega-3 fatty acid content greater than 9 wt% may range from about 13 wt% to about 56 wt%, encompassing every value in between; and/or ascorbyl palmitate may be present in an amount ranging from about 0.45 wt% to about 0.55 wt%, encompassing every value in between.

The above discussion relates to the gels of the topical compositions that may not comprise colloidal oatmeal or rice bran wax. If the collagen-free topical composition and/or the collagen-integrated topical composition comprises either or both colloidal oatmeal and/or rice bran wax, the composition may be a topical anhydrous collagen-free gel. But there are some differences in the amount of some of the above ingredients.

With respect to monoglycerides (e.g., monolaurin), the comments and/or amounts described hereinabove are also applicable to these compositions. Further, the amounts of ascorbyl palmitate described hereinabove are also applicable to topical compositions, as described below. However, the amount of marine oil, cetyl esters and/or the vegetable oil having an omega-3 fatty acid content greater than 9 wt% and/or MCT may be different. Further, the penetration factor (e.g., diffusion rate) may be greater than or equal to 3.0.

In an embodiment in which the topical compositions may be comprised of colloidal oatmeal and/or rice bran wax, the amount of MCT present may range from 60 wt% to about 75 wt%, encompassing every value in between, and/or in another embodiment, from 60 wt% to about 73 wt%, encompassing every value in between, and/or in another embodiment, from 60 wt% to 70 wt%, encompassing every value in between. For example, in some embodiments, MCT may be present in present in 60 wt%, or 61 wt%, or 62 wt%, or 63 wt%, or 64 wt%, or 65 wt%, or 66 wt%, or 67 wt%, or 68 wt%, or 69 wt%, or 70 wt%, or 75 wt%, or 76 wt%, or 77 wt%, or 78 wt%, or 79 wt%, or 80 wt%, or any value in between.

In an embodiment of the topical compositions additionally comprised of colloidal oatmeal or rice bran wax, the amount of linear fatty acid esters, such as cetyl ester, may be present in amounts ranging from about 4 wt% to about 7 wt%, encompassing every value in between, and/or in another embodiment, from about 5 wt% to about 7 wt%, encompassing every value in between, and/or in another embodiment, from about 5.5 wt% to about 6.5 wt%, encompassing every value in between, and/or in another embodiment, from about 5.7 wt% to about 5.9 wt%, encompassing every value in between. For example, in some embodiments, it may be present in 4 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt%, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, 0 7.0 wt%, or 7.1 wt%, or 7.2 wt%, or 7.3 wt%, or 7.4 wt%, or 7.5 wt%, or 7.6 wt%, or 7.7 wt%, or 7.8 wt,%, or 7.9 wt%, or 8.0 wt%, or any value in between.

In an embodiment, the collagen-free topical composition may additionally be comprised of rice bran wax and/or colloidal oatmeal, the amount of marine oil present may range from about 1 wt% to about 4 wt%, encompassing every value in between, and/or in another embodiment, from about 1.5 wt% to about 3.5 wt%, encompassing every value in between, and/or in another embodiment, from about 2.0 wt% to about 3.0 wt% encompassing every value in between, and/or in another embodiment, from about 2.3 wt% to about 2.6 wt%, encompassing every value in between, and/or in another embodiment, from about 2.3 wt% to about 2.5 wt%, encompassing every value in between. For example, in some embodiments, the marine oil may be present in 1.0 wt%, or 1.1 wt%, or 1.2 wt%, or 1.3 wt%, or 1.4 wt, or 1.5 wt%, or 1.6 wt%, or 1.7 wt%, or1.8 wt%, or1.9 wt%, or 2.0 wt%, or 2.1 wt%, or 2.2 wt, or 2.3 wt%, or 2.4 wt%, or 2.5 wt%, or 2.6 wt%, or 2.7 wt%, or 2.8 wt%, or 2.9 wt%, or 3.0 wt, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or any value in between.

In an embodiment, the collagen-integrated topical composition may additionally be comprised of rice bran wax and/or colloidal oatmeal, the amount of vegetable oil having an omega-3 fatty acid content greater than 9 wt% may range from about 1 wt% to about 4 wt%, encompassing every value in between, and/or in another embodiment, from about 1.5 wt% to about 3.5 wt%, encompassing every value in between, and/or in another embodiment, from about 2.0 wt% to about 3.0 wt%, encompassing every value in between, and/or in another embodiment, from about 2.3 wt% to about 2.6 wt%, encompassing every value in between, and/or in another embodiment, 2.3 wt% to about 2.5 wt%, encompassing every value in between. For example, in some embodiments, the marine oil may be present in 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt% or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or any value in between.

In an embodiment, the function of the monoglyceride, such as monolaurin, when present, in the topical compositions described herein, may be to help reduce the undesirable smell of the marine oil, such as cod liver oil.

In an embodiment, the monoglyceride may be present from 0.1 to about 15 wt%, encompassing every value in between and/or in another embodiment, from about 2 wt% to about 6 wt%, encompassing every value in between. In another embodiment, the monoglyceride may be present from about 3 wt% to about 5 wt%, encompassing every value in between, and/or in another embodiment, from about 3.5 wt% to about 4.5 wt%, encompassing every value in between. For example, in some embodiments, it may be present in 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt. %, or 5.5 wt%, or 5.6 wt%, or 5.7 wt%, or 5.8 wt%, or 5.9 wt% up to but excluding 6.0 wt%, or any value in between. Unlike the collagen-free containing, the amount of monoglyceride present, such as monolaurin, may not be dependent upon the function of the composition.

However, in some embodiments, linear fatty acid esters, such as cetyl esters, may not be present. In an embodiment, if present, the linear fatty acid esters, such as cetyl esters may be present in an amount ranging from about 0.05 wt% to about 4 wt%, encompassing every value in between, and/or in another embodiment, from about 1.0 wt% to about 3 wt%, encompassing every value in between. In another embodiment, if present within the topical compositions, the linear fatty acid esters may be present from about 1.5 wt% to about 2.5 wt%, encompassing every value in between. For example, in some embodiments, linear fatty acid esters, such as cetyl esters may not be present, or if present, in 0.05 wt%, or 0.1 wt%, or 0.2 wt%, or 0.3 wt%, or 0.4 wt%, or 0.5 wt%, or 0.6 wt%, or 0.7 wt%, or 0.8 wt%, or 0.9 wt%, or 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt or.3.4 wt%, or 3.5 wt%, or 3.6 wt%, or 3.7 wt%, or 3.8 wt%, or 3.9 wt%, or 4.0 wt%, or any value in between. Further, the addition of linear fatty acids, such as cetyl esters, to the formulation creates a silky smooth skin surface (finish) that is very pleasant to the touch. Moreover, the amount of linear fatty acid, such as cetyl esters, is not dependent upon the function of the composition.

The Cetyl Esters without wishing to be bound, may provide a late-drying odor-occlusive layer over any potentially oxidizable fat. As such, the net result may be no odor after at most 30 seconds.

Marine oil, such as cod liver oil, may provide very long chain omega-3 fatty acids (C> 18); omega-3%, such as hempseed oil, may provide long chain omega-3 fatty acids (C=18). The body enzymatically takes omega-3 fatty acids and either shortens or lengthens the chain length to make, for example, prostaglandins. If the omega-3 fatty acid is C18 or less, the elongation process produces both inflammatory and anti-inflammatory compounds. If the omega-3 fatty acid is C> 18 (e.g., greater than or equal to C20), then the produced products are all anti-inflammatory. Wounds need some inflammatory compounds for example to control bleeding, as well as anti-inflammatory compounds, for example, to increase blood flow by reducing resistance to blood drainage.

In the collagen-integrated topical composition of the present disclosure, it may be advantageous to use vegetable oil (e.g., hempseed oil), in addition to marine oil (e.g., cod liver oil). This combination may provide the most effective ratios of C18 + C> 18 omega-3-fatty acids. However, there is also the trade-off between inflammatory byproducts and anti-inflammatory products with odor control. Nevertheless, in collagen compositions described herein, the combination of C18 + C>18 omega-3-fatty acids lowers the foul fish odor smell. Thus, by using a greater amount of vegetable oil having an omega-3 fatty acid content greater than 9 wt%, such as hempseed oil, less marine oil, such as cod liver oil, may be necessary. Accordingly, as described hereinabove, by utilizing more vegetable oil having an omega-3 fatty acid content greater than 9 wt%, such as hempseed oil, and reducing the amount of marine oil, such as cod liver oil, the present collagen formulations reduce the smell of the marine oil, such as cod liver oil. However, with the amount of monoglyceride, such as monolaurin and linear fatty acid esters, such as cetyl esters present herein, any fish smell of the marine oil, such as cod liver oil, is considerably reduced or eliminated. As described herein, the addition of marine oil, such as hempseed oil, linear fatty acid esters, such as cetyl esters and MCT and monoglyceride such as monolaurin, to the fish oil composition in the amounts described herein reduces or eliminates completely the fish odor, and when the composition may be applied to the skin of a patient, the burn and/or skin condition or wound is reduced in size and the skin of the patient is moisturized.

The vegetable oil having an omega-3 fatty acid content greater than 9 wt%, for example, hempseed oil, may be present in an amount ranging from about 7 wt% to about 16 wt%, encompassing every value in between, and/or in another embodiment, from about 10 wt% to about 14 wt%, encompassing every value in between, and/or in another embodiment, from about 11 wt% to about 13 wt%, encompassing every value in between. For example, in some embodiments, vegetable oil having an omega-3 fatty acid content greater than 9 wt%, (e.g., hempseed oil), may be present in 7.0 wt%, or 7.1 wt%, or 7.2 wt%, or 7.3 wt%, or 7.4 wt. %, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt %, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt. %, or 9.5 wt%, or 9.6 wt%, or 9.7 wt%, or 9.8 wt%, or 9.9 wt%, or 10.0 wt%, or 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.6 we%, or 13.7 wt%, or 13.8 wt%, or 13.9 wt%, or 14 wt%, or any value in-between. The amount of vegetable oil having an omega-3 fatty acid content greater than 9 wt%, for example, hempseed oil, which may be present, is not dependent upon the function of the composition.

In an embodiment, the weight ratio of vegetable oil having an omega-3 fatty acid content greater than 9 wt%, such as hempseed oil, to marine oil, such as cod liver oil, may comprise about 1:1. In an embodiment, the amount of omega-3 fatty acid content greater than 9 wt% is slightly greater than the amount of marine oil in the collagen-integrated topical composition, and the ratio may range from at least 0.8 wt% to at most 2.0 wt%, encompassing every value in between, and/or in another embodiment, from at least 0.9 wt% to at most 1.7 wt%, encompassing every value in between. In an embodiment, the topical compositions may comprise a colloidal oatmeal. As such, the colloidal oatmeal may be present in less than or equal to 0.25 wt%. In some embodiments, the colloidal oatmeal may be present in an amount ranging from 0.01 wt% to about 0.25 wt%, encompassing every value in between, and/or in another embodiment, from about 0.03 wt% to about 0.20 wt%, encompassing every value in between, and/or in some embodiments, may be present in 0.13 wt%, or 0.14 wt%, or 0.15 wt%, or 0.16 wt%, or 0.17 wt%, or 0.18 wt%, or 0.19 wt%, or 0.20 wt%, or 0.21 wt%, or 0.22 wt%, or 0.23 wt%, or 0.24 wt%, or 0.25 wt%, or any value in-between.

Another component of the collagen-integrated topical composition may comprise a drug and/or a pharmaceutical. In an embodiment, the composition of the present disclosure may form about 0.05 wt% to about 0.15 wt%, encompassing every value in between, and/or in another embodiment, from about 0.08 wt% to about 0.12 wt%, encompassing every value in between, and/or in another embodiment, at about 0.10 wt%. In this embodiment, colloidal oatmeal may be used as a carrier for drugs useful for treating skin condition, wounds, burns, as long as the drug is oil soluble lipophilic. As stated above, "lipophilic" as used in the invention may imply that the drug is entirely being purely lipophilic has both a hydrophilic and lipophilic character, but more lipophilic character than hydrophilic character. The term lipophilic therefore encompasses solubilities which range from exclusive solubility in non-polar, water-immiscible organic solvents, to complexes having solubility both in these solvents and non-aqueous water immiscible solvents. The gradation of lipophilicity of the compositions of the present invention may be established by reference to partition coefficients using n-octanol/water, n-octanol/buffer, n-octanol/saline. In general, those drugs having n-octanol/saline partition coefficients greater than about 10 and especially 100 may be useful drugs for which the present compositions, whether containing fish collagen or not, may be carriers. Examples of drugs include anesthetics, such as lidocaine, in pain killing effective amounts.

In an embodiment, the compositions of the present disclosure may contain a safe and effective amount of a topical anesthetic and/analgesic which numbs tissue and reduces pain associated with the skin condition and/or wound. Examples of topical anesthetic drugs include benzocaine, lidocaine, bupivacaine, chlorprocaine, dibucaine, etidocaine, mepivacaine, tetracaine, dyclonine, hexylcaine, procaine, benzethonium chloride, methylbenzethonium chloride and benzalkonium chloride cocaine, ketamine, pramoxine, phenol, and pharmaceutically acceptable salts thereof. Further, the collagen-free compositions may be carriers for active ingredients other than anesthetics, such as benzethonium chloride, methylbenzethonium chloride and benzalkonium chloride, camphor, GABA, statins, such as valsartan; salicylic acid, for treating psoriasis, ethyl linoleate, as an anti-inflammatory anti-bacterial agent they may be carriers for dietary supplements, such as melatonin glutathione, and/or any topical anesthetic known in the art. In an embodiment of the present disclosure, the composition may comprise any one or more of therapeutically active compounds and pharmaceutically and/or cosmetically acceptable diluents, excipients, and/or carriers. One or more drugs may be present in the collagen-integrated topical compositions. The amount of each drug present may be a therapeutically effective amount of the drug for whatever treatment it may be present for, For example, in some embodiments, the total amount of drug present may range from about 1.5 wt% to about 5 wt%, encompassing every value in between, and/or in another embodiment from about 1.75 wt% to about 4 0 wt%, encompassing every value in between, and/or in another embodiment, from about 2.0 wt% to about 3.3 wt%, encompassing every value in between.

In an embodiment in which the topical compositions may be comprised of rice bran wax, the rice bran wax may be present in amounts ranging from about 4 wt% to about 10 wt%, encompassing every value in between, and/or in another embodiment, from about 5 wt% to about 9 wt%, encompassing every value in between, and/or in another embodiment, from about 6 to 8 wt% and/or in another embodiment, at about 7 wt%. For example, in some embodiments, it may be present in 4.0 wt%, or 4.1 wt%, or 4.2 wt%, or 4.3 wt%, or 4.4 wt%, or 4.5 wt%, or 4.6 wt%, or 4.7 wt%, or 4.8 wt%, or 4.9 wt%, or 5.0 wt%, or 5.1 wt%, or 5.2 wt%, or 5.3 wt%, or 5.4 wt%, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt%, or 7.5 wt%, or 7.6 wt%, or 7.7 wt%, or 7.8 wt,%, or 7.9 wt%, or 8.0 wt%, or 8.1 wt%, or 8.2 wt%, or 8.3 wt%, or 8.4 wt%, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt%, or 9.5 wt%, or 9.6 wt%, or 9.7 wt%, or 9.8 wt%, or 9.9 wt%, or 10.0 wt%, or any value in-between.

All of the possible combinations and permutations of the components listed herein are contemplated to be within the scope of the present collagen-integrated topical compositions. However, the sum of all of the components present in the compositions herein adds to 100 wt%. In an embodiment, the sum of the weight % of MCT, marine oil, monoglyceride, such as monolaurin, linear fatty acids, such as cetyl esters, vegetable oil having an omega-3 fatty acid content greater than 9 wt%, collagen, and the total amount of drug and/or ascorbyl palmitate may range from about 75 wt% to about 100 wt%, encompassing every value in between, and/or in another embodiment, from about 80 wt% to about 100 wt%, encompassing every value in between, and/or in another embodiment, from about 85 wt% to about 100 wt%, encompassing every value in between, and/or in another embodiment, from about 90 wt% to about 100 wt%, encompassing every value in between.

For example, in some embodiments, in topical compositions comprising colloidal oatmeal and/or rice bran wax, MCT may be present in an amount ranging from 60 wt% to about 75 wt%, encompassing every value in between, marine oil may be present in an amount ranging from about 1 wt% to about 4 wt%, encompassing every value in between, monolaurin may be present in 6.0 wt% to about 14 wt%, encompassing every value in between, linear fatty acid esters, such as cetyl esters may be present in an amount ranging from about 4 wt% to about 7 wt%, encompassing every value in between, vegetable oil having an omega-3 fatty acid content greater than 9 wt% may range from about 1 wt% to about 4 wt%, encompassing every value in between, and/or ascorbyl palmitate may be present in an amount ranging from about 0.2 wt% to about 1.0 wt%, encompassing every value in between, colloidal oatmeal, if present, may be present in amounts ranging from about 0.01 to about 0.25 wt%, encompassing every value in between, and/or rice bran wax, if present, may be present in about 5 wt% to about 9 wt%, encompassing every value in between.

In another embodiment, MCT may be present in an amount from 60 wt% to about 73 wt%, encompassing every value in between, marine oil may be present in an amount ranging from about 1.5 wt% to 3.5 wt%, encompassing every value in between, monolaurin may be present in about 7 wt% to about 14 wt% but not less than 6 wt%, encompassing every value in between, linear fatty acid esters, such as cetyl esters, may be present in an amount ranging from about 5 wt% to about 7 wt%, encompassing every value in between, vegetable oil having an omega-3 fatty acid content greater than 9 wt% may range from about 1.5 wt% to about 3.5 wt%, encompassing every value in between, ascorbyl palmitate may be present in an amount ranging from about 0.3 wt% to about 0.8 wt%, encompassing every value in between, colloidal oatmeal, if present, may be present in amounts ranging from about 0.05 wt% to about 1.5 wt%, encompassing every value in between, and/or rice bran wax, if present, may be present in about 6 wt% to about 8 wt%, encompassing every value in between. In a still further embodiment, MCT may be present in an amount from 60 wt% to about 70 wt%, encompassing every value in between, marine oil may be present in an amount ranging from about 2 wt% to 3 wt%, encompassing every value in between, monolaurin may be present in about 9 wt% to about 10 wt%, encompassing every value in between, linear fatty acid esters, such as cetyl esters may be present in an amount ranging from about 5.5 wt% to about 6.5 wt%, encompassing every value in between, vegetable oil having an omega-3 fatty acid content greater than 9 wt% may range from about 2 wt% to about 3 wt%, encompassing every value in between, ascorbyl palmitate may be present in an amount ranging from about 0.45 wt% to about 0.55 wt%, encompassing every value in between, and/or colloidal oatmeal, if present, total in 75 wt%, or 76 wt%, or 77 wt%, or 78 wt%, or 79 wt%, or 80 wt%, or 81 wt%, or 82 wt%, or 83 wt%, or 84 wt%, or 85 wt%, or 86 wt%, or 87 wt%, or 88 wt%, or 89 wt%, or 90 wt%, or 91 wt%, or 92 wt%, or 93 wt%, or 94 wt%, or 95 wt%, or 96 wt%, or 97 wt%, or 98 wt%, or 99 wt%, or 100 wt%, or any value in between.

Although any vegetable oils having an omega-3 fatty acid content of 9 wt%, and/or less may be present in any of the formulations described herein, in an embodiment, these vegetable oils may be selected from the group including but not limited to palm oil (including, but not limited to red palm oil and RBD palm oil, and red palm concentrate), coconut oil, including refined coconut oil, and/or a combination of thereof. The vegetable oil (in total if more than 1 vegetable oil may be present in the formulation) may be present in an amount ranging from about 2 wt% to about 20 wt%, encompassing every value in between, and/or in another embodiment, from about 4 wt% to about 15 wt%, encompassing every value in between, and/or in another embodiment from about 6 wt% to about 10 wt%, encompassing every value in between, and/or in another embodiment, from about 7 wt% to about 8 wt% encompassing every value in between, and/or in another embodiment, at about 7.46 wt%. The vegetable oil may comprise one ingredient, such as palm oil, and/or a combination of two or more ingredients, such as coconut oil and/or palm oil. The vegetable oil may comprise one ingredient, such as palm oil, and/or a combination of two or more ingredients, such as coconut oil and/or palm oil. In an embodiment, coconut oil may be present in combination with palm oil. In this combination, the coconut oil may be present in amounts of 1 wt%, and/or less than 1 wt%, so that at least 75 wt% of the vegetable oil is palm oil, such as RBD palm oil. In this combination, the coconut oil may be present in about 0.2 wt% to about 0.5 wt%, encompassing every value in between, and/or in another embodiment, from about 0.25 wt% to about 0.40 wt%, encompassing every value in between, and/or in another embodiment, from about 0.3 wt% to about 0.35 wt%, encompassing every value in between, and/or in another embodiment, at about 0.33 wt%. Regarding the palm oil, in this combination, it may be present from about 3.5 wt% to about 19 wt%, encompassing every value in between, and/or in another embodiment, from about 4 wt% to about 15 wt%, encompassing every value in between, and/or in another embodiment, from about 5 wt% to about 13 wt%, encompassing every value in between, and/or in another embodiment, from about 6 wt% to about 10 wt%, encompassing every value in between, and/or in another embodiment from about 6.5 wt% to about 8 wt%, encompassing every value in between, and/or in another embodiment, from about 7 wt% to about 7.5 wt%, encompassing every value in between, and/or in another embodiment, at about 7.11 wt%.

Moreover, moisturizers may additionally be present in the collagen-free topical compositions and/or the collagen-integrated topical compositions. In an embodiment, lauric triglyceride and/or oleic triglycerides may be moisturizers that may be added to any of the formulations described herein. However, lauric triglyceride may be a better skin moisturizer than oleic triglyceride. In an embodiment of the present disclosure, the compositions (i.e., the collagen-free topical composition and/or the collagen-integrated topical composition) may comprise, in addition to the components described hereinabove, any one or more of therapeutically active compounds and pharmaceutically and/or cosmetically acceptable diluents, excipients or carriers. In an embodiment, the topical compositions (i.e., collagen-free topical composition and/or collagen-integrated topical composition) of the present disclosure may be used as a carrier for drugs useful for treating skin conditions or burns or wounds as long as the drug is oil soluble or lipophilic. Once more, as stated above, "lipophilic" as used in this disclosure may indicate that the drug is entirely being purely lipophilic and/or has both a hydrophilic and/or lipophilic character, but more lipophilic character than hydrophilic character. The term lipophilic therefore may encompass solubilities which range from exclusive solubility in non-polar, water-immiscible organic solvents, to complexes having solubility both in these solvents and/or non-aqueous water immiscible solvents.

The gradation of lipophilicity of the compositions of the present invention may be established by reference to partition coefficients using n-octanol/water, and/or n-octanol/buffer, and/or n-octanol/saline. In general, those drugs having n-octanol/saline partition coefficients greater than about 10 and especially 100 may be useful drugs for which the present compositions may be carriers. Examples of drugs include anesthetics, such as lidocaine, in pain killing effective amounts. In an embodiment, the compositions of the present disclosure may contain a safe and effective amount of a topical anesthetic and/or analgesic which numbs tissue and reduces pain associated with the skin condition or wound. Non-limiting examples of the topical anesthetic drugs may include benzocaine, lidocaine, bupivacaine, chlorprocaine, dibucaine, etidocaine, mepivacaine, tetracaine, dyclonine, hexylcaine, procaine, camphor, cocaine, ketamine, pramoxine, phenol, and pharmaceutically acceptable salts thereof. When present, in an embodiment, the drug may be present in a safe to be effective for an effective amount of a topical anesthetic. For example, in some embodiments, it may be present in an amount ranging from about 0.5% to about 1.5 wt%, encompassing every value in between. In an embodiment, the anesthetic drug is lidocaine. However, the API (Active Pharmaceutical Agent) is lipophilic. Other non-inclusive examples include lipophilic salts such as Amphotericin B and itraconazole. In another embodiment of the collagen and collagen-free containing composition, ascorbyl palmitate ("AP") may be additionally present.

In an embodiment, if ascorbyl palmitate is present, the ascorbyl palmitate may be present in about 0.3 to about 0.6 wt%, encompassing every value in between, of the composition, and/or in another embodiment, it may be present at about 0.5 wt%. It has a large head group such that when the ascorbyl palmitate precipitates, steric hindrance prevents it from plugging the collagen pores.

In addition, without wishing to be bound, it is believed that the ascorbyl palmitate stops and/or retards liquid leakage of the anhydrous components from the collagen and collagen-free matrices. The AP mixture clarifies at about 160°F (71.1°C). When fish collagen is added, the mixture is quenched below the AP freezing point. AP is shaped like a Tootsie Pop, so it is sterically hindered from entering the collagen pores. Instead, it is believed that the AP fatty esters coat (precipitate on) the outside of each collagen granule. Unlike AP, when beeswax or other thickening agents and the other linear esters precipitate, they are mobile greases and penetrate the interior pores of the collagen granule, displacing air.

Besides the components listed above, the collagen-integrated topical compositions and/or collagen-free topical compositions of the present disclosure may contain other additional optional ingredients. In an embodiment, the composition may contain fragrances and/or perfumes to further conceal any potential fish smell. If present, the perfumes, or fragrances may be present in less than or equal to 2 wt%, such as for example from about 0.01 wt% to about 1.5 wt%, encompassing every value in between. Oil soluble fragrances include such components as vanilla, lemon oil, lavender, and the like.

In addition, in an embodiment, if necessary, the collagen-integrated compositions and/or the collagen-free topical compositions of the present disclosure may contain additional thickeners, such as beeswax, candelilla wax, carnauba wax, ceresine wax, microcrystalline wax, ozocerite wax, PEG 4000, PEG 600, paraffin wax, laurel wax, rice bran wax, vegemite wax and other high melting point waxes and/or gelling agents (e.g., hydroxypropyl cellulose, carboxymethyl cellulose (CMC)). Colloidal oatmeal may additionally be present in any of the formulations described herein. Colloidal oatmeal is made by grinding oat grain or *Avena sativa* into a fine powder. It may be an emollient and has been categorized by the FDA in 2003 as a skin protectant if the concentration may be greater than 0.07 wt% (there is no regulatory upper limit). It also has anti-inflammatory and antioxidant properties. In an embodiment, it may be present in collagen-free formulations of the topical composition. In another embodiment, it may be present in collagen-integrated topical compositions. Oatmeal may not be included in oil compositions because of separation during storage. When present, it may be present in gels at amounts of less than or equal to about 1 wt%, such as for example, in some embodiments, from about 0.7 wt% to about 0.8 wt%, encompassing every value in between, and/or in another embodiment, from about 0.4 to about 0.6 wt%, encompassing every value in between, and/or in another embodiment in less than or equal to about 0.5 wt%. In another embodiment, it may be present at about less than or equal to 0.8 wt% and/or about less than or equal to 0.08 wt%, encompassing every value in-between.

In an embodiment of the present disclosure, compositions comprised of colloidal oatmeal may not contain cetyl esters because the oatmeal may provide the same tactical skin feel smoothness as cetyl esters. In addition, non-limiting examples of antioxidants may include, but are not limited to, tocopherols, ascorbic acid, Vitamin K, sodium pyrosulfite, butylhydroxytoluene, butylated hydroxy-anisole, eidetic acid, and edetate salts, and mixtures thereof.

A wide variety of optional components/ingredients may be included in the compositions of the present invention. In an embodiment, the compositions may include, but are not limited to absorbents, abrasives, anticaking agents, antifoaming agents, antimicrobial agents, binders, biological additives, buffering agents, bulking agents, chemical additives, biocides, denaturants, cosmetic astringents, drug astringents, external analgesics, film formers, humectants, opacifying agents, fragrances, pigments, colorings, essential oils, skin sensates, emollients, skin soothing agents, pH adjusters, plasticizers, preservatives, preservative enhancers, propellants, reducing agents, additional skin-conditioning agents, skin penetration enhancing agents, skin protectants, solvents, suspending agents, emulsifiers, thickening agents, solubilizing agents, sunscreens, moisturizing agents, such as squalene, sunblocks, ultraviolet light absorbers or scattering agents, sunless tanning agents, antioxidants and/or radical scavengers, chelating agents, sequestrants, anti-acne agents, anti-inflammatory agents, antiandrogens, depilation agents, desquamation agents/exfoliants, organic hydroxy acids, vitamins and/or derivatives thereof, and/or any natural extracts known in the art. Such other materials are known in the art. Non-limiting examples of such materials are described in Harry's Cosmedcology, 7th Ed., Harry & Wilkinson (Hill Publishers, London 1982); in Pharmaceutical Dosage Forms--Disperse Systems; Lieberman, Rieger & Banker, Vols. 1 (1988) & 2 (1989); Marcel Decker, Inc.; in The Chemistry and Manufacture of Cosmetics, 2nd. ad., deNavarre (Van Nostrand 1962-1965); and in The Handbook of Cosmetic Science and Technology, 1st Ed. Knowlton & Pearce (Elsevier 1993). The compositions described herein may be formulated as a gel, ointment, cream, balm, whip, and/or lotion. It may be administered in any one of those forms and/or administered as an oil-impregnated wipe and/or spray, such as an aerosol spray, including, but not limited to, a bag-on-valve aerosol spray. Topical administration may also be accomplished with a liquid spray, an aerosol, and/or via iontophoresis, and/or through the use of liposomes, microbubbles and/or microcapsules. Gels, ointments, and/or creams may be formulated with additives, for example, with an aqueous or oily base with the addition of suitable thickening (e.g., wax, beeswax, PEG 4000, PEG 600, hard paraffin) and/or gelling agents (e.g., hydroxypropyl cellulose). Lotions may be formulated with additives, such as an aqueous and/or oily base and/or may also generally contain one or more emulsifying agents (e.g., wool wax alcohol, fatty acid glycol esters). The gels of the present disclosure are anhydrous structured gels which are applied topically to the skin of a mammal, such as humans.

The gels may be formulated with stabilizing agents (e.g., polyoxyethylene sorbitan monolaurate, carboxy methyl cellulose), dispersing agents (e.g., sodium oleate, propylene glycol), suspending agents (e.g., methyl cellulose, chitosan, acacia, tragacanth, pectin), thickening agents, and/or coloring agents (e.g., dyes, lackes), or emollients, such as rice bran wax. In some embodiments, for example, the topical compositions may include Pluronic gels, poloxamer gels, hydrogels containing cellulose derivatives, including hydroxyethyl cellulose, hydroxymethyl cellulose, carboxymethyl cellulose, hydroxypropylmethyl cellulose and/or mixtures thereof; and hydrogels containing polyacrylic acid (carbopols). Embodiments may also include creams/ointments conventionally used for topical cosmetic and/or pharmaceutical preparations (e.g., creams based on cetomacrogol emulsifying ointment). The above carriers may include alginate (as a thickener or stimulant), preservatives such as benzyl alcohol, buffers to control pH, melatonin, disodium hydrogen phosphate/sodium dihydrogen phosphate, agents to adjust osmolarity such as sodium benzethonium, and stabilizers methylbenzethonium chloride and benzalkonium chloride, GABA, statins, such as valsartan; and/or they may be carriers for dietary supplements, (e.g., EDTA); Oil soluble anti-oxidizers, such as Vitamin E, and/or astaxanthin. Additionally, in an embodiment, oil soluble vitamins, such as Vitamin D may be added. Oil soluble fragrances such as vanilla, lemon oil and lavender and/or oil-soluble coloring agents may also be added. In addition, sebum may be added. In an embodiment of the present disclosure, the composition may comprise any one or more of therapeutically active compounds and pharmaceutically and/or cosmetically acceptable diluents, excipients, and/or carriers known in the art.

In an embodiment, the following other additives may be present in the formulation described herein. As such, in this embodiment, antioxidants and/or preservatives may be added. Non-limiting examples of antioxidants include, but are not limited to, tocopherols, ascorbic acid, Vitamin K, Vitamin E, sodium pyrosulfite, butylhydroxytoluene, butylated hydroxyanisole, eidetic acid, and edetate salts, squalene, and/or mixtures thereof. Non-limiting examples of preservatives include, but are not limited to, citric acid, tartaric acid, lactic acid, malic acid, acetic acid, benzoic acid, and/or sorbic acid, and/or mixtures thereof.

In an embodiment, the formulation of the topical compositions may be an oil in one embodiment, a gel in another embodiment, a foam, a salve (where salves have a specific gravity greater than 0.8 lbs./ft³), a lotion, and/or a whip (where whip formulations have a specific gravity less than or equal to 0.8 lbs/ft³) in other embodiments. As described hereinabove, to form a gel, the monoglyceride may be present in at least 6 wt% of the collagen-free topical compositions of the present disclosure. In an embodiment of the present disclosure, the collagen-free topical compositions may be in the form of a gel and/or oil, while the collagen-integrated topical compositions may be in the form of an oil.

In an embodiment, the collagen-free topical composition and/or the collagen-integrated topical composition of the present disclosure may be prepared by using techniques known in the art. In this embodiment, for the collagen-free compositions in the form of a gel, all of the components may be mixed together in a vessel at high temperatures until homogenous, (e.g., the ingredients are substantially uniformly distributed in the vessel). In an embodiment, all the ingredients may be mixed gently without visible vortex at temperatures greater than or equal to about 165°F (melting point without AP), such as a temperature ranging from about 165°F to about 200°F, encompassing every value in between, and/or in another embodiment from about 180°F to about 190°F, encompassing every value in between, and/or in another embodiment, from about 175°F to about 200°F, encompassing every value in between, and/or in another embodiment from about 185°C to 195°C, encompassing every value in between, if ascorbyl palmitate is not present in the composition. If ascorbyl palmitate is present in the composition, in an embodiment, the ingredients may be mixed gently without visible vortex at temperatures greater than or equal to about 185°F (melting point with AP and/or benzethonium chloride) to about 200°F, encompassing every value in between, and/or in another embodiment, from about 185°F to about 195°F, encompassing every value in between. The vessel may be any appropriate glass container, such as glass jar or beaker, a tottle, which may be a bottle shaped like a tube, a round bottom flask or the like. After the mixture is homogenous, the mixture may be allowed to cool to room temperature. In some embodiments, this may be typically overnight, for example 12 hours. As it is cooling, phase separation may occur. For example, in these other embodiments, after the mixture is cooled, the mixture may have crystals present in floating oil (e.g., a fat/wax mixture which shall be referred to as "mud"). The mud may also be reheated to a temperature sufficient to melt the-mud, but not melt the AP to form an almost clear liquid. In an embodiment, the mud may be heated with gentle stirring such as about 2 to about 4 rpm, encompassing every value in between, and especially 3 rpm in a standard paddle mixer, to a temperature ranging from about 115°F to about 120°F, encompassing every value in between, in another embodiment, from about 115°F to about 130°F, encompassing every value in between, and/or, in another embodiment, from about 115°F to about 200°F, encompassing every value in between, and/or in another embodiment, from about 118°F to about 200°F, encompassing every value in between, from about 123°F to about 200°F, encompassing every value in between. In an embodiment, when the clear liquid is formed, it may be pumped into a filling machine, which then fills the mixture into a sealed container, such that the liquid may cool upon standing.

In an embodiment, the non-gel topical compositions (e.g., collagen-free topical composition and/or collagen-integrated topical composition) described herein may be prepared by techniques known in the art (e.g., U.S. Patent No. 10,463,699 and/or WO 2019/200364), the contents of which are incorporated by reference, in their entireties. In this embodiment, the collagen-free containing formulations which are oils may be prepared by mixing the various components at slightly elevated temperatures, such as about 30°C to about 60°C, encompassing every value in between, and/or in another embodiment, at about 35°C to about 50°C, encompassing every value in between and/or in another embodiment, at about 38°C to about 43°C, encompassing every value in between, until the resulting mixture is homogenous, and then allowing the mixture to cool to room temperature. In an embodiment, the compositions comprised of collagen, all of the components except the collagen and optional sea salt and any fragrances may be mixed together with stirring at a temperature sufficient to homogenously melt the components and form a homogenous paste. Typically, the composition may be heated at temperatures ranging from about 130°F to about 200°F, encompassing every value in between, in an embodiment, and/or in another embodiment from about 150°F to about 170°F, encompassing every value in between and/or in another embodiment at about 140°F. When the resulting mixture is homogenous, collagen is added at temperatures greater than 130°F, for, example at temperatures at about 150°F to about 170°F, encompassing every value in between, and the collagen may be mixed in with the other components. When substantially homogenous, in an embodiment, when the pores of the collagen are filled with oils, which may take about ten to about twenty minutes, the composition may be cooled to room temperature. Unexpectedly, benzethonium chloride, a halide salt that is not soluble in oil, may become soluble at about 185°F only when camphor is in the formulation. In an embodiment, this may provide the unexpected benefit of a salt that is well-known to never penetrate the stratum corneum to pass through the stratum corneum as a solute into the epidermis. In an embodiment, the oils may be added in an inert atmosphere, such as in the presence of an inert gas, such as nitrogen. In some embodiments, the composition may again be mixed until the composition is substantially homogenous, including incorporation of inert gas to form a collagen matrix with specific gravity <0.8.

In an embodiment, a composition that may comprise the stabilized formulation may be formulated for topical application as described herein and sealed in an air-tight container suitable for a single or multi use. Such a composition may be used to treat any of the skin conditions, burns and/or wounds described herein. The compositions of the present disclosure are gels or may be made into creams or lotions.

In an embodiment, the compositions may be useful for alleviating pain, especially severe pain, such as chronic pain. Chronic pain may be defined as pain that is unbearable to the subject. For example, the pain may be so unbearable to the subject that it limits a subject's strength and endurance and creates a challenge for a person to perform daily tasks and activities. The pain may feel sharp or dull, but lasts a long time, such as days, weeks, or months. It may be a chronic pain. It may cause a burning or aching sensation in the affected areas. Examples include, but are not limited to, Arthritis, or joint pain, Back pain, Neck pain, Cancer pain near a tumor, Headaches, including migraine, Testicular pain (orchialgia) Lasting pain in scar tissue, Muscle pain all over (such as with fibromyalgia), and the like.

In an embodiment, the combination of one or more collagen compositions and one or more collagen free compositions when applied topically may treat sacral wounds and/or heal ulcers and/or wounds. Further, unexpectedly, the combination of one or more collagen compositions and one or more collagen free compositions of the present disclosure when applied topically may not visibly scar after application of the formulation described herein to the wound, burn and/or skin condition.

In an embodiment, unexpectedly, when benzethonium chloride is heated with marine oils and vegetable oils with more than 9% PUFA and/or camphor greater than or equal to 0.3wt%, the lipid-insoluble halide salt becomes soluble in an anhydrous medium. For example, in some embodiments, the compositions may be produced by heating benzethonium chloride with 3% camphor into a clear liquid that remained clear even after freeze/thaw stress (e.g., 24 hours at about 2°C, then about 25°C). The 5-minute gap may allow time for the wound to become numb. What is different is that benzethonium chloride may be deposited into the infected tissue with at least one collagen free topical composition and/or the collagen-integrated topical composition, while benzethonium chloride retains residual, surface first aid antisepsis with the at least one collagen-free topical composition and/or the collagen-integrated topical composition. In other words, the oil may drag a biologically relevant salt into the epidermis to kill the infection; the collagen-integrated topical composition may also be configured to cover the wound with the same salt to prevent fresh infection entering the wound. The combination may kill at least one pathogen and/or prevent a wound from turning septic.

Practically, in an embodiment, a wound treatment may be executed by first disposing a collagen-free topical composition at the wound site, in which the collagen-free topical composition may comprise a combination of Penetration Ratio greater than or equal to 3 and/or an Omega-3 sum greater than or equal to 4. Next, in this embodiment, the wound site may be post treated with the collagen-integrated topical composition (e.g., the amorphous solid (e.g., putty)) comprising a Penetration Ratio of less than or equal to 1.0 and/or an Omega-3 sum greater than or equal to 4.0.

In some embodiments, the collagen-free topical composition may comprise at least 3% camphor, a quick acting topical analgesic, and/or 0.7% lidocaine, a slower acting analgesic. Camphor may be added not to numb the wound site, to prevent pain sensation when "unused" microcircuits suddenly have a rush of blood, to pressurize the vessel, and/or to temporarily increase the diameter of the previously compressed microcircuits. The pulse of blood flow and/or the change in vessel diameter may cause temporary pain. After the pulse passes, the pain may be inhibited.

Wound healing is evolutionarily optimized for speed of healing under dirty conditions, where a rapid inflammatory response with overlapping cytokine and inflammatory cascades allow the wound to heal quickly to prevent infection and future wound breakdown.

In an embodiment, the collagen-free topical composition and/or the collagen-integrated topical composition of the present disclosure both have a characteristic in common; the sum of the weights of the omega-3 fatty acids in these compositions may be greater than or equal to 4. In addition, the collagen-free compositions have a penetration ratio greater than or equal to 3, while the collagen-integrated topical compositions have a penetration ratio (e.g., diffusion rate) less than 1.0.

In an embodiment, the topical compositions (e.g., the collagen free-topical composition and/or the collagen-integrated topical composition) of the present disclosure may minimize the formation of scars in the process of treating wounds, burns, and/or skin conditions. In this embodiment, the topical compositions may be configured to retard and/or inhibit the formation of at least one scar known in the art in the process of treating wounds, at least one burn known in the art, and/or at least one skin condition known in the art. Scars arise after almost every dermal injury. Scars may be disfiguring and aesthetically unpleasant and cause severe itching, tenderness, pain, sleep disturbance, anxiety, depression, and disruption of daily activities. In an embodiment, the collagen-free topical composition may comprise a low penetration factor, such that the collagen-free topical composition may be configured to accelerate angiogenesis and/or minimize scarring. As such, in an embodiment, the penetration factor (>3.0) and the ratio of (ALA+SDA)/(EPA+DHA) may be between about 0.5 to 1.5, encompassing every value in between, in the anhydrous gel collagen-free topical composition, such that the collagen-free topical composition may be among the controlling factors to minimize scarring.

Without wishing to be bound, it is believed that compositions in the present disclosure may stop the excess growth of collagen-boosted granulation by simultaneously encouraging epithelial growth using omega-3 skin protectant oils and/or gels (i.e., the skin protectant topical composition). The change may be subtle because a monolayer of epithelial tissue covers the wound (e.g., invisible to the naked eye except for a visible gloss (e.g., caused by fat-rich light reflection) over the wound bed). The epithelial monolayer may separate added collagen from the wound bed. It is important to note that the epithelial tissue has a negative charge, and/or the wound bed has a positive charge that electrostatically "pulls" epithelial cells over the wound bed. In an embodiment, sea salt may be added to the collagen-integrated topical compositions, such that the collagen-integrated topical composition may be configured to increase exudate conductivity and/or thereby encourage electrical-attraction migration.

With gels of the present disclosure, without wishing to be bound, it is believed that transfer through the epidermis is slowed by using the compositions having a penetration factor greater than 1 and less than or equal to 3, encompassing every value in between. In an embodiment, the slowness of the gel collagen-free topical composition and/or gel collagen-integrated topical composition may allow time for some of the gel to cross the epithelial barrier via the hair follicle bulb. The follicle bulb has at least 3 "bulges" where stem cells rest until called for. The stimulated wound signals for stem cell help. The gels in the follicle may provide necessary building blocks for growth of stem cell progeny. Progeny exit the follicle and/or may be configured to migrate across the basement membrane onto the surface of the granulating bed. There are also sporadic stem cells in the intrafollicular epithelium in the basal cells above the basement membrane. The gels of the present disclosure stimulate epithelial stem cell progeny migration. Follicular progeny mixes with basal cell progeny and jointly migrate to the peri wound and onto the surface of the developing granulation bed. Electrical attraction helps distribute mixed progeny over the entire wound bed surface, preventing topical collagen from overgrowing the wound bed. Retentate saturated fats "grease" the pathways to allow stem cells to migrate rapidly over the emerging new granulation tissue. Once the new epithelial tissue has covered the emerging new granulation tissue, granulation growth stops.

Without wishing to be bound, it is believed that cells may absorb various compositions at a finite rate. Compositions that oversaturate the cells then passes by the satiated cell and into the blood stream. By using the gels described in the present disclosure to slow the oil passage by an epidermis cell and/or dermis cell, more of the gelled composition is absorbed by epidermis cells and/or dermis cells and less passes by into the bloodstream. The net effect is to feed more gelled composition to epidermis cells and/or dermis cells.

In an embodiment, by deliberately slowing the rate of absorption and/or increasing the absolute amount of topical omega-3 and topical MCT with gels within the collagen-free topical composition and/or the collagen-integrated topical composition, follicular stem cells' migration may be accelerated. Accelerated progeny migration then accelerates healing without scarring. In this manner, in this embodiment, the net effect may be healing non-healing wounds without disfiguring scarring and/or healing "intentional" wounds (e.g., surgical, for example in breast reconstruction and augmentation) without visible scarring.

As indicated hereinabove, in an embodiment, the composition of the present disclosure may be useful for treating wounds, the wound may be the result of an accidental injury or be the consequence of a medical procedure. The wound may be a surgical incision. The wound may be an ischemic tissue flap, such as in the course of cosmetic surgery. The wound may be one caused in the course of other cosmetic surgery, such as dermabrasion, microdermabrasion, chemical peel, laser resurfacing, etc. The wound may be a chronic injury. The wound may be a skin tear. The wound may be a cut or razor burn or scrape or created by incision. The wound may be a chronic wound or injury.

As used herein the term chronic injury may be a wound, burn and/or skin condition that does not heal in an expected way and in a predictable amount of time, unlike acute injuries. A chronic injury may be recognized by a number of symptoms including the loss of skin and/or tissue surrounding the wound, burn and/or skin condition or by the amount of time it takes to heal. Once a wound, burn and/or skin condition has become chronic, intensive medical intervention and treatments are normally required for healing. There are many types of chronic injuries, as well as many causes of chronic injuries, such as Diabetes, Vascular disease, Infection, Immobility, Trauma, Surgery, Burns, Radiation injury, electrical burns, chemical burns and the like. A chronic wound may never heal or may take years to do so. These wounds may cause severe emotional and physical stress; they may also create a significant financial burden on the patient and on the entire healthcare system.

The topical compositions described in the present disclosure may be useful in treating chronic wounds. For instance, in an embodiment, the topical compositions in the present disclosure may be designed for healing chronic wounds. Chronic wounds may be typically treated once per week.

An acute wound may be a wound that progresses through the phases of normal healing, resulting in the closure of the wound within a reasonable amount of time, for example, in some embodiments, up to four weeks.

In some embodiments, the topical compositions of the present invention may be useful for treating chapped skin and/or other skin disorders. Non-limiting examples of skin disorders may include acne, psoriasis, eczema, dermatitis, alopecia, rosacea, burns, chapped skin, poison ivy, shingles and the like. For example, in these other embodiments, the compositions of the present disclosure may be useful in treating the skin conditions described below.

The term "acne" is meant to include any skin condition where a skin pore becomes blocked and/or thereby becomes inflamed. The term acne includes without limitation superficial acne, including comedones, inflamed papules, superficial cysts, and pustules; and deep acne, including deep inflamed modules and pus-filled cysts. Specific acne conditions may include, but are not limited to, acne vulgaris, acne comedo, papular acne, premenstrual acne, preadolescent acne, acne venenata, acne cosmetica, pomade acne, acne detergicans, acne excoriee, gram negative acne, acne rosacea, pseudofolliculitis barbae, folliculitis, perioral dermatitis, and hidradenitis suppurativa. Acne may be a common inflammatory pilosebaceous disease characterized by comedones, papules, pustules, inflamed nodules, superficial pus-filled cysts, and (in extreme cases) canalizing and deep, inflamed, sometimes purulent sacs. Acne involves an interaction between hormones, keratinization, sebum, and bacteria that somehow determines the course and severity of acne. It often begins at puberty, when the increase in androgens causes an increase in the size and activity of the pilosebaceous glands. The earliest microscopic change is thought to be intrafollicular hyperkeratosis, which leads to blockage of the pilosebaceous follicle with consequent formation of the comedo, composed of sebum, keratin, and microorganisms, particularly Propionibacterium acnes. Lipases from P. acnes break down triglycerides in the sebum to form free fatty acids (FFA), which irritate the follicular wall. Retention of sebaceous secretions and dilation of the follicle may lead to cyst formation.

Non-limiting examples of skin conditions may also include dermatological conditions linked to disorders of keratinization involving differentiation and/or proliferation, in particular, acne vulgaris, comedonic or polymorphic acne, nodulocystic acne, acne conglobata, senile acne and/or secondary acnes such as solar, drug and/or occupational acne; for other types of keratinization disorders especially ichthyoses, ichthyosiform conditions, Darier's disease, palmoplantar keratoderma, leukoplakia and/or luecoplakiform conditions and/or lichen and/or lichen planus; dermatological disorders having an inflammatory or immunoallergic component, in particular, all forms of psoriasis, either cutaneous, mucosal and/or ungual, and/or psoriatic rheumatism, and/or cutaneous atopy such as eczema and/or respiratory atopy, dry skin, inflammation of the skin, solar erythema, skin allergies and/or any other skin disorders of the epidermis and/or dermis known in the art.

Psoriasis may be a skin condition characterized by hyperplasia of keratinocytes resulting in thickening of the epidermis and the presence of red scaly plaques. The lesions in this chronic disease typically are subject to remissions and exacerbations. There are several patterns, of which plaque psoriasis is the most common. Guttate psoriasis, with raindrop shaped lesions scattered on the trunk and limbs, is the most frequent form in children, while pustular psoriasis is usually localized to the palms and soles. The classical inflammatory lesions vary from discrete erythematous papules and plaques covered with silvery scales, to scaly itching patches that bleed when the scales are removed. Psoriasis may be a condition in which cell proliferation is increased up to 10 times the normal rate for an individual. The skin is the largest portion of the human body which is comprised of cells within three skin layers. Each of the skin layers is in a constant state of growth with the outer layer being formed of predominantly dead tissue which is naturally being discarded at a normal rate. Replacement of cells from underlying layers is accomplished by cell division and maturation where cells move upwardly and outwardly at a rate which varies dependent upon the age, sex, and/or health of an individual. Psoriasis causes an increased turnover of cells, which in turn increases the rate of cell growth and cell death. This increased rate of cell growth and cell death may result in injuries and/or disorders which accompany the increased synthesis of all tissue components and further elevate the strain placed upon skin or other tissue and the bio-synthetic capabilities of the cells within the affected area.

The terms eczema and dermatitis, as used herein, refers to skin conditions that generally involve severe inflammation of the skin, usually with redness, swelling, oozing, rusting, and/or scaling of lesions which are usually itchy. Eczema may take the form of contact dermatitis (due to skin contact with the cause) or atopic dermatitis in individuals who are "atopic" or allergic by nature. If the scalp is involved the disorder is known as seborrheic dermatitis. Dermatitis may be caused by chemicals, plants, shoes, clothing, metal compounds and even medicines used to treat dermatitis. In atopic dermatitis environmental temperature, humidity changes, bacterial skin infections, airborne allergens, and garments, e.g., wool, may all bring about dermatitis.

Alopecia, as used herein, refers to a skin condition that results in the loss of hair on the scalp and elsewhere. It usually starts with one or more small, round, smooth patches and occurs in males and females of all ages. Loss of hair in one or several small spots is common, but it is possible to lose all scalp hair (alopecia totalis), or every hair on the body (alopecia universalis), which is rare.

The skin condition, rosacea is of an unknown origin. It usually affects the middle third of the face causing skin redness, prominent vascularization, papules, pustules and swelling, as well as a predisposition to flushing and blushing. However, rosacea may also occur on other parts of the body including the chest, neck, back, or scalp. The blood vessels near the skin dilate and become more visible there through, resulting in telangiectasia. The resulting papules and pustules resemble teenage acne and are frequently mistaken for the same. Unlike acne, rosacea does not have blackheads or whiteheads. Rosacea, however, may occur in all age groups and in both sexes, where it tends to be more frequent in women but more severe in men. The flushing and blushing regions of the face are affected by rosacea. Emotional factors such as anxiety, embarrassment, or stress may evoke or aggravate rosacea. In addition, a flare-up may be caused by environmental or climate variances, and UV exposure is known to aggravate rosacea. Furthermore, diet is also known to aggravate rosacea. Spicy foods, alcoholic beverages, hot beverages, and smoking are known to cause flare-ups. Rosacea is not only an aesthetic complication. Rosacea may be a chronic disease that has rarely been documented to reverse its progression. If untreated, the condition worsens and spreads. Untreated rosacea may cause a disfiguring nose condition called rhinophyma, which is characterized by a bulbous, red nose and inflamed cheeks. Severe rhinophyma may require surgery, an invasive procedure that may be avoided by timely treatment. Another problem of advanced rosacea is ocular. Persons afflicted with rosacea may experience conjunctivitis, a burning and grittiness of the eyes. If untreated, it may lead to serious complications such as rosacea keratitis, which damages the cornea and may impair vision. Other skin conditions include dry/chapped skin.

Burns involve a type of skin integrity rupture. Burns represent one of the most painful processes that may be established in this tissue, needing the establishment of a coordinated therapy to help its recovery and pain treatment. Burns may be caused by several factors, among which, exposure to high or low temperatures, exposure to chemical compounds, by electricity, by exposure to radiation and mechanical friction. Burn severity and its risk are evaluated according to the amount of affected tissue and depth reached. The amount of affected tissue is represented by the percentage of burned corporeal surface (BCS). In this type of evaluation, burns may be divided into small, moderate, large, or massive burns, where regions inferior to 15% of BCS, from 15% up to 49% of BCS, from 50% up to 69% of BCS and over 70% of BCS, respectively. The extension of the affected area is determined through Lund-Browder scheme, which takes into consideration the burn proportion, in accordance with the age of the burned patient. Another rule that is most used for determining the extension of the affected area is that known as Wallace Rule or Rule of Nines, a technique less efficient than the foregoing, however, easy to memorize, being very much employed in emergency cases. This rule applies a value equaling nine or nine multiples to the affected parts, being 9% for each superior member, 9% for the head, 18% for each inferior member, 18% for each torso face and 1% for the genitalia.

The classification as first, second and/or third degree corresponds to burn depth. The first-degree injury corresponds to the burn that affects the skin most external layer (epidermis), not producing hemodynamic alterations, however the affected region is found hyperemic in absence of blisters or phlyctenae. This type of injury may be observed in erythema resulting from sunrays or heated water. The second-degree injury affects either the epidermis as part of the dermis and is mainly characterized by the formation of blisters or phlyctenae, as those resulting from scalding or thermal injury resulting from overheated liquid. The third-degree injury endangers the totality of skin layers (epidermis and dermis) and, in many cases, may affect other tissues, as the subcutaneous cellular tissue, muscular tissue and bone tissue. Third-degree burns are considered as the most severe of all thermal injuries, producing deforming injuries. For being deeper, it eliminates the nerve endings responsible for shooting the painful message. These types of burns need transplanting for recomposing destroyed tissues, since the structures and organelles necessary for the natural recovery process, were eliminated. Since burns are wounds that involve the skin, they develop afore mentioned complex process of regeneration and re-composition of injured tissue. The speed or grade of re-epithelization of the affected region is small the greater the area involved is, considerably increasing the recovery time, when the injuries start to cover a body surface over 10%, and/or 15%.

Immediately after the burn trauma, an inflammatory process develops, such that various agents may be delivered, occurring deposition of fibrins and platelets activated on the wound surface. A matrix rich in organic material is yielded, able to enclosure bacteria and other strange substances, which frequently aggravates the case, due to sepsis that may follow trauma. During this inflammatory process, a great quantity of exudates crops out of the burned region, leading the patient to an intense loss of liquids, which, depending on the burn extension and depth, may cause a severe dehydration case. The inflammatory process extends to adjacent tissues, factor that endangers the functions of these tissues initially intact. Extensive and deep burns cause alterations that are extended far beyond the affected local, such as anatomic, metabolic, physiological, endocrinology and immune alterations, requiring special care. Significant fluid losses, delivery of inflammatory multi-mediators and contamination by bacteria, occur. When disseminated in central organs through circulation, bacteria and inflammatory mediators may cause cardiac endangerment, failure of gastrointestinal mucous integrity and in extreme cases, multi-organic failure.

Hemodynamic alterations that occur after severe thermal injuries include decrease of cardiac output and reduced volume of circulating plasma, contributing all to a hypovolemic shock. Inflammatory mediators (including cytokines, prostaglandin, nitric oxide, and superoxide ions) have been implicated in causing further damage to tissues. It is believed that despite local benefit, such mediators induce undesirable effects when reaching significantly high levels. As an example, a greater damage to tissues may be caused by delivery of proteolytic enzymes and superoxide ions of macrophages and activated leucocytes.

Thus, burns are skin conditions that develop unbalance in a series of natural organic mechanisms, not limited to endangered tissues only, but involve numerous organs that may be affected. Additionally, large thermal injuries induce to a sharp increase in basal metabolic rate. Large nitrogen corporeal losses, observed in burned patients, mainly occur due to protein exudation through burned skin and also by the fact that, under such catabolic stress situation, corporeal proteins may become the metabolic substrate used for production of 15 to 20% of total energy required by the organism. Further to these abnormalities, hormonal levels change with an increase in catecholamines, cortisol and glucagon's, in the presence of normal or slightly increased levels of insulin. These hormonal alterations promote increase of proteolysis and lipolysis. Thus, the entire complex process is characterized by imbalance. The quick recovery of the skin of a burned mammal is of utmost importance for recovery of the normal organic functions.

In an embodiment, the topical compositions of the present disclosure may be used to treat at least one type of burn known in the art, at least one type of wound known in the art, and/or at least one type of skin condition known in the art, as described below.

For the treatment of wounds, burns and skin conditions, the composition of the present disclosure may be applied directly to the skin and/or wound as a gel. Alternatively, the stabilized formulation is administered in the form of a wound dressing, such that the topical compositions of the present disclosure may be applied onto a wound dressing and the wound dressing with the composition of the present may be is then applied to cover the area of the skin at the location of the skin condition or wound. As used herein, the terms "wound dressing" and "dressing" refer broadly to any substrate when prepared for, and applied to, a wound for protection, absorbance, drainage, improvement of cell environment, etc., and may include any one of the numerous types of substrates and/or backings that are commercially available, including films (e.g., polyurethane films), hydrocolloids (e.g., hydrophilic colloidal particles bound to polyurethane foam), hydrogels (e.g., cross-linked polymers containing about at least 60% water), foams (hydrophilic or hydrophobic), calcium alginates (e.g. non-woven composites of fibers from calcium alginate), silicone, collagen, keratin, and cellophane (e.g. cellulose with a plasticizer). For example, in some embodiments, the stabilized formulation may be applied to the surface of, and/or incorporated into, a solid contacting layer such as a dressing gauze or ECM matrix. Suitable gauze dressings may include, for example, dry woven and/or non-woven sponges, swabs, bandages and/or wraps with varying degrees of absorbency. Exemplary fabric composition may include but not limited to, for example, cotton, polyester, and/or rayon. In an embodiment, gauzes and/or non-woven dressings may be available sterile or non-sterile in bulk and with or without an adhesive border. In an embodiment, the dressings may also comprise one or more additional pharmaceutically active compound and/or carrier agent, including but not limited to, for example, saline, oil, zinc salts, cross-linked collagen, petrolatum, xeroform and/or scarlet red.

Various treatments may be employed. Skin surfaces of the most concern tend to be those not typically covered by clothing such as facial skin surfaces, hand and arm skin surfaces, foot and leg skin surfaces, and neck and chest skin surfaces. In particular, facial skin surfaces, including the forehead, perioral, chin, periorbital, nose, and/or cheek skin surfaces, may be treated with the compositions described herein.

In an embodiment, the treatment method may include applying collagen-free topical composition and/or the collagen-integrated topical composition onto the area of the body where the pain is felt by the subject, to a previously identified area of skin in need of treatment, and/or an area where one seeks to prevent, treat, and/or reduce the appearance of chapped skin and/or other skin disorders. Many regimens exist for the application of the composition(s). In this embodiment, the composition(s) may be applied at least once a day, twice a day, and/or on a more frequent daily basis, during a treatment period, as prescribed by the physician. When applied twice daily, the first and second applications are separated by at least 1 to 12 hours. For example, in some embodiments, the composition(s) may be applied in the morning and/or in the evening before bed.

In an embodiment, the treatment period may ideally be of sufficient time until the pain disappears, and/or then in some cases, 1-3 days longer to provide an improvement in the appearance of the skin. The treatment period may be at least 1 week, and in some embodiments the treatment period may last about 4 weeks, 8 weeks, or 12 weeks. In an embodiment, the treatment period may extend over multiple months (e.g., 3-12 months) and/or multiple years. In one embodiment the composition may be applied at least once a day during a treatment period of at least 4 weeks, 8 weeks, or 12 weeks. In one embodiment the composition may be applied twice a day during a treatment period of at least 4 weeks, 8 weeks, or 12 weeks. The treatment regimen may be determined by the physician

In an embodiment, effective amounts of each of the compositions in any combination may be applied topically to the area to be treated. The dose varies with the individual and the skin condition. The ideal dose is that The dose of the combination therapy varies with the individual and the skin condition. The ideal dose is that dose that may provide as much of the compositions as the skin will absorb. An excess amount of the compositions on the surface of the skin may also be applied and/or may comprise an additional odor. In a non-limiting example, patients who used the compositions described herein found that applying the compositions to the areas to be treated just prior to showering may be the most effective way to administer these compositions. The skin absorbs all it can; the shower washes off the excess. In an embodiment, a silky smooth, odorless finish from an effective amount of each of the compositions in any combination may remain on the skin for 24 hours.

The dosage regimen for treating skin conditions, burns and/or wounds may be determined by a physician in the normal course of practice and is selected in accordance with a variety of factors, including the age, weight, sex, medical condition of the patient, the severity of the condition, the route of administration, and/or pharmacological considerations such as the activity, efficacy, pharmacokinetic and/or toxicology profiles of the particular composition used, whether a dressing and/or drug delivery system is used and/or whether the composition is administered as part of a drug combination. This composition may be applied in therapeutically effective amounts. In an embodiment, the amount of the collagen-free composition described in this disclosure, may be applied to the wound, skin condition and/or burn may be about 0.1g//m²/day to about 2 g/m²/day, and/or in another embodiment, at about 0.5 g/m²/day to about 2 g/m² and/or in another embodiment, at about 0.5 g/m² to about 1 g/m². Further, the composition may be applied to cover the wound In addition, the collogen containing composition may be applied to the wound, in an embodiment in a range of about may be applied to the would the amount of the collagen composition in 0.1g//m² to about 2 g/m², and/or in another embodiment, at about 0.5 g/m² to about 2 g/m^{2,}/day and/or in another embodiment, at about 0.5 g/m²/day to about 1 g/m²/day. Further, the composition may be applied to cover the wound, skin condition, and/or burn.

The doses may be administered in single or divided applications. The doses may be administered once, and/or application may be repeated. Application may be repeated weekly until skin and/or wound healing is promoted, or a repeat application may be made in the event that healing slows or is stalled. Doses may be applied 1-7 days apart and/or more, as needed, as determined by the doctor and/or physician.

In an embodiment, the combination of one or more collagen-free comprising compositions and/or one or more collagen-integrated topical compositions, as described herein may be effective in treating at least one type of pressure wound.

In an embodiment, the amount of the composition described in this disclosure, and applied to the area of the pain may be 0. 1g//m²/day to about 2 g/m²/day, and/or in another embodiment, may be at about 0.5 g/m² to about 2 g/m² and/or in another embodiment, may be at about 0.5 g/m² to about 1 g/m².

As another non-limiting example of the effect of the penetration ratio ranging greater than or equal to 1, but less than about 3, may be a composition useful for reducing bags under the eye. In an embodiment, the composition may comprise MCT. It is important to recognize why a collagen-free oil/gel may be applied before a collagen-rich paste. The active ingredients may be dissolved in the oil/gel and thus penetrate deeply into the epidermis. In a paste, the active ingredients may be non-motile because of the viscosity of the paste. This means the active ingredients stay on the skin surface until the collagen absorbs may exudate and/or may be dissolved, freeing up oil entrapped in collagen pores. The net effect may be a pulse of active ingredient entering the epidermis (from the oil/gel) followed by a slow release of the same active ingredients between treatments (by release from dissolved hydrolyzed collagen).

In an embodiment, the present disclosure may also include a kit, in which the kit may comprise at least one collagen-free composition, as described hereinabove in separate container(s) and at least one collagen-integrated topical composition as described herein above in separate container(s), instructional material for use thereof and/or optionally any applicator known in the art configured for applying the compositions topically to the wound, burn and/or skin condition.

In an embodiment, the instructional material included in the kit may comprise instructions for treating the wound, burn and skin condition. The instructional material recites the amount of, and/or frequency with which the collagen and collagen-free compositions described herein may be required to be applied topically to the wound, burn and/or skin condition.

In an embodiment, depending upon the severity of the wound, burn and/or skin condition, the doctor may utilize composition described herein with a combination of one or more topical compositions, the contents of both of which are incorporated herein by reference, in their entireties. If the wound, burn, or skin condition is very painful to the patient, a first formulation comprising an analgesic, such as lidocaine, which is added as an ingredient in the compositions described herein, may be applied to the locus of the wound, burn and/or skin condition in effective amounts to alleviate pain associated with the wound and/or the skin condition. These compositions may be a composition described herein which may comprise an analgesic, such as lidocaine, which helps alleviate the pain associated with the wound, burn and/or skin condition and/or it may be one of the collagen-free containing compositions comprising lidocaine described in one or the aforementioned patent publications. Besides alleviating the pain, such formulation may help dislodge necrotic tissue associated with the skin condition and/or wound. The solvent action of the MCT helps loosen necrotic tissue. The excess formulation and slough may be wiped away.

After the application of this composition, in an embodiment, the doctor and/or patient and/or user may wait for sufficient time for the analgesic to numb the wound bed tissue, the burn tissue, and/or the loci of the skin condition on the skin. In this embodiment, this may take about 5 to about 30 minutes, encompassing every value in between. The loose necrotic tissue and/or any unabsorbed composition may be wiped out of the wound bed loci of the skin condition with a sterile gauze and/or wipe. Optionally, in an embodiment, with a scalpel, additional necrotic tissue may be sharply debrided from the bed of the wound or skin condition or situs of the burn. As the wound begins to close and/or the skin condition begins to clear up and/or the situs of the burn begins to heal, the need for sharp debridement may be reduced as viable new tissue replaces pre-existing necrotic tissue. In this embodiment, the analgesic formulation may then be wiped off with sterile material, such as gauze.

In an embodiment, especially if the wound or skin condition or burn may still painful, additional formulation containing the analgesic may be applied to the situs of the wound, burn and/or skin condition for another 5 to 60 minutes to further numb the pain, and then the analgesic formulation may be wiped off. The composition of the present disclosure may be applied in therapeutically effective amounts. One of ordinary skill in the art, such as a physician may make that determination based upon various factors, such as the severity of the wound, burn and/or the skin condition, the age of the patient, the health of the patient and the size of the wound, burn and/or skin condition including the depth thereof and/or the like. In an embodiment, the amount of the composition described in this disclosure, and/or may be applied to the wound, skin condition and/or burn may be 0.1g//m² to about 2 g/m², and/or in another embodiment, at about 0.5 g/m² to about 2 g/m², and/or in another embodiment, at about 0.5 g/m^{2 to} about 1 g/m². Further, the composition may be applied to cover the wound, skin condition, and/or burn.

Next, in an embodiment, the collagen-integrated topical formulation may next be applied to the loci of the wound, burn and/or skin condition in effective amounts to treat the wound, burn and/or skin condition and effect granulation. The collagen formulation encourages granulation. Since this composition is so viscous, the application thereof may be deposited as stripes (e.g., separate spots on the wound or skin condition). However, the body heat may soften the collagen-integrated topical composition until it flows into the irregularities of the wound bed surface or burn surface or surface of the skin condition and conjoins with adjacent stripes to form a contiguous coating of the entire wound bed surface or surface of the loci where the skin condition or burn is located. In this embodiment, the collagen formulation may then be covered with a sterile cloth and/or gauze or band-aid. Periodically, for example, in some embodiments, once a week and/or sooner, the situs of the wound, burn and/or skin condition may be inspected to see if it is fully granulated. If the situs of the wound, burn and/or skin condition is not fully granulated, then, in an embodiment, the old collagen formulation is allowed to remain and new collagen with a fresh collagen formulation and covered again. At the beginning of the treatment, the wound, burn, or skin condition is inspected more frequently; as the wound, burn and/or skin condition starts to heal and granulate, the inspection and replacement of the collagen composition with fresh collagen composition may be less frequent. One of ordinary skill in the art, such as a physician may make that determination based upon various factors, such as the severity of the wound, burn or the skin condition, the age of the patient, the health of the patient and the size of the wound, burn and/or skin condition including the depth thereof and the like. The process of replacing the old collagen formulation with new collagen formulation of the present disclosure may be repeated until the situs of wound or skin condition or burn is fully granulated. The amount applied is in therapeutically effective amounts. In an embodiment, the amount of the collagen composition and applied to the wound, skin condition or burn may be 0.1g//m²/day to about 2 g/m²/day, and/or in another embodiment, may be at about 0.5 g/m²/day to about 2 g/m²/day ^{,}and/or in another embodiment, may be at about 0.5 g/m²/day to about 1 g/m²/day. Further, the composition may be applied to cover the wound, skin condition, or burn. Further, the application of the collagen composition may be applied one, two or three times a day, depending on the instructions of the physician or the person treating the injury. This treatment may be continued until the situs of the wound, or skin condition or burn is fully granulated.

Once the wound or skin condition or burn is fully granulated and/or concurrent with the first collagen application, the skin protectant topical composition, which may be a formulation described herein which does not contain an analgesic may be applied to the periwound surrounding the situs of the wound bed surface or surface of the skin condition or burn. In an non-limiting example, the skin protectant topical composition may be the anhydrous gel composition, such that the skin protectant topical composition may be topically applied thereto. In this embodiment, the skin protectant topical composition may be configured to cover completely the periwound around the situs of the wound, burn and/or skin condition and the intact skin adjacent to the area, such as the periwound, in an amount effective to protect the situs from infection and/or ROS species and/or to retard the growth of pathogens, such as bacteria. In an embodiment, the periwound is saturated with the skin protectant topical composition. Any excess of the compositions used herein is wiped away.

In an embodiment, the skin protectant topical composition may be applied to the situs of the wound, particularly after the situs has closed, but not yet remodeled, the burn and/or skin condition, but also an area adjacent thereto, such as the peri wound, for example, or in another embodiment, the area that borders or is adjacent to and in close proximity to the situs, for example, an area up to 6 inches in diameter around the situs. Periodically, the wound is inspected and, if the skilled medical practitioner so determines, the skin protectant topical composition is reapplied as before. Again, the frequency of removing and reapplying the skin protectant topical composition is determined by the physician, and is dependent upon many factors, including the health and age of the patient, the size of the wound or skin condition or burn and the drainage from the wound, skin condition or burn, the sex of the patient, and the like.

In an embodiment, the composition may be applied in therapeutically effective amounts. In an embodiment, the amount of the collagen composition and applied to the wound, skin condition or burn may be 0.1g//m²/day to about 2 g/m²/day, and/or in another embodiment, at about 0.5 g/m²/day to about 2 g/m²/day, and/or in another embodiment, at about 0.5 g/m²/day to about 1 g/m²/day. Further, the composition may be applied to cover the wound, skin condition, or burn. Further, the application of the collagen composition may be applied one, two or three times a day, depending on the instructions of the physician or the person treating the injury.

Moreover, in an embodiment, the skin protectant topical composition may be used to stimulate hair growth. When this happens, stem cells are activated (if signaled for by the wound site). The stem cells may not migrate, but their progeny migrate across the basement layer separating the epidermis from the dermis and eventually onto the wound bed. The progeny form into a very thin film of epidermal material covering the granulating wound bed. Once formed, this monolayer of epithelial tissue reflects light (making it visible to the skilled practitioner) and separates the granulating bed from excess topical collagen. Topical collagen absorption is inhibited; the epithelial tissue thickens and forms an epidermis; scar formation is minimized, and the wound heals.

In an embodiment, the process of replacing the absorbed skin protectant topical composition may be repeated until the wound has healed and remodeling is complete. Typically, the skin protectant topical composition change may occur about once a day. Typically, it takes about two years for the wound to remodel, although the length of time for complete healing and/or remodeling may vary depending upon various factors, such as the size of the wound, the age and/or health of the patient, and the like.

In an embodiment, prior to the first step (e.g., prior to the application of the composition described herein comprised of analgesic), the skin protectant topical composition may be applied to an area proximate to the skin condition or wound but not on the skin condition or wound. The skin protectant topical composition is the anhydrous gel composition of the present disclosure comprised of marine oil, such as fish oil or algae oil, vegetable oil having an omega-3 fatty acid content greater than 9 wt%, MCT (medium chain triglycerides), monoglyceride, such as monolaurin, linear fatty acid esters, such as cetyl esters and/or waxes. It has a penetration factor greater than or equal to 1, but less than 3. This composition may be an oil.

In an embodiment, in the skin protectant topical composition (e.g., a collagen-free topical composition) MCT may be present in an amount greater than or equal to 50 wt%. In an embodiment, MCT may be present in an amount ranging from about 50 wt% to about 70 wt%, encompassing every value in between, and/or in another embodiment, from about 50 wt% to about 60 wt%, encompassing every value in between, and/or in another embodiment, from about 51 wt% to about 55 wt%, encompassing every value in between, and/or in another embodiment, from about 52 wt% to about 54 wt%, encompassing every value in between, and/or in another embodiment at about 53 wt%. For example, in some embodiments, MCT may be present in 50 wt%, or 51 wt%, or 52 wt%, or 53 wt%, or 54 wt%, or 55 wt%, or 56 wt%, or 57 wt%, or 58 wt%, or 59 wt%, or 60 wt%, or 61 wt%, or 62 wt%, or 63 wt%, or 64 wt%, or 65 wt%, or 66 wt%, or 67 wt%, or 68 wt%, or 69 wt%, or 70 wt%, or any value in between. In an embodiment, MCT may be present in about 3 to about 4 times the weight amount of marine oil present. In a further embodiment, MCT may be present in about 1.5 to about 2 times the weight amount of the sum of the marine oil and vegetable oil having an omega-3 fatty acid content greater than 9 wt%.

In an embodiment, The marine oil (e.g., cod liver oil) may be present in the skin protectant topical composition of the present disclosure in an amount ranging from about 5 wt% to 13.56 wt%, encompassing every value in between, and/or in another embodiment, from about 8 wt% to 13.56 wt%, encompassing every value in between, and/or in another embodiment from about 10 wt% to about 13.56 wt%, encompassing every value in between, and/or in another embodiment, from about 12 wt% to 13.56 wt%, encompassing every value in between, and/or in another embodiment, 13.56 wt%. For example, in some embodiments, the marine oil, (e.g., cod liver oil) may be present in 5.0 wt%, or 5.1 wt%, or 5.2 wt%, or 5.3 wt%, or 5.4 wt. %, or 5.5 wt%, or 5.6 wt%, or 5.7 wt%, or 5.8 wt%, or 5.9 wt%, or 6.0 wt%, or 6.1 wt%, or 6.2 wt%, or 6.3 wt%, or 6.4 wt%, or 6.5 wt%, or 6.6 wt%, or 6.7 wt%, or 6.8 wt%, or 6.9 wt%, or 7.0 wt%, or 7.1 wt%, or 7.2 wt%, or 7.3 wt%, or 7.4 wt. %, or 7.5 wt%, or 7.6 wt%, or 7.7 wt%, or 7.8 wt%, or 7.9 wt%, or 8.0 wt%, or 8.1 wt%, or 8.2 wt%, or 8.3 wt%, or 8.4 wt. %, or 8.5 wt%, or 8.6 wt%, or 8.7 wt%, or 8.8 wt%, or 8.9 wt%, or 9.0 wt%, or 9.1 wt%, or 9.2 wt%, or 9.3 wt%, or 9.4 wt. %, or 9.5 wt%, or 9.6 wt%, or 9.7 wt%, or 9.8 wt%, or 9.9 wt%, or 10.0 wt%, or 10.1 wt%, or 10.2 wt%, or 10.3 wt%, or 10.4 wt%, or 10.5 wt%, or 10.6 wt%, or 10.7 wt%, or 10.8 wt%, or 10.9 wt%, or 11.0 wt%, 11.0 wt%, or 11.1 wt%, or 11.2 wt%, or 11.3 wt%, or 11.4 wt%, or 11.5 wt%, or 11.6 wt%, or 11.7 wt%, or 11.8 wt%, or 11.9 wt%, or 12.0 wt%, or 12.1 wt%, or 12.2 wt%, or 12.3 wt%, or 12.4 wt%, or 12.5 wt%, or 12.6 wt%, or 12.7 wt%, or 12.8 wt%, or 12.9 wt%, or 13.0 wt%, or 13.1 wt%, or 13.2 wt%, or 13.3 wt%, or 13.4 wt%, or 13.5 wt%, or 13.56 wt%, or any value in between.

In an embodiment, the weight ratio of the sum of the weights of (ALA + SDA)/sum of the weights of (EPA and DHA) (hereinafter the "PUFA ratio") present in the skin protectant topical composition (e.g., collagen-free topical composition) is higher than 1:1, it may range from about 1.4 to about 2.0, encompassing every value in between, in one embodiment, and/or in another embodiment from about from about 1.5 to about 1.9, encompassing every value in between, and/or in another embodiment. 1.6 to about 1.8, encompassing every value in between, and/or in another embodiment, about 1.75.

In the formulations of the present disclosure, the function of the monoglyceride, such as monolaurin, when present, in the formulations described herein, as stated above, is to help reduce the undesirable smell of the marine oil, such as cod liver oil. Without wishing to be bound, it is believed that the monoglyceride, such as monolaurin, also helps the marine oil be absorbed by the skin.

In an embodiment, monolaurin is restricted by its inherent solubility in an oil to less than or equal to 0.6%. In some embodiments, it may be present in about 0.6 wt%. For example, monoglycerides, such as monolaurin, may be present in 0.1 wt%, or 0.2 wt%, or 0.3 wt%, or 0.4 wt%, or 0.5 wt%, or 0.6 wt%, or any value in-between.

In addition, another ingredient in the skin protectant topical composition that may help remove the odor in compositions described herein are the linear fatty acid esters, especially cetyl esters. In an embodiment, the linear fatty acid esters, such as cetyl esters may be present in an amount ranging from about 0.5 wt% to about 3 wt%, encompassing every value in between, and/or in another embodiment, from about 0.75 wt% to about 2 wt%, encompassing every value in between, and/or in another embodiment, from about 1.0 to about 1 75 wt%, encompassing every value in between. In another embodiment, the linear fatty acid esters, such as cetyl esters may be present in an amount ranging from about 1.4 wt% to about 1.6 % by weight of the composition, encompassing every value in between, and/or in another embodiment, about 1.50 wt%. For example, in some embodiments, cetyl esters may be present in 0.75 wt%, or 0.8 wt%, or 0.9 wt%, or 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or any value in between. Further, in this embodiment, it is believed that adding cetyl esters to the skin protectant topical composition may create a silky smooth skin surface (finish) that is very pleasant to the touch.

A further means of reducing the fish oil and/or odor in both the collagen-free topical composition and/or the collagen-integrated topical composition is to use less of the marine oil, such as cod liver oil. Vegetable oil having an omega-3 fatty acid content greater than 9 wt%, such as hempseed oil, is another source of omega-3 fatty acids. Marine oil, such as cod liver oil, may provide very long chain omega-3 fatty acids (C> 18); vegetable oil having an omega-3 fatty acid content greater than 9 wt%, such as hempseed oil, may provide long chain omega-3 fatty acids (C=18).

In compositions of the present disclosure, it may be advantageous to use vegetable oil having an omega-3 fatty acid content greater than 9 wt%, such as hempseed oil, in addition to marine oil, such as cod liver oil. This combination may provide the most effective ratios of C18 + C>18 omega-3-fatty acids. However, there is also the trade-off between inflammatory byproducts and anti-inflammatory products with odor control. Nevertheless, in compositions described herein, in an embodiment, the combination of C18 + C> 18 omega-3-fatty acids lowers the fish odor smell. Thus, by using a greater amount of vegetable oil having an omega-3 fatty acid content greater than 9 wt%, such as hempseed oil, less marine oil, such as cod liver oil, is necessary. Accordingly, as described hereinabove, in this embodiment, by utilizing more vegetable oil having an omega-3 fatty acid content greater than 9 wt%, such as hempseed oil, and/or reducing the amount of marine oil, such as cod liver oil, the present formulations reduce the smell of the marine oil, such as cod liver oil. However, with the amount of monoglyceride, such as monolaurin and linear fatty acid esters, such as cetyl esters present herein, any fish smell of the marine oil, such as cod liver oil, may be considerably reduced and/or may be eliminated. As described herein, in an embodiment, with the addition of marine oil, such as hempseed oil, linear fatty acid esters, such as cetyl esters and/or MCT and/or monoglyceride such as monolaurin, to the fish oil composition in the amounts described herein, the topical composition(s) may reduce and/or may entirely eliminate any potential fish odor, and/or when the composition may be applied to the skin of a patient, the bag under the eye of the patient may be reduced in size and/or the skin of the patient may also be moisturized.

In an embodiment, the amount of vegetable oil having an omega-3 fatty acid content greater than 9 wt%, for example, hempseed oil, may range from about 10 wt% to about 35 wt%, encompassing every value in between, and/or in another embodiment, from about 15 wt% to about 32 wt%, encompassing every value in between, and in a further embodiment, from about 20 wt% to about 30 wt%, encompassing every value in between, and/or in another embodiment, from about 24 wt% to about 27 wt%, encompassing every value in between. For example, in some embodiments, vegetable oil having an omega-3 fatty acid content greater than 9 wt%, for example, hempseed oil, may be present in 10.0 wt%, or 11.0 wt%, or 12.0 wt%, or 13.0 wt%, or 14 wt%, or 15 wt%, or 16 wt%, or 17 wt%, or 18 wt%, or 19 wt%, or 20 wt%, or 21 wt%, or 22 wt%, or 23 wt%, or 24 wt%, or 25 wt%, or 26 wt%, or 27 wt%, or 28 wt%, or 29 wt%, or 30 wt%, or 31 wt%, or 32 wt%, or 33 wt%, or 34 wt%, or 35 wt%, or any value in-between.

All of the possible combinations and permutations of the components listed herein may be contemplated to be within the scope of the present compositions. In an embodiment, the sum of the weight % of MCT, marine oil, monoglycerides (e.g., monolaurin), linear fatty acid esters (e.g., cetyl esters), or vegetable oil having an omega-3 fatty acid content greater than 9 wt% may range from about 80 wt% to about 100 wt%, encompassing every value in between, and/or in another embodiment, from about 85 wt% to about 100 wt%, encompassing every value in between, and/or in another embodiment, from about 90 wt% to about 100 wt%, encompassing every value in between, and/or in another embodiment, from about 92 wt% to about 100 wt%, encompassing every value in between. For example, in some embodiments, the components may be present in total in 80 wt%, or 81 wt%, or 82 wt%, or 83 wt%, or, 84 wt%, or 85 wt%, or 86 wt%, or 87 wt%, or 88 wt%, or 89 wt%, or 90 wt%, or 91 wt%, or 92 wt%, or 93 wt%, or 94 wt%, or 95 wt%, or 96 wt%, or 97 wt%, or 98 wt%, or 99 wt%, or 100 wt%, or any value in between.

In an embodiment, the weight ratio of the sum of the weights of (ALA + SDA)/sum of the weights of (EPA and DHA) (hereinafter the "PUFA ratio") present in the composition may range from about 0.5 to about 1.5 and/or in another embodiment. 0.75 to about 1.25, encompassing every value in between, and/or in another embodiment, the ratio may be from about 0.9 to about 1.1, encompassing every value in between, and/or in another embodiment, about 1.0.

In an embodiment, the composition may additionally contain vegetable oil, as defined herein. There may be 1 vegetable oil, or more than 1 vegetable oil added to the composition. In one embodiment, the total sum of the vegetable oil may range from about 3 wt% to about 9 wt%, encompassing every value in between, and/or in another embodiment, from about 5 wt% to about 7 wt%, encompassing every value in between. In an embodiment, the vegetable oil is coconut oil, and/or in another embodiment, it may be palm oil, (e.g., RBD palm oil) and/or in another embodiment, it may be a combination of coconut oil and palm oil, especially RBD palm oil. If the vegetable oil may be a combination of coconut oil and palm oil, the coconut oil may be present in greater amounts, with a weight ratio of coconut oil to palm oil ranging from about 1.2:1 to about 2:1, encompassing every value in between, in one embodiment, and from about 1.4:1 to about 1.8:1, encompassing every value in between, in another embodiment and about 1.5:1 to about 1.7:1, encompassing every value in between.

In an embodiment, the skin protectant topical composition (e.g., collagen-free topical composition) of the present disclosure may be anhydrous oils and/or may be prepared by techniques known in the art (e.g., U.S. Patent No. 10,463,699 and/or WO 2019/200364), the contents of both of which are incorporated by reference. These oils may be prepared by mixing the various components at slightly elevated temperatures, such as about 30°C to about 60°C, encompassing every value in between, and/or in another embodiment, at about 35°C to about 50°C, encompassing every value in between, and/or in another embodiment, at about 38°C to about 43°C, encompassing every value in between, until the resulting mixture is homogenous, and/or then the hot composition is placed into the vessel, which is then sealed and where the composition is allowed to cool to room temperature. In an embodiment, the composition is pumped into a filling machine, which then fills the composition into a sealed container, such that the liquid may cool upon standing. The composition may be sold as an oil, ointment, salve, paste, cream, and/or whip.

In an embodiment, the treatment method may be to apply the oily composition(s) (e.g., collagen-free topical composition) to the bags under the eyes. In this embodiment, the collagen-free topical composition may be placed on the palm of one hand and a cotton tipped swab is rolled into the puddle of in the palm oil and then rolled over one eye and then the other. The composition(s) may be applied at least once a day, twice a day, or on a more frequent daily basis, during a treatment period, as prescribed by the physician. When applied twice daily, the first and second applications are separated by at least 1 to 12 hours, encompassing every value in between.

In an embodiment, the collagen-free topical composition and/or the collagen-integrated topical composition may be applied in the morning and/or in the evening before bed. The treatment period is ideally of sufficient time to provide an improvement in the appearance of the skin. The treatment period may be a day, or a few days or a week or longer. In one embodiment the composition may be applied at least once a day during a treatment period of several days, e.g., 1 day, and up to a week or more.

In an embodiment, the dosage regimen for the collagen-free topical composition for treating the bags under the eye may be is determined by a physician in the normal course of practice and is selected in accordance with a variety of factors, including the age, weight, sex, and medical condition of the patient, the severity of the condition, the route of administration, pharmacological considerations such as the activity, efficacy, pharmacokinetic and toxicology profiles of the particular composition used, whether a dressing or drug delivery system is used and whether the composition is administered as part of a drug combination. Effective amounts of each of the compositions are applied topically to the bags under the eye. The dose varies with the individual and the skin condition. The ideal dose is that dose that may provide as much of the compositions as the skin will absorb. In addition, in some embodiments, an odorless finish may remain on the skin for 24 hours. In an embodiment, the amount of the composition described in this disclosure, and applied to the bag under the eye may be 0.1g//m² to about 2 g/m², and/or in another embodiment, at about 0.5 g/m² to about 2 g/m^{2,}and/or in another embodiment, at about 0.5 g/m² to about 1 g/m².

A third example of a collagen-free topical composition may comprise a penetration factor greater than 10. In these compositions comprised of MCT, marine oil, such as fish oil or algae oil, vegetable oil having an omega-3 fatty acid content greater than 9 wt%, MCT (medium chain triglycerides), monoglyceride, such as monolaurin, linear fatty acid esters, such as cetyl esters and/or waxes, the amount of MCT present may be greater than or equal to 80 wt%. MCT is well-known as a permeation enhancer. These compositions are formulated with MCT>80% to drive unsaturated fats through the epidermis (Permeation Factor >10). It has been found that compositions that have such high penetration values may be useful for maintaining healthy skin.

In an embodiment, MCT may be present in 80 wt%, and/or greater up to about 91 wt%, encompassing every value in between. For example, in an embodiment, MCT may be present from about 80 wt% to about 91 wt%, encompassing every value in between, and/or in another embodiment, from about 80 wt% to about 90 wt%, encompassing every value in between. Thus, for example, in some embodiments, MCT may be present in 80 wt%, or 81 wt%, or 82 wt%, or 83 wt%, or 84 wt%, or 85 wt%, or 86 wt%, or 87 wt%, or88 wt%, or 89 wt, or 90 wt%, or 91 wt%, or any value in between.

In this non-limiting example, since the amount of MCT is so high, then the other ingredients present are lower than in most other compositions comprised of MCT. For example, in some embodiments, the marine oil, such as cod liver oil may be present in less than 5wt%, and lower than 4 wt%. For example, in some embodiments, the marine oil, such as cod liver oil, may be present in amounts ranging from about 1 wt% to about 4 wt%, encompassing every value in between, and/or in another embodiment, from about 1.5 wt% to about 3.5 wt%, encompassing every value in between, and/or in another embodiment, from about 2 wt% to about 2.5 wt%, encompassing every value in between. For example, in some embodiments, marine oil, such as cod liver oil, may be present in 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or any value in between.

Similarly, vegetable oil having an omega-3 fatty acid content greater than 9 wt%, may be present in less than 5 wt%, and/or in another embodiment, lower than 4 wt%. For example, in some embodiments, the marine oil, such as hempseed oil, may be present in amounts ranging from about 1 wt% to about 4 wt%, encompassing every value in between, and/or in another embodiment, from about 1.5 wt% to about 3.5 wt%, encompassing every value in between, and/or in another embodiment, from about 2 wt% to about 2.5 wt%, encompassing every value in between. For example, in some embodiments, vegetable oil having an omega-3 fatty acid content greater than 9 wt%, such as hempseed oil, may be present in 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or 3.1 wt%, or 3.2 wt%, or 3.3 wt%, or 3.4 wt%, or 3.5 wt%, or 3.6 wt%, or 3.7 wt%, or 3.8 wt%, or 3.9 wt%, or 4.0 wt%, or any value in between.

In addition, the sum of the weights of marine oil, such as cod liver oil, and the vegetable oil having omega-3 fatty acid content greater than 3 wt% may be at most 10 wt%, and/or in another embodiment, may be at most 8 wt% and/or in another embodiment, may be lower than 6 wt%.

In an embodiment, the linear fatty acid esters, such as cetyl esters, may be present in an amount ranging from about 0.5 wt% to about 3 wt%, encompassing every value in between, and/or in another embodiment, from about 0.75 wt% to about 2 wt%, encompassing every value in between, and/or in another embodiment, from about 1.0 wt% to about 1.75 wt%, encompassing every value in between. In another embodiment, the linear fatty acid esters, such as cetyl esters may be present in an amount ranging from about 1.4 wt% to about 1.6 wt% of the composition, encompassing every value in between, and/or in another embodiment, about 1.50 wt%. For example, in some embodiments, cetyl esters may be present in 0.75 wt%, or 0.8 wt%, or 0.9 wt%, or 1.0 wt%, or 1.1 wt%, or 1.2 wt%, or 1.3 wt%, or 1.4 wt%, or 1.5 wt%, or 1.6 wt%, or 1.7 wt%, or 1.8 wt%, or 1.9 wt%, or 2.0 wt%, or 2.1 wt%, or 2.2 wt%, or 2.3 wt%, or 2.4 wt%, or 2.5 wt%, or 2.6 wt%, or 2.7 wt%, or 2.8 wt%, or 2.9 wt%, or 3.0 wt%, or any value in between. Further, in an embodiment, the linear fatty acid esters within the topical compositions may create a silky smooth skin surface (e.g., finish) that is very pleasant to the touch.

In an embodiment, monoglyceride, such as monolaurin, is restricted by its inherent solubility in an oil to less than or equal to 0.6 wt%. In another embodiment, it may be present in about 0.6 wt%. For example, monoglycerides, such as monolaurin, may be present in 0.1 wt%, or 0.2 wt%, or 0.3 wt%, or 0.4 wt%, or 0.5 wt%, or 0.6 wt%, or any value in between.

The functions of the monoglyceride, such as monolaurin, as well as the linear fatty acid esters is as described with respect to the other compositions described hereinabove. However, since the amount of marine oil, such as cod liver oil, is so small, the smell from the cod liver oil is not as in intense as in other compositions containing larger amounts of marine oil.

All of the possible combinations and permutations of the components listed herein are contemplated to be within the scope of the present compositions. However, the sum of all of the components present in the compositions herein adds to 100 wt%. In an embodiment, the sum of the weight % of MCT, marine oil, monoglycerides, such as monolaurin, linear fatty acid esters, such as cetyl esters, vegetable oil having an omega-3 fatty acid content greater than 9 wt%, and may range from about 85 wt% to about 100 wt%, encompassing every value in between, and/or in another embodiment, from about 90 wt% to about 100 wt%, encompassing every value in between, and/or in another embodiment, from about 92 wt% to about 100 wt%, encompassing every value in between. For example, in some embodiments, the components may be present in total in 85 wt%, or 86 wt%, or 87 wt%, or 88 wt%, or 89 wt%, or 90 wt%, or 91 wt%, or 92 wt%, or 93 wt%, or 94 wt%, or 95 wt%, or 96 wt%, or 97 wt%, or 98 wt%, or 99 wt%, or 100 wt%, or any value in between.

The composition of this example may be an anhydrous oil and/or may be prepared by techniques known in the art (e.g., U.S. Patent No. 10,463,699 and/or WO 2019/200364), the contents of both of which are incorporated by reference, in their entireties. These oils may be prepared by mixing the various components at slightly elevated temperatures, such as about 30°C to about 60°C, encompassing every value in between, and/or in another embodiment, at about 35°C to about 50°C, encompassing every value in between, and/or in another embodiment, at about 38°C to about 43°C, encompassing every value in between, until the resulting mixture is homogenous, and then the hot composition is placed into the vessel, which is then sealed and where the composition is allowed to cool to room temperature. In an embodiment, the composition is pumped into a filling machine, which then fills the composition into a sealed container, such that the liquid may cool upon standing. The composition may be sold as an oil, salve, paste, cream, and/or whip.

As indicated hereinabove, this composition with greater than or equal to 80 wt% MCT may be useful in maintaining healthy skin. For maintenance of healthy skin, this composition may be rubbed on all parts of the body. Most subjects, especially women, may rub this composition on the face and the decolletage, and the legs. The composition(s) may be applied at least once a day, twice a day, and/or on a more frequent daily basis. In an embodiment, the composition(s) may be applied in the morning and/or in the evening before bed. In an embodiment, it is rubbed onto the body of the subject after showering. In some embodiments, the topical composition may be applied to the body of the subject in effective amounts to provide and/or maintain healthy skin. It may be placed on a proximate area which is at most about 2 times the diameter of the size of the wound and/or skin condition up to at least about six (6) inches from the skin condition and/or wound and then over the entire area approximate thereto. For example, in some embodiments, if the wound and/or skin condition and/or burn is on a limb, finger, and/or toe, then the skin protectant topical composition may be applied topically over substantially the remainder of the limb, finger, and/or toe, respectfully. If it is on the shoulder, back and/or stomach then the skin protectant topical composition may be applied topically over substantially the rest of the shoulder, back, and/or stomach, respectively. It may be applied in therapeutically effective amounts. In an embodiment, the amount of the composition described in this disclosure, and applied to the bag under the eye is 0.05g//m²/day to about 1.0 g/m²/day, and/or in another embodiment, at about 0.25 g/m²/day to about 0.5 g/m²/day

For a wound, skin condition and/or burn, in an embodiment, the composition may be applied in the amount comprising the range of about 0.1g//m² to about 2 g/m², encompassing every value in-between and/or in another embodiment, at about 0.5 g/m² to about 2 g/m², and/or in another embodiment, at about 0.5 g/m² to about 1 g/m². The function thereof is to exfoliate old dead and dying skin in the proximate area of the wound or skin condition to bring new, viable skin condition to the surface. Without wishing to be bound, it is believed that as this composition is absorbed, the anti-inflammatory omega-3 oils help reduce inflammation in the microvascular system. The vascular system, particularly the microcapillaries, relaxes to allow greater blood flow throughout the skin. The increased blood flow helps heal the wound more rapidly.

Besides the components listed above, in some embodiments, the various compositions described herein may contain other additional optional ingredients. In addition, if necessary, the composition of the present disclosure may contain additional thickeners, such as beeswax, candelilla wax, carnauba wax, ceresine wax, microcrystalline wax, ozocerite wax, PEG 4000, PEG 600, paraffin wax, laurel wax, vegemite wax and/or any other high melting point waxes and/or gelling agents known in the art.

The compositions may be formulated with stabilizing agents (e.g., polyoxyethylene sorbitan monolaurate, carboxy methyl cellulose), thickening, agents, such as beeswax, candelilla wax, carnauba wax, ceresine wax, microcrystalline wax, ozocerite wax, PEG 4000, PEG 600, paraffin wax, laurel wax, rice bran wax, vegemite wax and/or any other high melting point waxes (e.g., hydroxypropyl cellulose, carboxymethyl cellulose (CMC)), and/or coloring agents (e.g., dyes, lackes) known in the art. Further, the compositions may be carriers for active ingredients other than anesthetics, such as benzethonium chloride, methylbenzethonium chloride and/or benzalkonium chloride, GABA, statins, such as valsartan, and/or they may be carriers for dietary supplements, such as melatonin or glutathione, and/or the like. In some embodiments of the present disclosure, the composition may comprise any one or more of therapeutically active compounds known in the art and/or any one or more pharmaceutically and/or cosmetically acceptable diluents, excipients, and/or carriers known in the art.

Other additives that may be present in the formulation described herein. For example, in some embodiments, any antioxidants and/or preservatives known in the art may be added. Non-limiting examples may comprise, but are not limited to, tocopherols, ascorbic acid, Vitamin K, Vitamin E, sodium pyrosulfite, butylhydroxytoluene, butylated hydroxyanisole, edetic acid, and edetate salts, squalene, and/or mixtures thereof. Non-limiting examples of preservatives may include citric acid, tartaric acid, lactic acid, malic acid, acetic acid, benzoic acid, and/or sorbic acid.

The skin-condition, burn, and/or wound may then be covered by gauze or band-aid or any other covering or dressing that is typically used to protect the skin-condition or wound from infection. In an embodiment, it is covered with a compression wrap. In another embodiment, especially when there is no bleeding or if the wound may be a minor cut, such as a paper cut, after the collagen layer may be applied, the wound or skin condition is not covered.

Further, formulations of the present disclosure may not scar and/or the formulations of the present disclosure may be configured to minimize scarring after application of the formulation described herein to the wound, burn and/or skin condition.

In addition, another aspect of the present disclosure pertains to the anhydrous topical composition comprising antimicrobial properties. In an embodiment, the collagen-integrated topical composition having antimicrobial properties may comprise but is not limited to MCT, the vegetable oil (e.g., coconut oil), the hempseed oil, the marine oil (e.g., cod liver oil), the triethyl citrate, the ethyl linoleate, the caprylic acid (e.g., octanoic acid), the red palm concentrate, the monolaurin (e.g., glyceryl laurate), cetyl esters, the ascorbyl palmitate, the beeswax, the fish collagen, and/or the sea salt.

In an embodiment, the MCT may be present in the collagen-integrated topical composition in an amount comprising the range of at least 5.0 wt% to at most 55.0 wt%, encompassing every value in between. For example, in some embodiments, MCT may comprise at least 8.0 wt% of the collagen-integrated topical composition. In addition, in this embodiment, the vegetable oil may be present in the collagen-integrated topical composition in an amount comprising the range of at least 0.2 wt% to at most 20.0 wt%, encompassing every value in-between. For example, in some embodiments, the vegetable oil may comprise at least 10.0 wt% of the collagen-integrated topical composition.

In addition, in this embodiment, the vegetable oil (e.g., hempseed oil) may be present in the collagen-integrated topical composition in an amount comprising the range of at least 10.0 wt% to at most 30.0 wt%, encompassing every value in between. For example, in some embodiments, the hempseed oil may comprise at least 12.0 wt% of the collagen-integrated topical composition. The marine oil may be present in the collagen-integrated topical composition in an amount comprising the range of at least 10.0 wt% to at most 30.0 wt%, encompassing every value in between. For example, in some embodiments, the marine oil may comprise at least 11.4 wt% of the collagen-integrated topical composition. The triethyl citrate may be present in the collagen-integrated topical composition in an amount comprising the range of at least 1.0 wt% to at most 10.0 wt%, encompassing every value in between. For example, in some embodiments, the triethyl citrate may comprise at least 2.0 wt% of the collagen-integrated topical composition. In this embodiment, the ethyl linoleate may be present in the collagen-integrated topical composition in an amount comprising the range of at least 1.0 wt% to at most 10.0 wt%, encompassing very value in between. For example, in some embodiments, the ethyl linoleate may comprise at least 2.0 wt% of the collagen-integrated topical composition.

Moreover, in an embodiment, the caprylic acid (e.g., octanoic acid) may be present in the collagen-integrated topical composition in an amount comprising the range of at least 0.1 wt% to at most 3.0 wt%, encompassing every value in between. For example, in some embodiments, the caprylic acid may comprise at least 1.3 wt% of the collagen-integrated topical composition. The red palm concentrate may be present in the collagen-integrated topical composition in an amount comprising the range of at least 0.1 wt% to at most 9.0 wt%, encompassing every value in between. For example, in some embodiments, the red palm concentrate may comprise at least 0.2 wt% of the collagen-integrated topical composition. The monolaurin (e.g., glyceryl laurate) may be present in the collagen-integrated topical composition in an amount comprising the range of at least 0.4 wt% to at most 15.0 wt%, encompassing every value in-between. For example, in some embodiments, the monolaurin may comprise at least 4.3 wt% of the collagen-integrated topical composition. In addition, the cetyl esters may be present in the collagen-integrated topical composition in an amount comprising the range of at least 0.5 wt% to at most 5.0 wt%, encompassing every value in between. For example, in some embodiments, the cetyl esters may comprise at least 2.0 wt% of the collagen-integrated topical composition.

Furthermore, in an embodiment, the ascorbyl palmitate may be present in the collagen-integrated topical composition in an amount comprising the range of at least 0.1 wt% to at most 1.5 wt%, encompassing every value in between. For example, in some embodiments, the ascorbly palmitate may comprise at least 0.5 wt% of the collagen-integrated topical composition. The beeswax may be present in the collagen-integrated topical composition in an amount comprising the range of at least 1.0 wt% to at most 5.0 wt%, encompassing every value in between. For example, in some embodiments, the beeswax may comprise at least 4.0 wt% of the collagen-integrated topical composition. The fish collagen may be present in the collagen-integrated topical composition in an amount comprising the range of at least 20 wt% to at most 60 wt%, encompassing every value in between. For example, in some embodiments, the fish collagen may comprise at least 41.4 wt% of the collagen-integrated topical composition. The sea salt may be present in the collagen-integrated topical composition in an amount comprising the range of at least 0.1 wt% to at most 1.5 wt%, encompassing every value in between. For example, in some embodiments, the sea salt may comprise at least 0.9 wt% of the collagen-integrated topical composition. All of the possible combinations and/or permutations of the components listed herein may be contemplated to be within the scope of the present collagen-integrated topical composition. However, the sum of all of the components present in the collagen-integrated topical composition may herein add to 100 wt%.

Additionally, in an embodiment, the collagen-free topical composition having antimicrobial properties may comprise but is not limited to MCT, the vegetable oil (e.g., coconut oil), the hempseed oil, the marine oil (e.g., cod liver oil), the caprylic acid (e.g., octanoic acid), the red palm concentrate, the palm olein, the analgesic (e.g., lidocaine), the monolaurin (e.g., glyceryl laurate), cetyl esters, and/or the ascorbyl palmitate.

In an embodiment the MCT may be present in the collagen-free topical composition in an amount comprising the range of at least 5.0 wt% to at most 55.0 wt%, encompassing every value in-between. For example, in some embodiments, MCT may comprise at least 32.0 wt% of the collagen-free topical composition. In another example, in some embodiments, MCT may comprise at least 53.0 wt% of the collagen-free topical composition In addition, in this embodiment, the vegetable oil may be present in the collagen-free topical composition in an amount comprising the range of at least 0.2 wt% to at most 20.0 wt%, encompassing every value in-between. For example, in some embodiments, the vegetable oil may comprise at least 0.3 wt% of the collagen-free topical composition.

In addition, in this embodiment, the hempseed oil may be present in the collagen-free topical composition in an amount comprising the range of at least 10.0 wt% to at most 30.0 wt%, encompassing every value in-between. For example, in some embodiments, the hempseed oil may comprise at least 25.2 wt% of the collagen-free topical composition. In another example, in some embodiments, the hempseed oil may comprise at least 14.0 wt% of the collagen-free topical composition. The marine oil (e.g., cod liver oil) may be present in the collagen-free topical composition in an amount comprising the range of at least 10.0 wt% to at most 30.0 wt%, encompassing every value in-between. For example, in some embodiments, the marine oil may comprise at least 25.2 wt% of the collagen-free topical composition. In another example, in some embodiments, the marine oil may comprise at least 13.6 wt% of the collagen-free topical composition.

Moreover, in an embodiment, the caprylic acid (e.g., octanoic acid) may be present in the collagen-free topical composition in an amount comprising the range of at least 0.1 wt% to at most 3.0 wt%, encompassing every value in-between. For example, in some embodiments, the caprylic acid may comprise at least 0.3 wt% of the collagen-free topical composition. The red palm concentrate may be present in the collagen-free topical composition in an amount comprising the range of at least 0.1 wt% to at most 9.0 wt%, encompassing every value in-between. For example, in some embodiments, the red palm concentrate may comprise at least 0.3 wt% of the collagen-free topical composition. The palm olein may be present in the collagen-free topical composition in an amount comprising the range of at least 6.0 wt% to at most 16.0 wt%, encompassing every value in between. For example, in some embodiments, the palm olein may comprise at least 14.6 wt% of the collagen-free topical composition. In another example, in some embodiments, the palm olein may comprise at least 7.1 wt% of the collagen-free topical composition.

In addition, the analgesic (e.g., lidocaine) may be present in the collagen-free topical composition in an amount comprising at least 0.1 wt% to at most 1.5 wt%, encompassing every value in between. For example, in some embodiments, the analgesic may comprise at least 0.8 wt% of the collagen-free topical composition. The monolaurin (e.g., glyceryl laurate) may be present in the collagen-free topical composition in an amount comprising the range of at least 0.4 wt% to at most 15.0 wt%, encompassing every value in between. For example, in some embodiments, the monolaurin may comprise at least 0.6 wt% of the collagen-free topical composition. In another example, in some embodiments, the monolaurin may comprise at least 9.5 wt% of the collagen-free topical composition. Additionally, the cetyl esters may be present in the collagen-free topical composition in an amount comprising the range of at least 0.5 wt% to at most 5.0 wt%, encompassing every value in between. For example, in some embodiments, the cetyl esters may comprise at least 1.0 wt% of the collagen-free topical composition. In another example, in some embodiments, the cetyl esters may comprise at least 2.0 wt% of the collagen-free topical composition

Furthermore, in an embodiment, the ascorbyl palmitate may be present in the collagen-free topical composition in an amount comprising the range of at least 0.1 wt% to at most 1.5 wt%, encompassing every value in-between. For example, in some embodiments, the ascorbly palmitate may comprise at least 0.5 wt% of the collagen-free topical composition. All of the possible combinations and/or permutations of the components listed herein may be contemplated to be within the scope of the present collagen-free topical composition. However, the sum of all of the components present in the collagen-free topical composition may herein add to 100 wt%.

In an embodiment, the caprylic acid, the MCT, the omega-3, monolaurin (e.g., octanoic acid), fish collagen (e.g., hydrolyzed collagen) and/or squalene within the red palm oil may comprise antimicrobial, antifungal, and/or anti-viral properties, such that the topical compositions may comprise antimicrobial, antifungal, and/or anti-viral properties. In this manner, the monolaurin may be configured to signal for an immune cell response, As such, when the topical compositions may be disposed about at least one portion of the skin, the topical compositions may trigger the immune cell response within the skin. In this manner, the collagen-integrated topical composition and/or the collagen-free topical composition may comprise large bacterial (e.g., MRSA USA300 and/or PA27312) inhibition. For example, in some embodiments, at 24 hours, the topical formulations may have had similar areas of MRSA USA300 bacterial inhibition, all of which were significantly larger than silver sulfadiazine.

### Method of Formulation

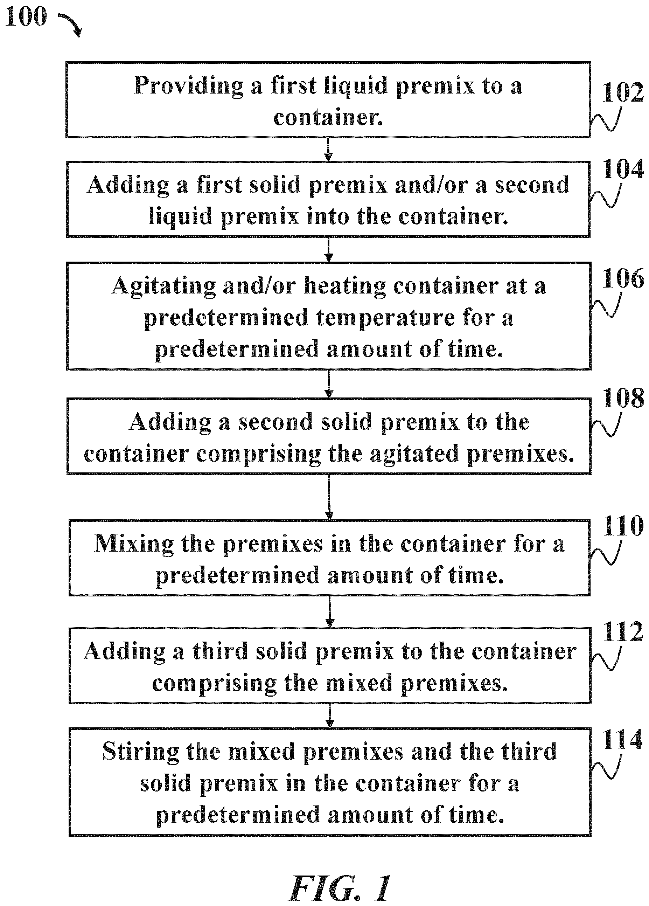

As shown in **FIG. 1****,** a method **100** is depicted for formulation a collagen-integrated topical composition. The steps delineated are merely exemplary of a preferred order for forming the collagen-integrated topical composition. The steps may be carried out in another order, with or without additional steps included therein. Additionally, the steps may be carried out with alternative embodiments of the collagen-integrated topical composition, as contemplated in the above description.

As shown in **FIG. 1****,** the method **100** for forming the collagen-integrated topical composition, begins with step **102,** providing a first liquid premix into at least one container. In an embodiment, the first liquid premix may comprise MCT, vegetable oil, hempseed oil, marine oil (e.g., cod liver oil) triethyl citrate, ethyl linoleate, and/or caprylic acid. The next step, step **104,** comprises adding a first solid premix and/or a second liquid premix into the container comprising the first liquid premix. As such, in this embodiment, the first solid premix may comprise monolaurin, cetyl esters, ascorbyl palmitate and/or beeswax. In this manner, the second liquid premix may comprise red palm concentrate. Next, in an embodiment, at step **106,** the container may be agitated and/or heated at a predetermined temperature (e.g., 165°F) for a predetermined amount of time (e.g., 15 minutes), such that the first liquid premix, the second liquid premix, and/or the first solid premix may be agitated, generating an agitated mixture. In an embodiment, the container may be agitated at an RPM of at least 10 Hz. At step **108,** a second solid premix may be added to the container with the agitated mixture. The second solid premix may comprise hydrolyzed fish collagen. In this embodiment, after the second solid premix has been added to the mixture, the container may be agitated at an RPM of at least 13 Hz to at most 20 Hz, encompassing every value in-between. In this manner, the predetermined temperature may be altered to greater than or equal to 130°F.

Additionally, as shown in **FIG. 1**, in an embodiment, at step 110 of method 100, the mixture within the container may be stirred together, while the container is being agitated and/or heated at the altered predetermined temperature. In this embodiment, the mixture may be stirred for at least three (3) minutes. Next, at step 112, a third solid premix may be added to the stirred mixture within the container. In this embodiment, the third solid premix may comprise the sea salt. Finally, at step 114, the third solid premix and the stirred mixture may be agitated and/or stirred for a predetermined amount of time (e.g., at least 3 minutes). As such, the mixture may then be covered at less than or equal to 97°F. In this embodiment, the mixture may then be transferred to at least on curing bowl for at least 3 days. Moreover, in this embodiment, after the 3 days, N₂ may be added to the curing bowl, while the mixture is stirred for at least 2 minutes. Furthermore, in this embodiment, the N₂ infused mixture may be covered and/or cured for at least 7 days. As such, after the at least 7 days, the N₂ infused mixture may comprise the collagen-infused topical composition, such that the collagen-infused topical composition may be transferred into at least one drum. In this manner, the at least one drum may be flushed with N₂ for at least 15 minutes.

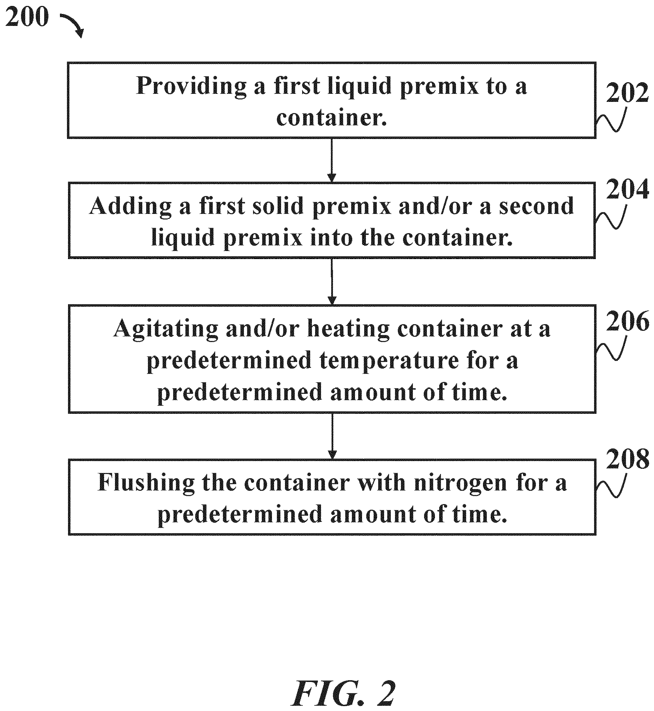

As shown in **FIG. 2**, a method **200** is depicted for formulation a collagen-free topical composition comprising an analgesic (e.g., lidocaine) (e.g., the lidocaine lavage). The steps delineated are merely exemplary of a preferred order for forming the collagen-free topical composition comprising the analgesic. The steps may be carried out in another order, with or without additional steps included therein. Additionally, the steps may be carried out with alternative embodiments of the collagen-free topical composition comprising the analgesic, as contemplated in the above description.

As shown in **FIG. 2****,** the method **200** for forming the collagen-free topical composition comprising the analgesic (e.g., lidocaine), begins with step **202,** providing a first liquid premix into at least one container. In an embodiment, the first liquid premix may comprise MCT, hempseed oil, marine oil (e.g., cod liver oil), caprylic acid, and/or palm oil (e.g., RBD Palm Oil - CP6). The next step, step **204,** comprises adding a first solid premix and/or a second liquid premix into the container comprising the first liquid premix. As such, in this embodiment, the first solid premix may comprise monolaurin, cetyl esters, and/or the analgesic (e.g., lidocaine). In this manner, the second liquid premix may comprise red palm concentrate. Next, in an embodiment, at step **206,** the container may be agitated and/or heated at a predetermined temperature (e.g., at least 140°F) for a predetermined amount of time (e.g., at least 15 minutes), such that the first liquid premix, the second liquid premix, and/or the first solid premix may be agitated, generating an agitated mixture. In an embodiment, the container may be agitated at an RPM of at least 13 Hz. In this embodiment, once the container reaches the predetermined temperature (e.g., 140°F), the heat may be turned off, such that the container may be cooled. Finally, as shown in **FIG. 2****,** at step **208** of method **200,** the container comprising the agitated and/or heated mixture may be flushed with N₂ for at least 5 minutes, such that the collagen-free topical composition comprising the analgesic may be formed. In this manner, after that least one 5 minutes, the container comprising the collagen-free topical composition comprising the analgesic may be sealed.

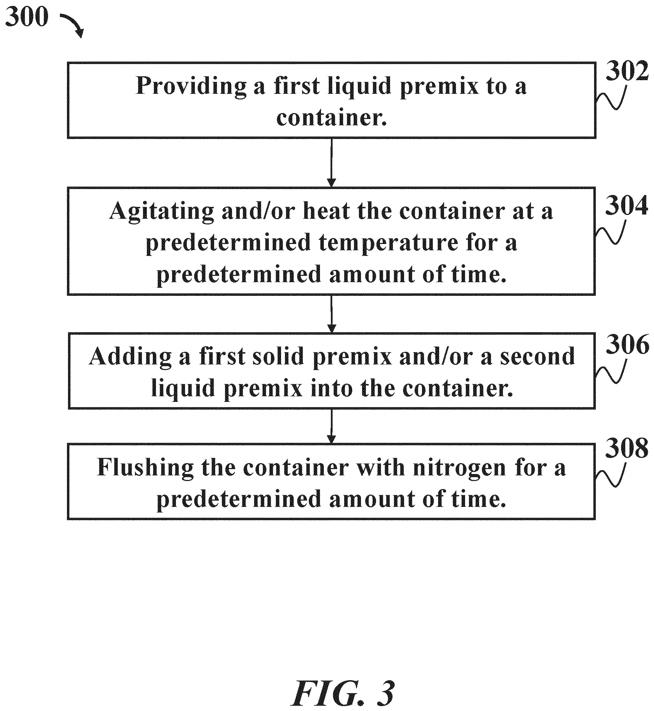

As shown in **FIG. 3****,** a method **300** is depicted for formulation a collagen-free topical composition. The steps delineated are merely exemplary of a preferred order for forming the collagen-free topical composition. The steps may be carried out in another order, with or without additional steps included therein. Additionally, the steps may be carried out with alternative embodiments of the collagen-free topical composition, as contemplated in the above description.

As shown in **FIG. 3****,** the method **300** for forming the collagen-free topical composition, begins with step **302,** providing a first liquid premix into at least one container. In an embodiment, the first liquid premix may comprise MCT, vegetable oil, hempseed oil, marine oil (e.g., cod liver oil) and/or palm oil (e.g., RBD Palm Oil - CP6). Next, in an embodiment, at step **304,** the container may be agitated and/or heated at a predetermined temperature (e.g., 120°F) for a predetermined amount of time (e.g., 15 minutes), such that the first liquid premix may be agitated, generating an agitated mixture. In an embodiment, the container may be agitated at an RPM comprising a range of at least 7 Hz to at most 10 Hz, encompassing every value in-between. The next step, step **306,** comprises adding a first solid premix into the container comprising the agitated mixture. As such, in this embodiment, the first solid premix may comprise monolaurin, cetyl esters, and/or ascorbyl palmitate. In this manner, the predetermined temperature may be altered such that the temperature may be greater than or equal to 160°F. Finally, as shown in **FIG. 3****,** at step **308** of method **300,** the container comprising the agitated and/or heated mixture may be flushed with N₂ for at least 5 minutes, such that the collagen-free topical composition may be formed. In this manner, after that least one 5 minutes, the container comprising the collagen-free topical composition may be sealed.

The following examples are provided for the purpose of exemplification and are not intended to be limiting.

### EXAMPLES

### Example 1

### Preparation of CS1i

CS1i is comprised of the following ingredients:
OMEZA CS1i GEL SKIN PROTECTANT

| | |
|---|---|
| MCT Oil | 53.00% |
| Monolaurin | 9.50% |
| Cetyl esters NF | 2.00% |
| virgin coconut oil | 0.33% |
| palm oil | 7.11% |
| ascorbyl palmitate | 0.50% |
| Hempseed oil | 14.00% |
| Cod Liver Oil | 13.56% |
| Colloidal Oatmeal | 0.00% |
| **Total** | 100.00% |

| | |
|---|---|
| (ALA+SDA)/(EPA+DHA) [PUFA Ratio] | 1.00 |
| ALA+SDA+EPA+DHA (sum omega3) | 5.75 |
| (C8+C10)/sum(other triglycerides) Penetration Ratio | 1.59 |

It is prepared as described hereinabove:

MCT, Hempseed oil, Cod liver oil, monolaurin, cetyl esters, coconut oil and palm oil are heated with mixing to 185°F in a glass vessel until a homogenous mixture is obtained. The mixture is then cooled with stirring at 1.5 rpm in a Kitchen AID mixer ("1) to 93°F. AP, monolaurin and cetyl esters sequentially freeze. The mixture is cooled overnight to room temperature. The resulting residue may be a two-phase, striated mixture of oil and frozen fat/wax. The mixture is then reheated to120°F with 3rpm stirring with a standard paddle mixer, whereby the frozen fat/wax mixture melts, but the aspartyl palmitate is not melted. The 120°F homogenous mixture is pumped to a filling machine, which is used to fill the mixture into sealed plastic containers. As the mixture cools to room temperature in the container, it forms a gel.

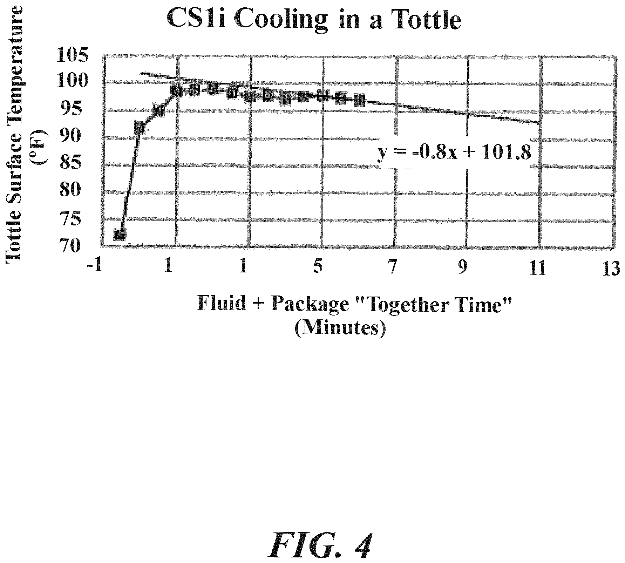

Gelation takes place in 1minute as shown in **FIG. 4**. The hot melt and cold plastic come to thermal equilibrium at a temperature less than the fat/wax gelation temperature (~102°F).

Without wishing to be bound, it is believed that the mechanism of action is as follows:

AP crystals form during agitated process cooling at -122°F. The level of AP determines the number of crystal nuclei formed. If there are too few AP nuclei, the gels are large. If there are too many AP nuclei, the gels may be too small. The range of the AP provided in this disclosure is found to be the proper amount, with the best results at 0.5 wt%. When the mixture cools without agitation, phase separation occurs. The fat/wax mixture starts to melt at about 118°F, below the melting point of AP nuclei, and the fat/wax mixture is all liquid at about 120°F. When the hot melt is filled into cold plastic containers, a new temperature equilibrium is established below the melting point of fat/wax mixture, and a gel forms around each AP nuclei. The gelled, closed package continues to lose heat after package sealing in ways well-known to skilled artisans.

### COMPARATIVE EXAMPLE 1

CS1c contains the following ingredients:

| | Omeza Gel CS1c Skin Protectant |
|---|---|
| MCT Oil | 52.09% |
| Hempseed oil | 12.00% |
| Cod Liver Oil | 16.21% |
| Monolaurin | 9.50% |
| Cetyl esters NF | 2.00% |
| coconut oil | 0.33% |
| colloidal oatmeal | 0.50% |
| RBD Palm Oil | 7.12% |
| ascorbyl palmitate | 0.25% |
| Total | 100.00% |
| (C8+10)/(sum other triglycerides) - Penetration Factor | 1.54 |
| (ALA+SDA)/(EPA+DHA) [PUFA Ratio] | 0.75 |
| ALA+SDA+EPA+DHA (sum omega3) | 6.01 |

Omeza Gel CS1c has 0.5% colloidal oatmeal, a skin protectant active ingredient and 0.25% ascorbyl palmitate. The ascorbyl palmitate is a process aid to stabilize the gel at about 40°C (potential distribution temperature abuse).

CS1c is prepared as described hereinabove with respect to CS1i.

CS1i is more stable and has a longer shelf life than CS1c and CS1.

| | Omeza Gel CS1 Skin Protectant |
|---|---|
| MCT Oil | 52.09% |
| Hempseed oil | 12.00% |
| Cod Liver Oil | 16.21% |
| Monolaurin | 9.50% |
| Cetyl esters NF | 1.00% |
| virgin coconut oil | 0.33% |
| Colloidal oatmeal | 0.50% |
| RBD palm oil | 8.25% |
| perfume B | 0.12% |
| Total | 100.00% |
| (ALA+SDA)/(EPA+DHA) [ PUFA Ratio] | 0.75 |
| ALA+SDA+EPA+DHA (sum omega3) (C8+C10)/sum (other triglycerides) - | 6.02 |
| Penetration Factor | 1.49 |

AP was added to CS1 (to make CS1i) to solve a stability problem. Stability in CS1 and CS1c fail in three ways:

With too low mixing at about 120°F, colloidal oatmeal separates out.

This is acceptable in single use packages because both phases are extruded during one-time use and then rubbed in, making the as-applied concentration constant. However, in multi-use packaging this defect is not acceptable as the first use is not the same concentration as the last use.

When the MCT>50%, oil can separate to the bottom of the container during temperature excursions (e.g., in a hot trailer truck). Oil has a specific gravity of 0.92. Gel specific gravity -0.87. After a multi-use package is opened, shear occurs as remaining product moves towards the package outlet through narrow aperture dispensing means. In wide-mouth containers, the wipe of "fingers" into the gel shears adjacent mixture. Shearing creates capillaries between gel sites. High-density oil is transported by capillary action to the low-density surface and seen by the consumer as a separated, surface-oil defect.

The unexpected addition of AP in the absence of colloidal oatmeal solves this problem by making uniformly sized gels that do not allow the local capillaries to form when sheared. The viscosity increases as AP concentration increases. Moreover, AP addition resolved the multi-use package stability issue without compromising the cosmetic elegance of the gel itself.

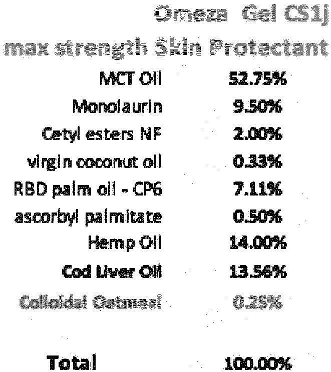

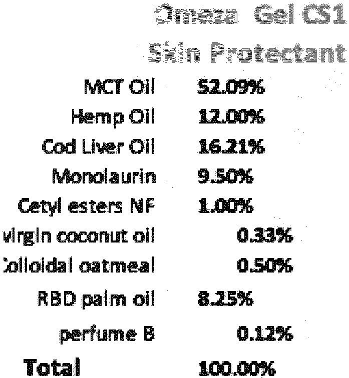

### COMPARATIVE EXAMPLE 2:

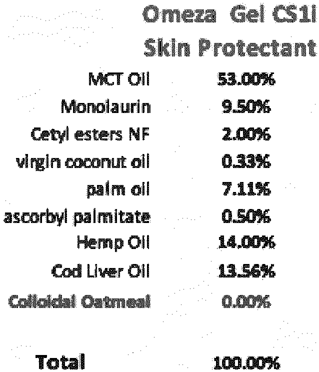

CS1 and CS1j, the compositions of which are described herein, are prepared as described hereinabove for CS1i. The effect of colloidal oatmeal was explored by comparing the stability of CS1i which has no colloidal oatmeal with CS1j which has 0.25 wt% of colloidal oatmeal and CS1, which has 0.5 wt% of colloidal oatmeal. In CS1j and CS1, there was surface oil separation with AP at about 0.5 wt% and 0.25 wt% colloidal oatmeal, respectively. CS1i is stable.

### EXPLANATION OF COMPARATIVE EXAMPLE 2

Anhydrous oil/wax mixture stability is difficult to predict because there are multitudes of eutectic freezing temperatures. Stability fails three ways:

Undissolved solids (e.g., colloidal oatmeal) can precipitate during gelation. Heavy oils sink through the lighter gel and accumulate on the bottom of the package. Surprisingly, heavy oils can percolate to the surface as free oil due to capillary action after shear.

It was found that colloidal oatmeal may be suspended if gelation takes place within a 3minute window. This is accomplished by quenching hot, stirred melt with a cold package such that the commingled temperature < 102°F. The produced gel is stable until sheared. When sheared the gel breaks down and the product rubs into the skin.

The colloidal oatmeal gels may work well for single use packages. In multi-use packages, the seemingly stable gel is sheared during dispensing and develops capillary channels adjacent to the source of shear. The consumer sees unacceptable floating clear oil and unsheared gel on the subsequent application.

After extensive experimentation, an AP level of 0.5 wt% colloidal oatmeal was ideal along with the elimination of colloidal oatmeal for multi-use packages (CS1i). Packaged shelf life is 24months; there is no observed leakage in a 150use large package.

### EXAMPLE 2

### Preparation of K7ca

K7ca has the following composition:

| | |
|---|---|
| MCT Oil | 61.00% |
| Monolaurin | 10.00% |
| Cetyl esters NF | 5.84% |
| ascorbyl palmitate | 0.50% |
| Hempseed oil | 2.47% |
| Cod Liver Oil | 2.36% |
| rice bran wax | 7.00% |
| camphor | 5.30% |
| caprylic acid | 2.53% |
| GABA | 1.00% |
| Melatonin | 1.00% |
| glutathione | 1.00% |
| **Total** | 100.00% |
| (ALA+SDA)/(EPA+DHA) [PUFA Ratio] | 1.00 |
| ALA+SDA+EPA+DHA (sum omega3) | 1.00 |
| (C8+10)/(sum other triglycerides) Penetration Factor | 13.19 |

Structured gels form large micelle-like structures with huge macro interior encapsulating volume. The spherical structure is formed with a surfactant skin reinforced with needle-like rice bran wax crystals and layers of frozen esters. This structure allows topical delivery of nutraceuticals, API, minerals and the like. K7ca is an example of how nutraceuticals (GABA (gamma aminobutyric acid), melatonin & glutathione) and volatile compounds (camphor, caprylic acid) may be combined and then used topically. The high Penetration Factor (>3) drags the calming nutraceuticals into the skin while concurrently, body heat volatilizes the light compounds.

The net effect is to put the user to sleep faster.

### EXPLANATION OF EXAMPLE 2

Camphor and caprylic acid are volatile and when applied in K7ca to the chest, the volatiles are breathed into the nose and lungs. Caprylic acid is an antimicrobial compound. Wheezing is eliminated and the nose clears. Breathing becomes easier and the patient sleeps soundly.

GABA, melatonin, and glutathione are nutraceuticals with purported calming attributes. Together, easier breathing and calming compounds induce deeper sleep.

### EXAMPLE 3

### Preparation of K7LH5

K7LH5 has the following composition:

| | **Remodeling Cream** - **K7LH5 OTC Skin Protectant Lotion** |
|---|---|
| MCT | 68.00% |
| Monolaurin | 10.00% |
| Cetyl Esters NF | 5.84% |
| Ascorbyl Palmitate | 0.50% |
| Hempseed oil | 2.41% |
| Cod Liver Oil | 2.36% |
| RBD Palm Oil | 3.00% |
| Rice Bran Wax | 7.00% |
| Squalene/Vitamin E | 0.20% |
| Colloidal Oatmeal | 0.10% |
| Caprylic Acid | 0.23% |
| Coconut Oil | 0.33% |
| Fragrance | 0.03% |

| | |
|---|---|
| **Total** | 100.00% |
| (ALA+SDA)/(EPA+DHA) [PUFA Ratio] | 1.00 |
| ALA+SDA+EPA+DHA (sum omega3) | 1.00 |
| (C8+10)/(sum unsat other triglycerides) Penetration Ratio | 8.47 |

K7LH5 is prepared in accordance with the procedure described herein.

It is an optimized formula with Sum of omega3 = 1.0%, with <5% cod liver oil and MCT >60% and <80%. Importantly, K7LH5 has 7% rice bran wax (>4% and <10%). The PUFA ratio is >0.5 and <1.5. Caprylic acid (C8 FFA) is used as a process aid to control viscosity (mean viscosity ~ 700,000cp±15%). Post cooling whipping (Kitchen Aid mixer at about "8" for 15 seconds) can reduce the viscosity by 67%. The time of whipping may be adjusted to match the viscosity and specific gravity target.

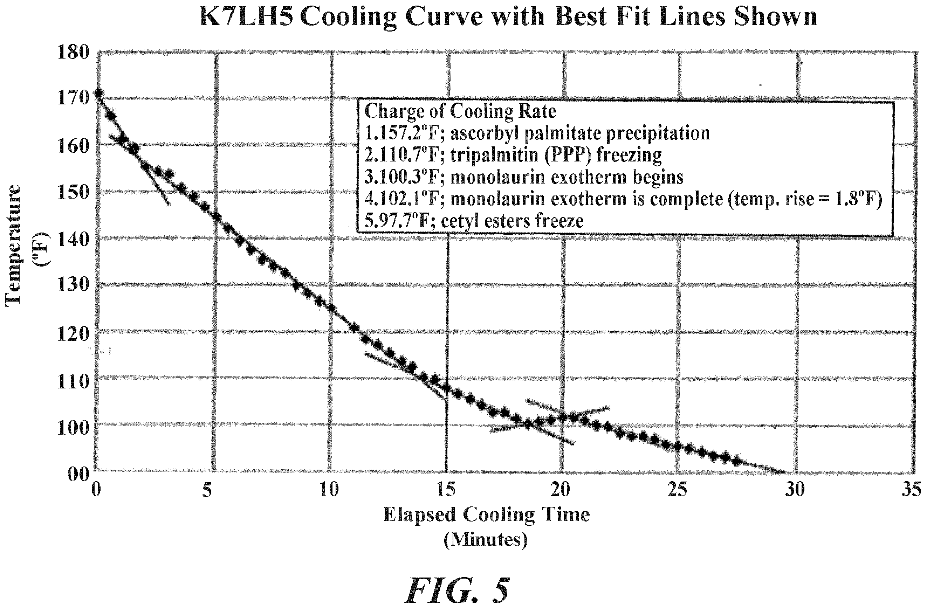

**FIG. 5** shows the cooling curve of K7LH5 with 5 eutectic temperatures, each adding structural support (increasing viscosity).

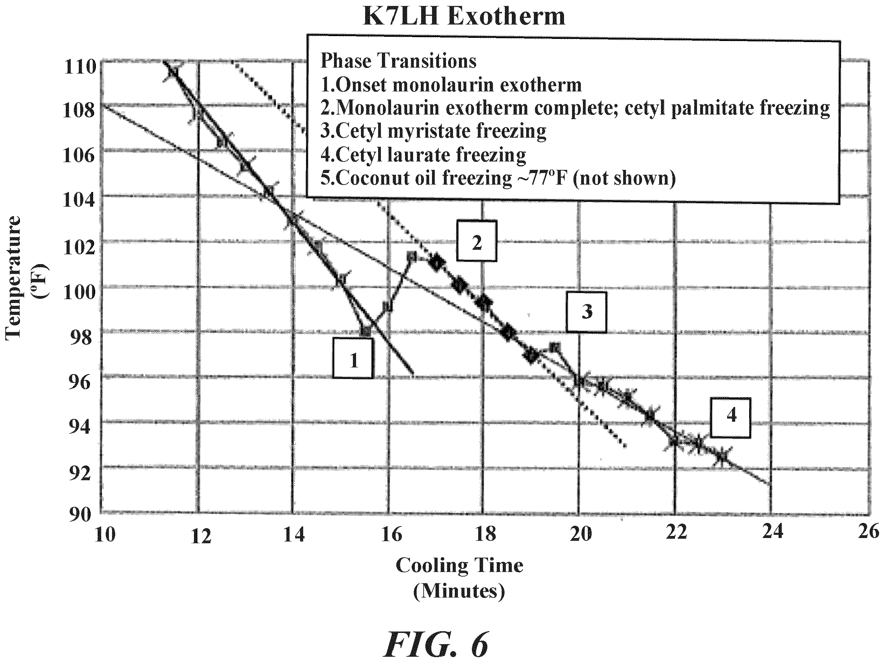

**FIG. 6** shows the cooling in more detail such that the constant temperature tie lines of three cetyl esters freezing is seen.

Solid coconut fats (C12:0 and C14:0) congeal overnight to achieve a final, stable viscosity.

K7LH5 is designed for healthy skin. It is particularly advantageous for remodeling recently healed wounds and for reducing scarring.

For example, a badly burned 47 year old female applied K7LH5. After 5weeks of daily use, K7LH5 converted a large donor site into normal skin with normal skin color. Her grafted burns took longer to remodel, but at 7weeks, the skin texture was normal (smooth to the touch).

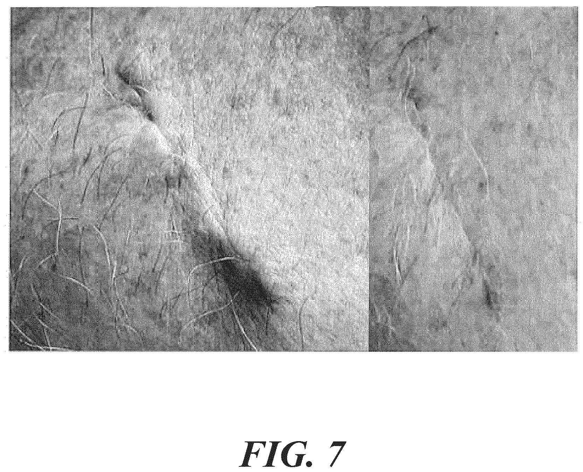

As another example, as shown in **FIG. 7****,** a 79 year old man had a pacemaker installed with a 4inch glued-together entry site. The wound was closed with adhesive, not stitches or staples. The base of the wound became infected and was treated with a topical antibiotic. The wound is depicted on the left side. It was raised and irregular. After 4 weeks of daily morning application, the scar was less than 2 mm wide. The scar had normal flesh tone each morning; after application, the scar was pink for ~8hours each day before reverting to normal color. After 45 days of daily treatment with K7LH5, the raised scar tissue disappeared, as depicted on the right side of **FIG. 7****.**

In another example, a thin, 47year old female was badly burned on both legs and then had a large skin graft donor site on her undamaged thigh. The patient applied 1g/squirt to the lower leg and 1g/squirt to the upper leg daily of K7LH5, a total of 4g/day. Assuming her leg area was 4*1.6m² (upper + lower leg * 2 legs) her usage was 4g/day/4*1.6m² = 0.6g/m². After two months, there was no visible scarring in the donor site or in the graft site (except the suture hole scars were visible). In this example, the patient treated both entire legs daily, not just the burned area, because she was so pleased with the elegance of her skin.

Visible light inspection (Keyence VHX-7000 Digital Microscope) revealed that rice bran wax forms long needle-like crystals (well-known). In the presence of previously formed ascorbyl palmitate nuclei (previously unknown), the needle crystals form in, among and around surfactant "skin" (monolaurin) of micelles, reinforcing the strength of the micelle structure after cooling. The net effect is to create robust hollow spheres that are filled with "leaky oil" or other "cargo." When rubbed in, the oil is released, and the waxy/surfactant skin forms an occlusive moisture-barrier film over the application site.

### EXPLANATION OF EXAMPLE 3

When injured skin is healing, the body increases the vascular infrastructure to bring tissue-building necessary nutrients and oxygen to the wound site. Temporary collagen (scar tissue) is deposited to seal the wound. Once sealed, the body signals to remove excess vascularization and to replace temporary collagen with permanent collagen. Doctors call this 1-2year process remodeling. If the scar is under stress or strain, for example contracting to seal the wound site, scar tissue increases in unwanted ways.

K7LH5 has >60% MCT to provide energy to adjacent cells. MCT-fed cells produce macrophages, the debris cleanup organisms the body uses to remove detritus. Rice bran wax precipitates immediately upon transfer from the heating vessel to the cooling vessel. The wax forms into waxy crystals, not unlike melted candle wax. Ascorbyl palmitate precipitates second, forming an array of tiny crystal nuclei. As soon as the AP nuclei form, rice bran wax goes through a crystal reorganization to form a series of long needle like crystals around the AP nuclei. The earlier waxy precipitate is removed from the vessel walls and morphs into needles. Cooling continues until tripalmitin (PPP) freezes, from the palm oil coating the rice bran wax needles. Eventually, monolaurin begins to freeze (exothermic increase in temperature) forming large micelles encapsulating nonfrozen oils. The surfactant is reinforced by rice bran wax-coated AP nuclei and tripalmitin frozen fat making a stronger skin around the liquid oils.

The structure is further reinforced by sequential freezing of cetyl stearate, cetyl myristate and cetyl laurate on the outside of the reinforced surfactant skin. [The cetyl esters could be precipitating on the inside of the monolaurin skin.] The actual location is not currently known. Overnight, coconut fats freeze and make the encapsulated oil "slushy." Viscosity increases to 700,000cp (very thick). If the cold product is whipped in a mixing bowl, viscosity will drop from 700,000cp to 200,000cp and then recover overnight to 500,000cp. Whipping reduces specific gravity from 0.91 to 0.87. The length of the whipping is adjusted to match specific gravity and viscosity specifications in ways well known to skilled artisans.

Rub in releases encapsulated liquid oils. Rub in is almost instantaneous because permeation enhancing MCT is >60% and sum omega3 is 1%. This results in a Penetration Factor of 8.47, 5X greater than the Penetration Factor of CS1i (1.59), a relatively unstructured gel. K7LH5 is called a structured gel because of the stable monolaurin micelle formation in an anhydrous medium.

K7LH5 rubs in fast but leaves behind a silky smooth, moisture barrier wax layer.

What is believed to happen, without wishing to be bound is that each micelle ruptures, releasing liquid oils that penetrate the epithelium. The wax-reinforced surfactant skin forms a uniform occlusive layer over the treated intact skin and reduces trans-epidermal water loss (TEWL). The three sequential layers of cetyl esters wind up on the outside of the occlusive wax layer, making the occlusive layer silky smooth to the touch. It is quite remarkable that a formula with over 23% wax has a wonderful, silky smooth consumer feel.

Scarring is reduced indirectly. Without wishing to be bound, the occlusive layer keeps the wounded/closed skin moisturized and supple, allowing the skin to minimize shear/stress. The oils lubricate the corneocytes further reducing response to stress. Maintenance levels of nutrients/omega3/omega6 provide raw materials for the temporary-to-permanent collagen transition (in a low stress/strain environment).

Finally, low levels (0.23%) of C8 free fatty acid help buffer skin pH to allow matrix metalloprotease enzymes to deconstruct temporary collagen. All this complexity is invisible to the user. But it is simple to apply to the wound, burn and/or skin condition: 1. Push the bottle pump to get 1ml of K7LH5; 2. Rub in for 3-4seconds; and 3. Get dressed.

### EXAMPLES 4 AND 5

Further examples were prepared in accordance with the methods described herein:

| | **Omeza Sleep Gel PM3j OTC Skin Protectant Gel** |
|---|---|
| MCT Oil | 62.46% |
| Monolaurin | 10.00% |
| Cetyl esters NF | 5.84% |
| virgin coconut oil | 4.50% |
| camphor | 4.70% |
| ascorbyl palmitate | 0.50% |
| Hempseed oil | 5.10% |
| Cod Liver Oil | 4.90% |
| C8 FFA | 2.00% |
| **Total** | 100.00% |
| | |
| (ALA+SDA)/(EPA+DHA) [PUFA Ratio] | 1.00 |
| ALA+SDA+EPA+DHA (sum omega3) | 2.07 |
| (C8+C10)/sum(other triglycerides) Penetration Ratio | 4.80 |

| | Psoriasis Cream - K7dd1 Psoriasis Lotion |
|---|---|
| MCT Oil | 65.86% |
| Monolaurin | 10.00% |
| Cetyl esters NF | 5.84% |
| ascorbyl palmitate | 0.50% |
| Hempseed oil | 2.41% |
| Cod Liver Oil | 2.36% |
| RBD Palm Oil | 3.00% |
| rice bran wax | 7.00% |
| squalene/Vitamin E | 0.50% |
| lidocaine | 0.40% |
| salicylic acid | 1.80% |
| virgin coconut oil | 0.33% |
| Total | 100.00% |
| | |
| (ALA+SDA)/(EPA+DHA) [PUFA Ratio] | 1.00 |
| ALA+SDA+EPA+DHA (sum omega3) | 1.00 |
| (C8+C10)/sum(other triglycerides) Penetration Ratio | 7.93 |

PM3j is a sleep aid with camphor as the active ingredient. C8 FFA (caprylic acid) is not volatile at ambient temperatures but is active at roughly the body temperature of a human. It is rubbed onto the upper chest in amounts sufficient clear the sinus from mucus and to reduce wheezing and snoring. In an embodiment, this PM3j composition is rubbed onto the upper chest in about 0.1g//m²/day to about 2 g/m²/day, and/or in another embodiment, at about 0.5 g/m²/day to about 2 g/m²/day, and/or in another embodiment, at about 0.5 g/m²/day to about 1 g/m²/day.

When PM3j is rubbed onto the upper chest, camphor and C8 FFA co evaporate and are breathed into the nose. The sinus clears and any wheezing in the lungs stops. The mechanism is unclear, but it is believed that C8 FFA is an antimicrobial that reduces low grade infections in both the sinus and the lungs. the result is quite striking: 1. snoring is reduced, 2. a better night's sleep is achieved, and 3. over time, wheezing is eliminated.

K7dd1 is a healing lotion applied to psoriasis sufferers after psoriasis scales are physically abraded from the site. K7dd1 uses salicylic acid as the active ingredient. U.S. regulations do not allow mixing salicylic acid with other active ingredients, so CLO and lidocaine are at low levels (e.g., below the active ingredient level). Rice bran wax is used to form an occlusive layer over treated skin to reduce TEWL.

It may be applied onto the area where psoriasis is found on the skin in therapeutically effective amounts. In an embodiment, the amount of the collagen composition applied may be 0.1g//m²/day to about 2 g/m²/day, and/or in another embodiment, at about 0.5 g/m²/day to about 2 g/m²/day, and/or in another embodiment, at about 0.5 g/m²/day to about 1 g/m²/day until the skin is clear.

### EXAMPLE 6

### Preparation of K7at

**K7at** is prepared in accordance with the procedure described herein.

| | **Omeza Lotion K7at Antitussive Lotion** |
|---|---|
| MCT Oil | 62.00% |
| Monolaurin | 10.00% |
| Cetyl esters NF | 5.84% |
| caprylic acid | 2.00% |
| ascorbyl palmitate | 0.53% |
| Hempseed oil | 2.41% |
| Cod Liver Oil | 2.36% |
| RBD Palm Oil | 2.96% |
| rice bran wax | 7.00% |
| camphor | 4.70% |
| squalene/Vitamin E | 0.20% |
| **Total** | 100.00% |
| (ALA+SDA)/(EPA+DHA) [PUFA Ratio] | 1.00 |
| ALA+SDA+EPA+DHA (sum omega3) (C8+10)/(other triglycerides) Penetration | 1.00 |
| Ratio | 8.03 |

The percent are in weight percent.

### EXAMPLE 7

### Preparation of Collagen Matrix Antimicrobial Barrier

The following compositions are prepared as described herein:
**Collagen Matrix Antimicrobial Barrier**
**Collagen Matrix Pressure Sores CR21b**

| | **i. Weight %** |
|---|---|
| MCT Oil | 8.00% |
| Coconut fatty acid | 2.10% |
| Monolaurin | 4.00% |
| Cetyl Esters | 2.00% |
| Yellow beeswax | 4.00% |
| Virgin coconut oil | 10.00% |
| Ascorbyl Palmitate | 0.50% |
| Hempseed oil | 11.65% |
| Cod Liver Oil | 11.20 % |
| Fish Collagen | 41.40% |
| Triethyl citrate | 2.00% |
| Ethyl linoleate | 2.00% |
| Benzethonium chloride | 0.20 % |
| Lidocaine | 0.45% |
| Squalene/vitamin E | 0.20% |
| Total | 100.00% |
| | |
| C8 + 10/sum /Unsat Fat( Penetration Factor) | 0,50 |
| Sum wax (Retentate Factor) | 10.80 |
| (ALA + SDA)/(EPA + DHA) (PUFA Ratio) | 1.00 |
| ALA+SDA+EPA+DHA (sum omega 3) | 4.74 |
| n6/n3 | 1.46 |

OMEZA EYE OIL-RG7d
MAX STRENGTH SKIN PROTECTANT

| | Weight% |
|---|---|
| MCT Oil | 47.84% |
| Coconut oil | 4.00% |
| Monolaurin | 0.60% |
| Cetyl Esters | 1.50% |
| RBD Palm Oil-CP6 | 2.50% |
| Hempseed oil | 26.10% |
| Cod Liver Oil | 13.56% |
| Squalene/Vitamin E | 0.20% |
| Lidocaine | 0.70% |
| Camphor | 3.00% |
| | |
| TOTAL | 100.00% |
| C8 + 10/sum /Unsat Fat( Penetration Factor) | 1.42 |
| Sum wax (Retentate Factor) | 5.10 |
| (ALA + SDA)/(EPA + DHA) (PUFA Ratio) | 1.75 |
| ALA+SDA+EPA+DHA (sum omega 3) | 7.91 |
| n6/n3 | 1.96 |

Pressure wounds are different from venous ulcers, diabetic foot ulcers and acute surgical wounds.

The following are key differences that include: (1) the wounds are deep and often tunneled; (2) the wounds are painful; (3) debridement often makes the wound larger without initiating healing; (4) the wounds usually smell from fecal/urine contamination; (5) it is difficult to permanently offload and protect the wound site; (6) the patients skew elderly and often are in facilities for the infirm; (7) caregivers are often marginally trained health care persons; (8) colorful products often stain garments and bedding; and/or (9) the patients are usually nutrient challenged.

The products for sacral wounds must be designed to meet these differences: (1) no color; (2) antimicrobial activity; (3) collagen availability; (4) temporary moisture barrier; (5) nothing abrasive; (6) fragrance; (7) pain relief; and/or (8) topical nutrition.

CR21b and RG7d are designed to work together as a two-part system: (1) saturate the wound with PUFA oils; (2) temporarily mask pain and odor; (3) pack the wound with slow-release collagen, PUFA oils, and slow-release pain relievers; (4) provide Vitamins, A, D, C and/or E; (5) create a temporary semi-occlusive moisture loss barrier over the sacral wound.

Importantly, CR21b contains monolaurin and lauric acid (from coconut fatty acid). Monolaurin and lauric acid synergistically work together to control the pathogen population. The 2:1 ratio of monolaurin to lauric acid is much higher than the ratios in other formulations because no other Omeza formulation uses coconut free fatty acid.

RG7d has a PUFA ratio (1.75). RG7d has two pain relief compounds, lidocaine, and/or camphor. RG7d is a clear, light-yellow oil. CR21b is a first aid antiseptic (benzethonium chloride), skin protectant (CLO) and pain reliever (e.g., lidocaine & camphor).

Vitamins, trace minerals, carbon (from MCT) are needed to form new epidermis. These nutrients are provided topically by first an oil/gel and then later by a paste. They cannot be combined into a single fluid because of phase separation.

### EXAMPLE 8

### Preparation of Psoriasis Two Part Solution

The following compositions are prepared as described in the disclosure:
**Collagen Matrix DD5**
**Max Strength Psoriasis Scrub**

| Weight % | |
|---|---|
| MCT Oil | 9.70% |
| Salicylic acid | 3.00% |
| Squalene/Vitamin E | 1.00% |
| Monolaurin | 4.30% |
| Red palm concentrate | 0.50% |
| Yellow beeswax | 4.00% |
| Virgin Coconut Oil | 10.00% |
| Ascorbyl Palmitate | 0.50% |
| Hempseed oil | 12.31% |
| Cod Liver Oil | 11.79% |
| Fish Collagen | 41.40% |
| Sea salt | 1.50% |
| Total | 100.00% |
| | |
| (ALA+SDA)/(EPA+DHA) [PUFA Ratio] | 1.00 |
| ALA+SDA+EPA+DHA (sum omega3) | 5.00 |
| (C8+10)/(sum other triglycerides) Penetration Ratio | 0.34 |

**PSORIASIS CREAM- K7dd1 PSORIASIS LOTION**

| Weight % | |
|---|---|
| MCT Oil | 65.86% |
| Monolaurin | 10.00 % |
| Cetyl Esters | 5.84% |
| Ascorbyl palmitate | 0.50% |
| Hempseed oil | 2.41% |
| Cod Liver Oil | 2.36% |
| RBD Palm Oil | 3.00% |
| Rice bran wax | 7.00% |
| Squalene/Vitamin E | 0.50% |
| Lidocaine | 0.40% |
| Salicylic acid | 1.80% |
| Virgin coconut oil | 0.33 % |
| Total | 100.00% |
| | |
| (ALA+SDA)/(EPA+DHA) [PUFA Ratio] | 1.00 |
| ALA+SDA+EPA+DHA (sum omega3) | 1.00 |
| (C8+C 10)/sum(other triglycerides) Penetration Ratio | 7.93 |

a. Psoriasis is a condition in which skin cells build up and form scales and itchy, dry patches. It is caused by immune system malfunction and is considered incurable. However, people who suffer from psoriasis may become asymptomatic if the dry scaly patches are scrubbed off and then the raw skin is treated with a hydrating, soothing lotion, or gel that inhibits new scale formation.
b. Salicylic acid is a beta hydroxy acid widely used to exfoliate dead and dying skin.

Psoriasis Cream (K7dd1) is a structured gel with cod liver oil <5% and no colloidal oatmeal plus 1.8% salicylic acid. There is a low level of lidocaine (0.4%) to help provide temporary itching relief.

Importantly, DD5 has a very low Penetration Factor (0.54); K7dd1 has a very high Permeate Factor (11.68). DD5 is rubbed onto psoriatic skin to help exfoliate the psoriatic scale and damaged skin. This is a necessarily rough operation and underlying skin is damaged. After DD5 is rinsed off with water, K7dd1 is rubbed into the exfoliated area. The skin heals and the psoriatic itching stops. The two-part use of co-designed formulations allows overly aggressive exfoliation that is ameliorated by a compatible anhydrous moisturizer.

In this process, a surface skin problem with a non-absorbing, mildly abrasive product is treated sequentially followed by a rapidly absorbed, soothing product that inhibits recurrence.

Psoriatic treatments are required by regulation (OTC) to have salicylic acid between 1.8 and 3.0%. The structured gel, high Penetration Factor carrier drags the salicylic acid deeper into the epidermis zone and/or dermis zone to prevent more surface damage and help prevent new scales to form.

The net effect is to remove the old scales (both chemically and mechanically) and inhibit new scale development.

### EXAMPLE 9

### Preparation of Omeza Burn Stuff RG 10 Spray

| **Omeza Burn Stuff RG10** | |
|---|---|
| **Spray** | |
| **MCT Oil** | **72.14%** |
| **Monolaurin** | **0.60%** |
| **Cetyl esters NF** | **1.50%** |
| **coconut oil** | **2.50%** |
| **Hemp Oil** | **7.39%** |
| **Cod Liver Oil** | **7.09%** |
| **coconut fatty acid** | **2.50%** |
| **benzethonium chloride** | **0.10%** |
| **squalene/Vitamin E** | **2.48%** |
| **lidocaine** | **0.70%** |
| **camphor** | **3.00%** |
| | |
| **Total** | **100.00%** |
| **(C8+10)/(sum other triglycerides) Permeation Factor** | **4.00** |

Omeza Burn Stuff Rg10 Spray is prepared as described above. Users report immediate and/or long lasting chronic pain and/or burn relief.

### EXAMPLE 10

### Preparation of Omeza CN9:

CN9 has the following composition:

| **Omeza Deep Pain Relief - CN9** | |
|---|---|
| **4% + camphor+CBD** | |
| **MCT Oil** | **49.40%** |
| **Hemp Oil** | **14.20%** |
| **Cod Liver Oil** | **13.56%** |
| **Monolaurin** | **10.00%** |
| **Cetyl esters NF** | **2.14%** |
| **CBD** | **3.70%** |
| **Lidocaine** | **4.00%** |
| **Camphor** | **3.00%** |
| **Total** | **100.00%** |
| | |
| **(ALA+SDA)/(EPA+DHA) [PUFA Ratio]** | **1.00** |
| **ALA+SDA+EPA+DHA (sum omega3)** | **5.76** |
| **(CS+10)/(sum total other triglycerides) Penetration Ratio** | **1.88** |

CN9 is prepared as described herein for gels. CN9 is an unstructured gel. Users report immediate and lasting chronic pain relief.

### EXPLANATION OF EXAMPLE 10

CN9 is prepared to transport CBD into the blood stream. CBD dissolves readily in MCT. Without wishing to be bound; cod liver oil and hempseed oil help transport CBD into the dermis. Lidocaine and camphor are OTC active topical analgesic compounds. Camphor may provide immediate cooling pain relief Lidocaine may provide longer lasting, but temporary, pain relief. MCT and omega3 reduce inflammation, directly and indirectly providing long term pain relief by addressing the root cause of the pain. Together, deep pain relief is possible.

CN9 may provide immediate and long-term pain relief without opioids or any other APIs.

### EXAMPLE 11

### Preparation of Omeza RG7:

RG7 has the following composition:

| **Omeza Eye Oil - RG7** | |
|---|---|
| **Max Strength Skin Protectant** | |
| | **Weight %** |
| **MCT Oil** | **51.54%** |
| **coconut oil** | **4.00%** |
| **Monolaurin** | **0.60%** |
| **Cetyl Esters NF** | **1.50%** |
| **RBD Palm Oil - CP6** | **2.50%** |
| **Hemp Oil** | **26.10%** |
| **Cod Liver Oil** | **13.56%** |
| **squalene/Vitamin E** | **0.20%** |
| **Total** | **100.00%** |
| | |
| **(ALA+SDA)/(EPA+DHA) [PUFA Ratio]** | **1.75** |
| **ALA+SDA+EPA+DHA (sum omega3)** | **7.91** |
| **(C8+10)/(sum other triglycerides) Penetration Ratio** | **1.18** |

RG7 is prepared as described herein above for oils. RG7 is designed to reduce the bags under eyes. It is designed to stay on the surface of the eye bags and not penetrate rapidly because the undereye skin is very thin and if absorbed quickly, the omegas will be lost to the body as a whole (Penetration Factor = 1.18). RG7 also has a PUFA Ratio outside the normal range (1.75).

The technique is to put 0.2ml of oil of one palm and roll a cotton-tipped swab in the puddle. The oily swab is rolled over one eye bag and then the other after showering.

Oil spreads out over the entire eye bag within seconds. The oil is absorbed over 5minutes-- a long time if rubbed into the skin, but with eye bags, rubbing is never appropriate. Within one week, users' eye bags are visibly smaller, reaching a new equilibrium in about 1-2 months.

### EXPLANATION OF EXAMPLE 11

RG7 is a clear oil, so monolaurin and cetyl esters are at their saturation levels. MCT is >50% for rapid permeability, but the sum of coconut oil + palm oil + hempseed oil + cod liver oil = 46.2%, which guarantees an oily residue. The weight ratio of omega 6 fatty acids to omega 3 fatty acids (n6//n)3 ratio is 2. The Penetration factor is low (1.18).

Without wishing to be bound, it is believed that RG7 feeds the skin under the eye to strengthen weakened eye bag skin. Fat accumulates under the eye. It is also believed that some of the accumulated under eye fat is dissolved by MCT and relocates away from the eye.

### EXAMPLE 12

### Preparation of Omeza Gel Rg9:

RG 9, prepared in accordance with the teachings herein for an oil, has the following composition:

| **Omeza Gel RG9** | |
|---|---|
| **Skin Protectant** | |
| **MCT Oil** | **80.66%** |
| **Monolaurin** | **0.60%** |
| **Cetyl esters NF** | **1.50%** |
| **virgin coconut oil** | **033%** |
| **RBD palm oil** | **2.00%** |
| **squalene/Vit E** | **0.20%** |
| **Hemp Oil** | **735%** |
| **Cod Liver Oil** | **7.06%** |
| **coconut FFA** | **030%** |
| **Total** | **100.00%** |
| | |
| **(ALA+SDA)/(EPA+DHA) [PUFA Ratio]** | **1.00** |
| **ALA+SDA+EPA+DHA (sum omega3)** | **3.00** |
| **(C8+C10)/sum(other triglycerides) Penetration Factor** | **5.00** |

RG9 adds coconut free fatty acid to the composition. Without wishing to be bound, it is believed that monolaurin and lauric acid (principal fatty acid in coconut FFA) create a synergistic antimicrobial composition. RG9 has a high Penetration Factor (5) to drive oils into the skin but leave monolaurin and lauric acid on the skin surface. This combination reduces skin infections while temporarily protecting the underlying skin.

### EXAMPLE 13

### Preparation of K7e and/or K7e3:

K7e and K7e3 have the following composition:

| **Omeza Oil - K7e** | |
|---|---|
| **Facial Hydration** | |
| **MCT Oil** | **89.79%** |
| **Hemp Oil** | **2.41%** |
| **Cod Liver Oil** | **2.36%** |
| **Monolaurin** | **0.60%** |
| **Cetyl esters NF** | **1.50%** |
| **virgin coconut oil** | **0.34%** |
| **RBD palm oil** | **3.00%** |
| **Total** | **100.00%** |
| | |
| **(ALA+SDA)/(EPA+DHA) [PUFA Ratio]** | **1.00** |
| **ALA+SDA+EPA+DHA (sum omega3)** | **1.00** |
| **(C8+C10)/sum(other triglycerides) Penetration Ratio** | **11.42** |

| | **Omeza Oil K7e3 Burn Lavage** |
|---|---|
| **MCT Oil** | **88.86%** |
| **Hemp Oil** | **2.41%** |
| **Cod Liver Oil** | **2.36%** |
| **Monolaurin** | **0.60%** |
| **Cetyl esters NF** | **1.50%** |
| **virgin coconut oil** | **0.34%** |
| **squalene/Vit E** | **0.20%** |
| **RBD palm oil** | **2.58%** |
| **lidocaine** | **0.80%** |
| **camphor** | **035%** |
| **Total** | **100.00%** |
| **(ALA+SDA)/(EPA+DHA) [PUFA Ratio]** | **1.00** |
| **ALA+SDA+EPA+DHA (sum omega3)** | **1.00** |
| **(C8+10)/(sum other triglycerides) - Penetration Ratio** | **11.64** |

Both K7e and K7e3 are prepared as described hereinabove for oils.

Testers describe K7e as "dry oil" because it instantly penetrates the thin skin of the face. It was found that, contrary to damaged skin, healthy skin does not need high doses of omega3 and omega6 fatty acids. Instead, low doses (sum omega3 0.3 to 4.9%) with a (ALA+SDA)/(EPA+DHA) ratio from 0.5 to 1.5 were preferred for healthy skin. What users report is smoother skin, softer skin texture and skin with more turgor (strength).

The core K7e formula is adaptable to carry other lipophilic active ingredients into the skin, such as lidocaine and camphor (analgesic active ingredients). When lidocaine and camphor are added (K7e3), users with extreme sunburn report that their skin does not peel off and the burn is gone permanently.

### EXPLANATION OF EXAMPLE 13

MCT oil is an alternate source of cellular energy to glucose. Without wishing to be bound, it is believed that its consumption does not make inflammatory lactate, so it is indirectly anti-inflammatory. Without wishing to be bound, it is also believed that MCT consumption by cells accelerates the production of M2 macrophages. M1 (inflammatory) and M2 (anti-inflammatory) are in a dynamic equilibrium based on what the body signals it needs. With healthy skin, it is believed that MCT >80% may increase the total concentration of debris-removing macrophages. What users report is smoother skin, softer skin texture and skin with more turgor (strength).

When lidocaine and camphor are added (K7e3), users with extreme sunburn report that their skin does not peel off and the burn is gone permanently.

Camphor appears to increase the permeability of free fatty acids and monolaurin in ways poorly understood. When camphor is added to various compositions, there is significantly reduced residual oil on the skin surface.

### EXAMPLE 14

### Preparation of Omeza Gel RG10:

Omeza Gel RG10 has the following composition:

| **Omeza Burn Stuff RG10 Spray** | |
|---|---|
| **MCT Oil** | **72.14%** |
| **Monolaurin** | **0.60%** |
| **Cetyl esters NF** | **1.50%** |
| **coconut oil** | **2.50%** |
| **Hemp Oil** | **7.39%** |
| **Cod Liver Oil** | **7.09%** |
| **coconut fatty acid** | **2.50%** |
| **benzethonium chloride** | **0.10%** |
| **squalene/Vitamin E** | **2.48%** |
| **lidocaine** | **0.70%** |
| **camphor** | **3.00%** |
| | |
| **Total** | **100.00%** |
| **(C8+10)/(sum other triglycerides) Penetration Factor** | **4.00** |
| **(ALA+SDA)/(EPA+DHA) [PUFA Ratio]** | **1.00** |
| **ALA+SDA+EPA+DHA (sum omega3)** | **3.00** |

RG10 is prepared as follows: All of the ingredients are mixed together and are heated to 185°F where the organic salt, benzethonium chloride, dissolves in the presence of camphor >0.3 wt%. When homogenous, the mixture is cooled to room temperature.

But when the antimicrobial salt is dissolved in an anhydrous lipophilic oil, the solute is dragged through the stratum corneum where it can kill subsurface bacteria in the epidermis and dermis.

### EXPLANATION OF EXAMPLE 14

RG10 solubilizes benzethonium chloride. Without wishing to be bound, it is believed that the high Penetration Factor (4.0) drags the solute into the epidermis and dermis where it kills pathogens. RG10 also contains 2.5% coconut free fatty acid and 0.6% monolaurin. The monolaurin and lauric acid also transfer into the epidermis and dermis (because the monolaurin is a solute (monolaurin precipitates at concentrations >0.6%)). The benzethonium solute and the monolaurin/lauric acid solute have different mechanisms of action (MOA) to kill pathogens. It is well known that multiple MOA attacks on pathogens is more effective than any single MOA. The result is faster elimination of clinically relevant subsurface infections.

### EXAMPLE 15

### Topical Compositions Comprising Antimicrobial Properties:

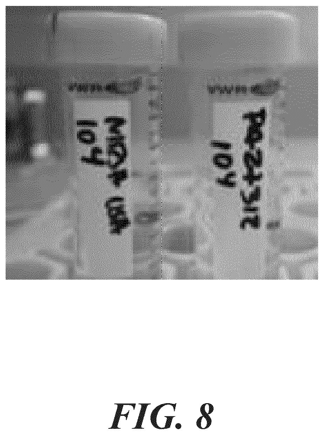

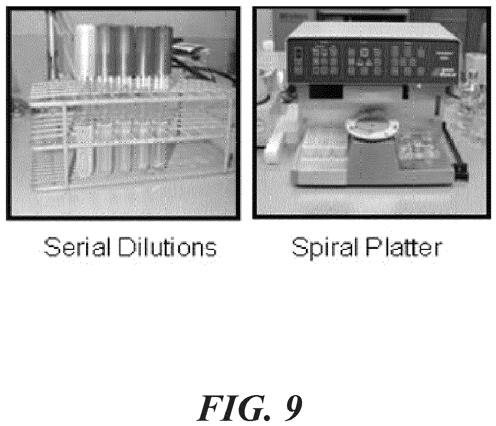

A fresh culture of pathogenic isolates was used in these studies. The bacterial strains were Methicillin Resistant *Staphylococcus aureus* MRSA USA300 (MRSA USA300) and *Pseudomonas aeruginosa* ATCC 27312 (PA27312). The freeze-dried bacteria cultures were recovered per standard recovering protocol. All challenge inoculum suspensions were prepared by swabbing a 3-cm diameter area of the growth from a culture plate into 4.5 ml of sterile phosphate buffer solution (PBS). This resulted in a suspension consisting of approximately 10¹⁰ colony forming units/ml (CFU/ml) for each bacteria. Serial dilutions were made until a concentration of 10⁴ CFU/ml is achieved for all bacteria, as shown in **FIG. 8****.** The concentration was confirmed using historical optical density measurements. In addition, serial dilutions of the suspensions were plated onto specific media for these microorganisms using the Spiral Plater System, as shown in **FIG. 9****,** that deposits a small amount (50 µl) of suspension over the surface of a rotating culture media agar plate to quantitate the exact concentration of viable organisms prior to the experiment. Concentrations of 10⁴CFU/ml for each bacteria was tested in this study.

### EXPERIMENTAL DESIGN

The following products (Treatment Groups) were tested:
A. OMEZA^{®} Collagen Matrix (i.e., collagen-integrated topical composition)
B. OMEZA^{®} Skin Protectant (i.e., collagen-free topical composition)
C. OMEZA^{®} Lidocaine Lavage (i.e., collagen-free topical composition comprising analgesic (e.g., lidocaine)
D. Positive Control (Mupirocin for MRSA USA300 and Silver sulfadiazine for PA27312)
E. Negative Control Petrolatum
F. Negative Control Sterile PBS

### METHODOLOGY AND METHODS

Three (3) plates of Tryptic soy agar with 5% sheep blood (TSAII) per treatment for each bacteria was challenged with 100ul of each inoculum 10⁴ CFU/ml (MRSA USA300 or PA27312).

Inoculums were spread around each plate using 3mm sterile glass beads.

Three (10mm) punch biopsies were made on all inoculated TSAII blood culture media plates and those biopsies were removed with a sterile spatula to create wells on each plate.

Approximately 150-200 µl of each treatment was placed in the well of the inoculated TSA II plates. OMEZA^{®} Collagen Matrix treatment application. OMEZA^{®} Skin Protectant treatment application. OMEZA^{®} Lidocaine Lavage treatment application. Positive Control (Mupirocin or Silver sulfadiazine) treatment application. Negative Control Petrolatum or Sterile PBS.

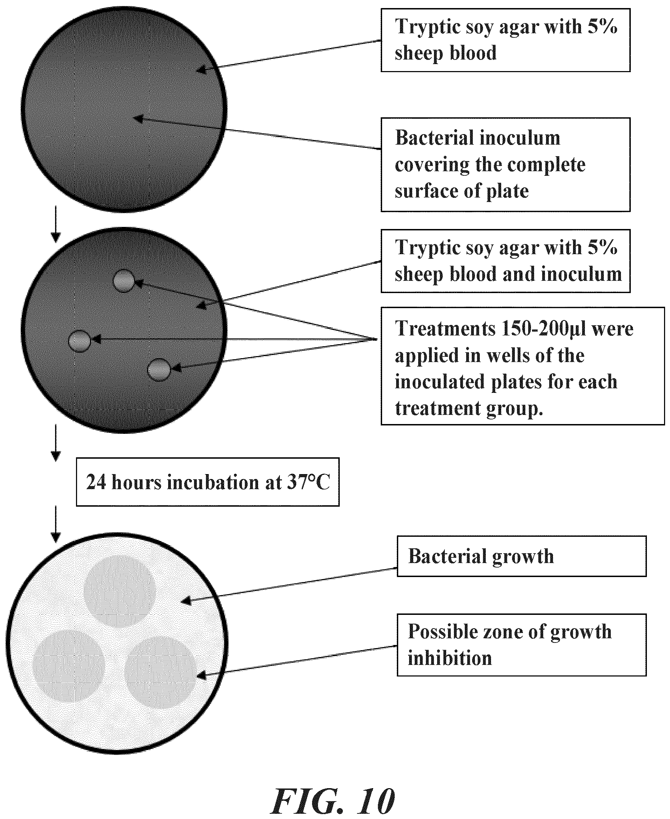

Treatments were allowed to diffuse into agar for approximately 2-3 hours at room temperature prior to incubating plates for 24 hours at 37°C (a summary of these procedures in methodology diagram are provided in **FIG. 10**).

After the incubation period, the zones of bacteria growth inhibition (if any) were measured using ImageJ 1.41o software.

Based on the data gathered, the antibacterial with the greatest ability to inhibit bacterial growth was determined.

### RESULTS

IBM SSPS Statistics Version 26 with ANOVA statistical analysis was used to determine any differences between treatments. Appendix 1 shows the raw data.

### MRSA USA300. Bacterial Concentration 10⁴ CFU/ml

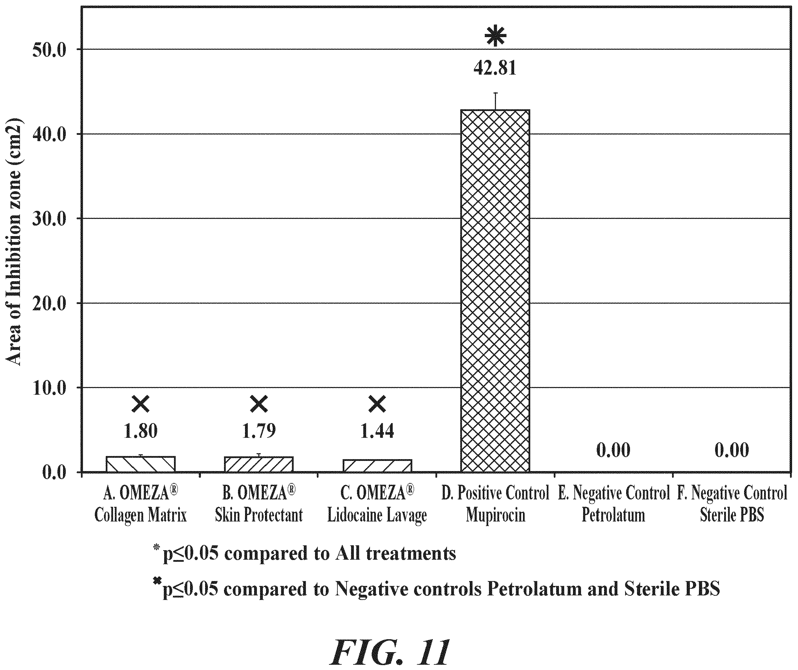

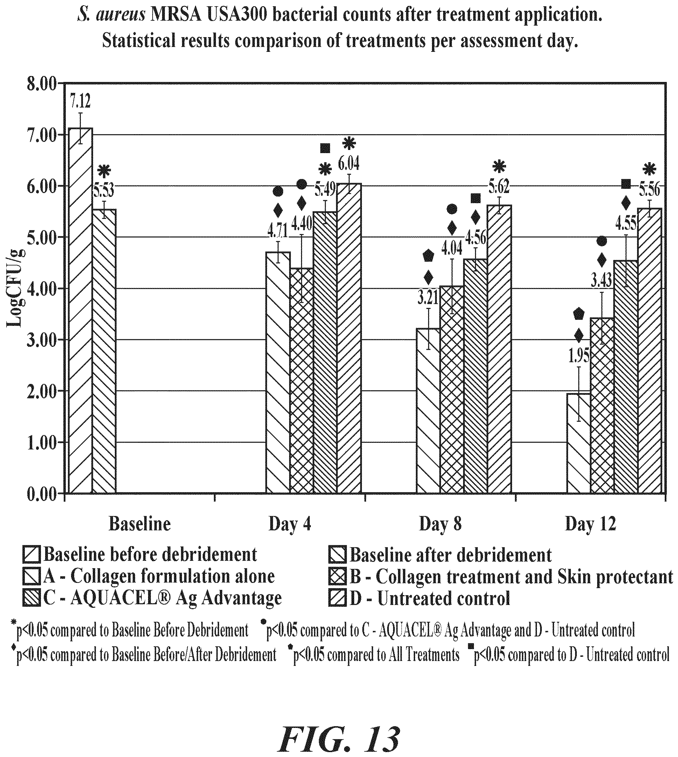

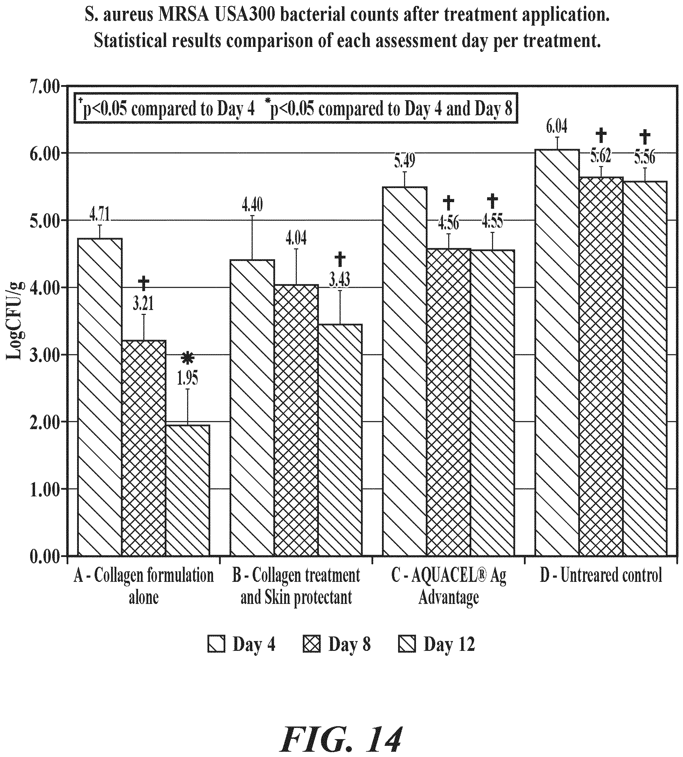

TSAII plates challenged with 10⁴ CFU/ml concentration of MRSA USA300 showed those wells with Positive Control Mupirocin having the highest areas of inhibition zones (42.81±0.29 cm²), showing significantly (p≤ 0.05) higher areas than all other treatment groups, as shown in **FIG. 11****,** in conjunction with **FIG. 13** and **FIG. 14****.**

Plates inoculated MRSA USA300 (10⁴ CFU/ml bacterial concentration) and challenged with OMEZA^{®} Collagen Matrix, OMEZA^{®} Skin Protectant and OMEZA^{®} Lidocaine Lavage had similar inhibition zones (1.80±0.19, 1.79±0.29 and 1.44±0.13 cm², respectively) exhibiting statistically (p≤ 0.05) significant larger areas than both negative controls tested (Petrolatum and Sterile PBS).

### Pseudomonas aeruginosa ATCC27312. Bacterial Concentration 10⁴ CFU/ml

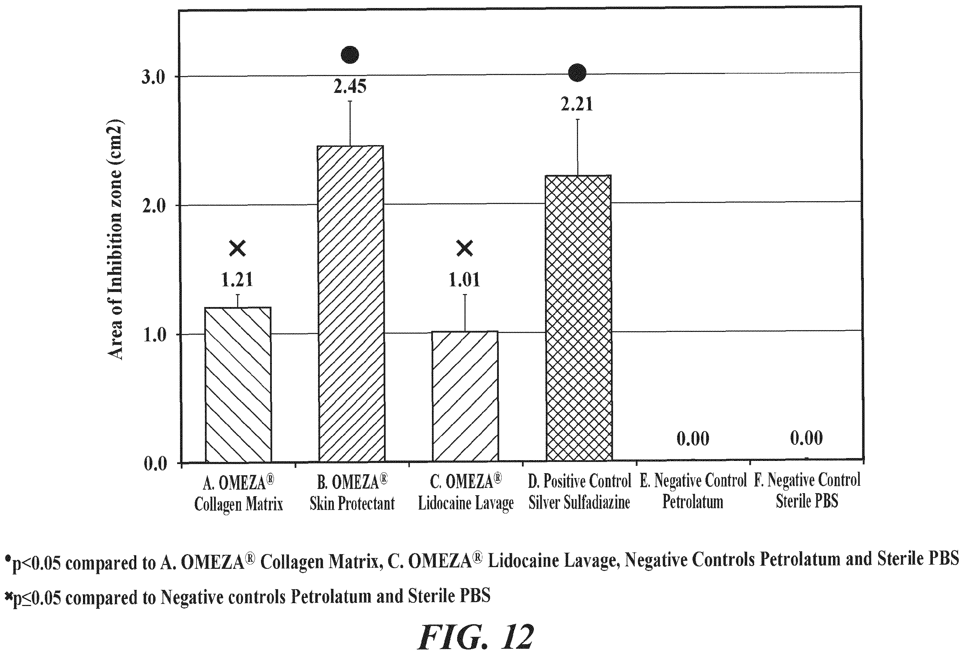

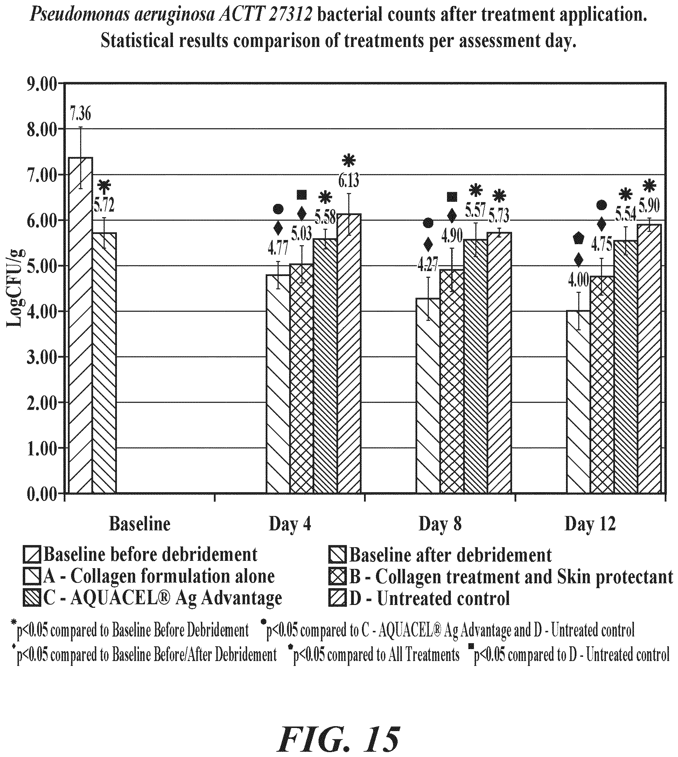

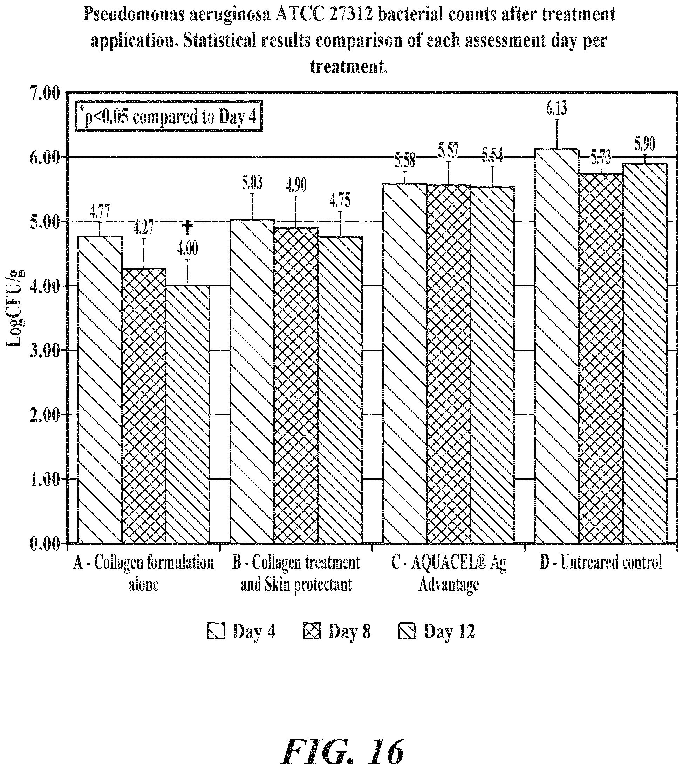

For the TSAII plates challenged with 10⁴ CFU/ml concentration of PA27312, those wells with OMEZA^{®} Skin Protectant had the highest significant (p<_0.05) inhibition zones (2.45±0.35 cm²) compared with all treatment groups (OMEZA^{®} Collagen Matrix, OMEZA^{®} Lidocaine Lavage and both negative controls Petrolatum and Sterile PBS), except with plates challenged with Positive control Silver Sulfadiazine (2.21±0.44 cm²). Positive control Silver Sulfadiazine as well as OMEZA^{®} Skin Protectant exhibited the similar significant (p≤0.05) inhibition zones compared to OMEZA^{®} Collagen Matrix, OMEZA^{®} Lidocaine Lavage and both negative controls Petrolatum and Sterile PBS. Plates inoculated with PA27312 and treated with OMEZA^{®} Collagen Matrix and OMEZA^{®} Lidocaine Lavage resulted in significant inhibition p≤ 0.05) zone compared to both negative controls tested (Petrolatum and Sterile PBS), as shown in **FIG. 12****,** in conjunction with **FIG. 15** and **FIG. 16****.**

### CONCLUSIONS

The results of the study demonstrated that the treatment that created the largest zone of inhibition against MRSA USA300 was positive control Mupirocin. All OMEZA treatments showed larger zones as compared to negative controls (Petrolatum and Saline). OMEZA^{®} Skin Protectant showed the largest inhibition against PA27312 followed by Silver Sulfadizine. All treatments showed larger zones of inhibition as compared to negative controls.

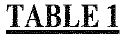

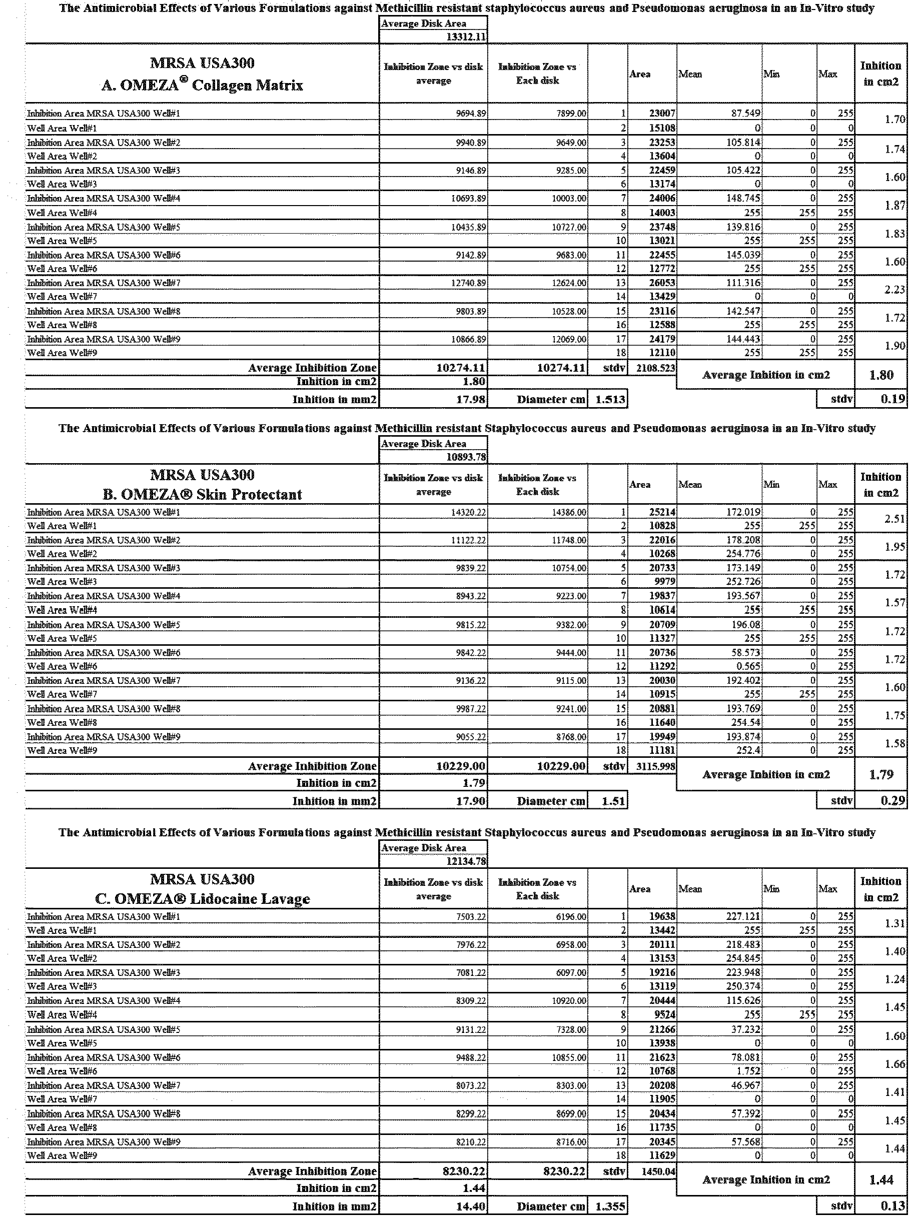

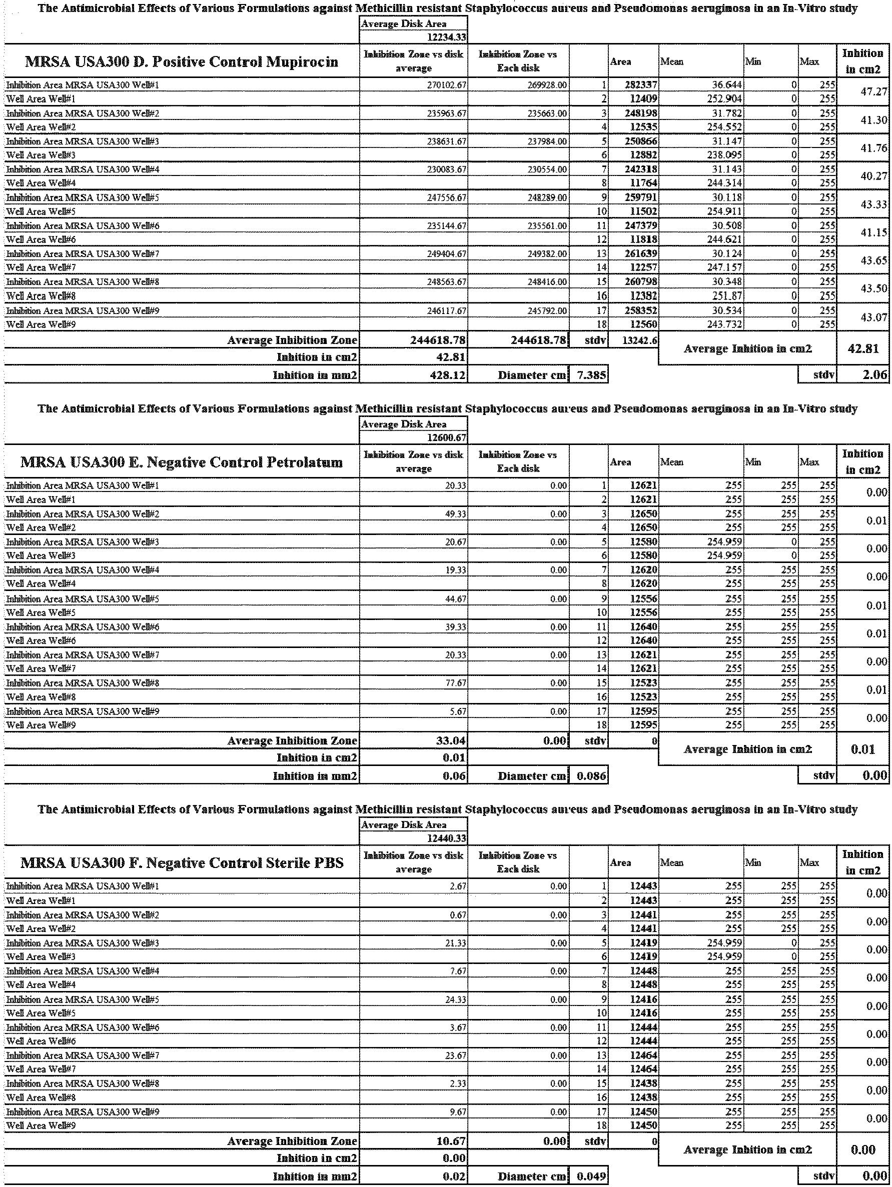

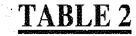

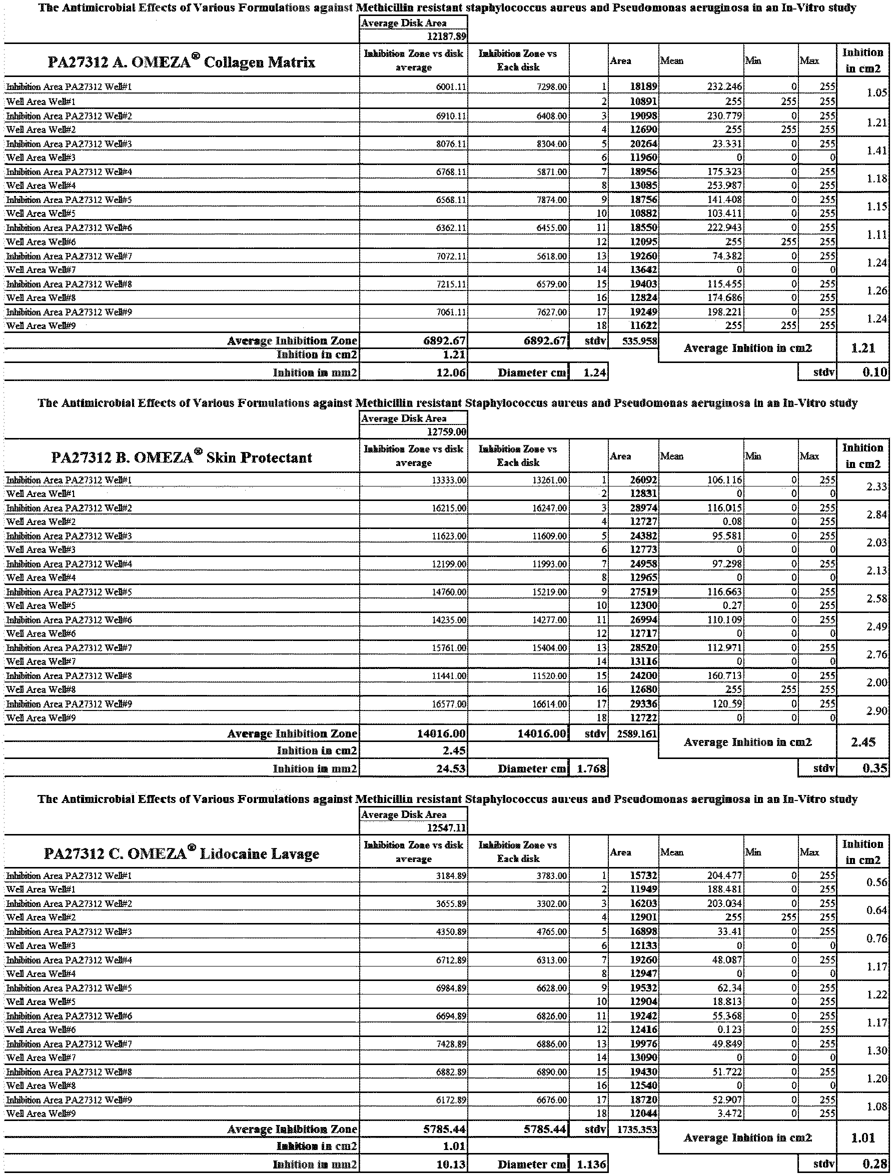

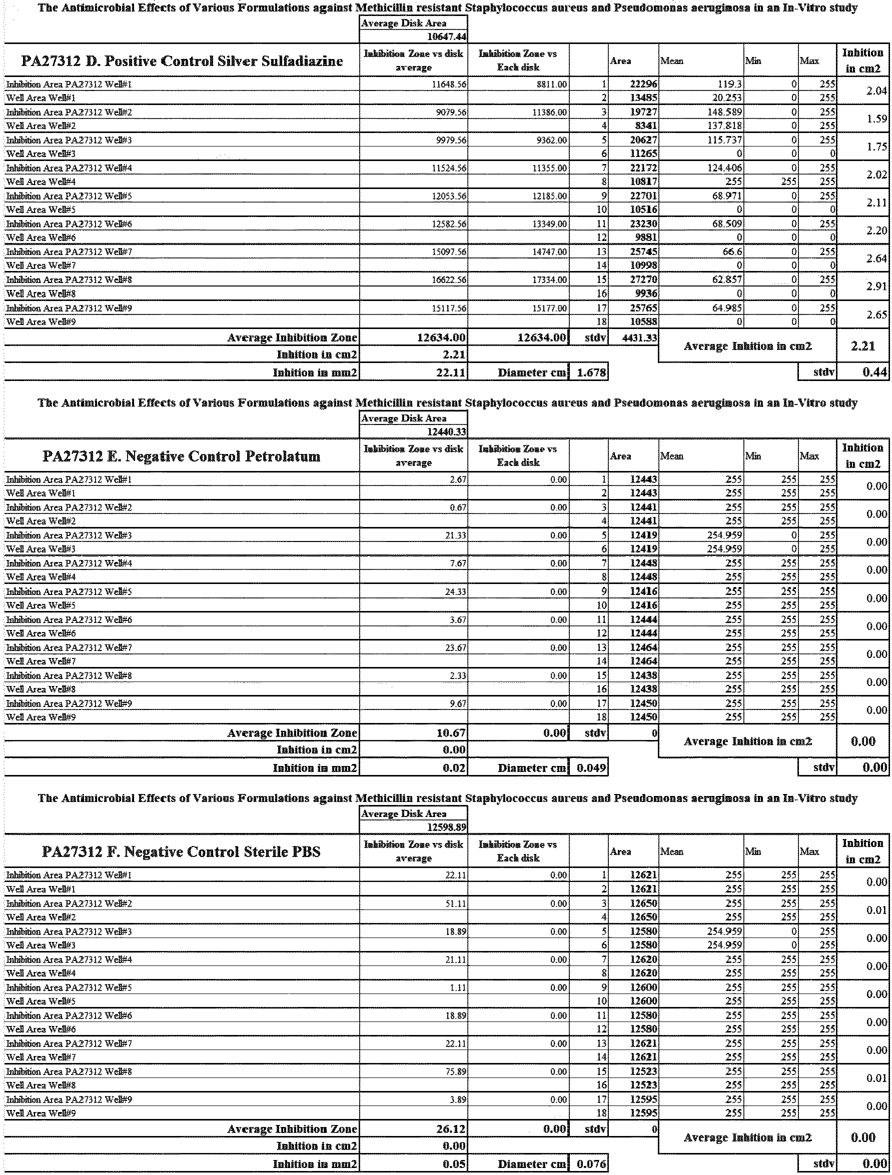

The advantages set forth above, and those made apparent from the foregoing description, are efficiently attained. Since certain changes may be made in the above construction without departing from the scope of the invention, it is intended that all matters contained in the foregoing description or shown in the accompanying drawings shall be interpreted as illustrative and not in a limiting sense.

### INCORPORATION BY REFERENCE

Bettle III, Griscom. FISH OIL TOPICAL COMPOSITION. U.S. 10,463,699 B2, United States Patent and Trademark Office, November 5, 2019.

Bettle III, Griscom, et al. TOPICAL COMPOSITION COMPRISED OF COD LIVER OIL FOR TREATING WOUNDS AND SKIN DISORDERS. WO 2019/200364 A1, World Intellectual Property Organization, October 17, 2019.

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

It is also to be understood that the following claims are intended to cover all of the generic and specific features of the invention herein described, and all statements of the scope of the invention which, as a matter of language, might be said to fall in-between.

## Claims

1. An anhydrous topical composition comprising an omega fatty acid product, the omega fatty-acid product comprising:
at least 5% marine oil by weight;
at least 5% vegetable oil by weight;
a fish collagen;
wherein the marine oil, the vegetable oil, or both comprise an omega-3 fatty acid content greater than 9% by weight; and
wherein the omega fatty acid product is antimicrobial, whereby the omega fatty acid product comprises an inhibition zone of a dermal area of at least 1.2 cm².

2. The anhydrous topical composition of claim 1, further comprises one or more members selected from a group consisting of hempseed oil, triethyl citrate, ethyl linoleate, a caprylic acid, a monolaurin, a cetyl ester, an ascorbyl palmitate, a beeswax, a sea salt, and a combination of thereof, optionally wherein the composition comprises at least 0.5% ascorbyl palmitate by weight.

3. The anhydrous topical composition of claim 1 or claim 2, wherein the composition comprises a thickening agent.

4. The anhydrous topical composition of any one of claims 1 to 3, wherein the vegetable oil comprises one or more members selected from a group consisting of coconut oil, hempseed oil, red palm olein, and a combination of thereof.

5. The anhydrous topical composition of any one of claims 1 to 4, wherein the omega fatty acid product comprises a penetration rate into a dermal area of at least 1.0 to at most 3.0.

6. The anhydrous topical composition of any one of claims 1 to 5, wherein the omega fatty acid product is configured to inhibit MRSA, PA27312, or both.

7. An anhydrous topical composition comprising an omega fatty acid product, the omega fatty-acid product comprising:
at least 5% marine oil by weight;
at least 10% hempseed oil by weight;
at least 1% cetyl esters by weight;
wherein the marine oil, the hempseed oil, or both comprise an omega-3 fatty acid content greater than 9% by weight; and
wherein the omega fatty acid product is antimicrobial, whereby the omega fatty acid product comprises an inhibition zone of a dermal area of at least 2.1 cm².

8. The anhydrous topical composition of claim 7, wherein the composition further comprises at least 0.8% lidocaine by weight.

9. The anhydrous topical composition of claim 7 or claim 8, wherein the composition further comprises a thickening agent.

10. The anhydrous topical composition of any one of claims 7 to 9, further comprising one or more members selected from a group consisting of a hempseed oil, a monolaurin, medium chain triglycerides (hereinafter "MCT"), an ascorbyl palmitate, free fatty acids (hereinafter "FFA"), and a combination of thereof, optionally wherein the composition further comprises at least 0.5% ascorbyl palmitate by weight.

11. The anhydrous topical composition of any one of claims 7 to 10, wherein the omega fatty acid product comprises a semi-solid state, whereby when the omega fatty acid product is sheared, the omega fatty acid product does not form local capillaries, optionally wherein the omega fatty acid product is configured to inhibit MRSA, PA27312, or both.

12. A method for synthesizing an anhydrous topical composition comprising an omega fatty acid product, the method comprising:
providing a mixture comprising a marine oil;
subjecting said mixture to a heating vessel and a cooling vessel to yield a colloidal product;
using the colloidal product, a derivative thereof, or both to form the composition comprising the omega fatty acid product, wherein the omega fatty acid product comprises (i) at least 5% marine oil by weight; (ii) at least 5% vegetable oil by weight; and (iii) a fish collagen;
wherein the marine oil, the vegetable oil, or both comprise an omega-3 fatty acid content greater than 9% by weight; and
wherein the omega fatty acid product is antimicrobial, whereby the omega fatty acid product comprises an inhibition zone of a dermal area of at least 1.2 cm².

13. The method of claim 12, further comprising the step of using a thickening agent to aggregate the mixture, the colloidal product, or both, optionally wherein the vegetable oil comprises one or more members selected from a group consisting of coconut oil, hempseed oil, red palm olein, and a combination of thereof.

14. The method of claim 12 or claim 13, wherein the omega fatty acid product comprises a penetration rate into a dermal area of at least 1.0 to at most 3.0.

15. The method of any one of claims 12 to 14, wherein the anhydrous topical composition further comprises the one or more members selected from a group consisting of hempseed oil, triethyl citrate, ethyl linoleate, a caprylic acid, a monolaurin, a cetyl ester, an ascorbyl palmitate, a beeswax, a sea salt, and a combination of thereof, optionally wherein the anhydrous topical composition comprises at least 0.5% ascorbyl palmitate by weight.
